# EUROPEAN PATENT SPECIFICATION

(11) **EP 3 468 555 B1**
(45) Date of publication and mention of the grant of the patent: **09.08.2023**
(21) Application number: 17812663.7
(22) Date of filing: 12.06.2017
(51) Int. Cl.: A61K 31/426, A61K 31/421, C07D 277/36, C07D 263/18, A61P 31/04, A61K 31/7036, A61K 31/357, C07D 417/06, A01N 31/00, A61L 2/16, C11D 3/48, A01N 43/78, C11D 3/28, C11D 3/34, C11D 7/32, C11D 11/00

(54) **COMPOSITIONS FOR USE IN TREATING BACTERIAL INFECTIONS**
ZUSAMMENSETZUNGEN ZUR VERWENDUNG BEI DER BEHANDLUNG VON BAKTERIELLEN INFEKTIONEN
COMPOSITIONS POUR UTILISATION DANS LE TRAITEMENT D'INFECTIONS BACTÉRIENNES

(30) Priority: 14.06.2016 US 201662349823 P
(43) Date of publication of application: 17.04.2019
(73) Proprietor: Versitech Limited, Hong Kong (HK)
(72) Inventor: KAO, Yi Tsun, Richard, Hong Kong (HK); YAM, Hin Cheung, Bill, Hong Kong (HK); GAO, Peng, Hong Kong (HK); IU, Ho Ting, Hong Kong (HK)
(74) Representative: de Arpe Fernandez, Manuel
(86) International application number: PCT/CN2017/087898
(87) International publication number: WO 2017/215552

(56) References cited:
- US-A1- 2004 006 112
- PHILIPPE VILLAIN-GUILLOT ET AL: "Structure-Activity Relationships of Phenyl-Furanyl-Rhodanines as Inhibitors of RNA Polymerase with Antibacterial Activity on Biofilms", JOURNAL OF MEDICINAL CHEMISTRY, vol. 50, no. 17, 1 August 2007 (2007-08-01), pages 4195-4204, XP055664838, US ISSN: 0022-2623, DOI: 10.1021/jm0703183
- SUNDARAM KAVERI ET AL: "Synthesis, antibacterial activity against MRSA, and in vitro cytotoxic activity against HeLa cell lines of novel 3-[alpha]-carboxy ethyl-5-benzylidene rhodanine derivat", RESEARCH ON CHEMICAL INTERMEDIATES : AN INTERNAT. JOURNAL, AMSTERDAM, NL, vol. 41, no. 2, 24 May 2013 (2013-05-24), pages 1011-1021, XP035426214, ISSN: 0922-6168, DOI: 10.1007/S11164-013-1251-8 [retrieved on 2013-05-24]
- CHEN Z H ET AL: "Synthesis of new chalcone derivatives containing a rhodanine-3-acetic acid moiety with potential anti-bacterial activity", EUROPEAN JOURNAL OF MEDICINAL CHEMISTRY, ELSEVIER, AMSTERDAM, NL, vol. 45, no. 12, 1 December 2010 (2010-12-01), pages 5739-5743, XP027526535, ISSN: 0223-5234 [retrieved on 2010-09-19]
- ZINGLÉ CATHERINE ET AL: "Catechol-rhodanine derivatives: Specific and promiscuous inhibitors ofEscherichia colideoxyxylulose phosphate reductoisomerase (", BIOORGANIC & MEDICINAL CHEMISTRY : A TETRAHEDRON PUBLICATION FOR THE RAPID DISSEMINATION OF FULL ORIGINAL RESEARCH PAPERS AND CRITICAL REVIEWS ON BIOMOLECULAR CHEMISTRY, MEDICINAL CHEMISTRY AND RELATED DISCIPLINES, ELSEVIER, NL, vol. 22, no. 14, 14 May 2014 (2014-05-14), pages 3713-3719, XP029033696, ISSN: 0968-0896, DOI: 10.1016/J.BMC.2014.05.004
- PATEL BHARGAV A ET AL: "The synthesis and SAR study of phenylalanine-derived (Z)-5-arylmethylidene rhodanines as anti-methicillin-resistantStaphylococcus aureus(MRSA) compounds", BIOORGANIC & MEDICINAL CHEMISTRY LETTERS, PERGAMON, AMSTERDAM, NL, vol. 23, no. 20, 20 August 2013 (2013-08-20), pages 5523-5527, XP028731097, ISSN: 0960-894X, DOI: 10.1016/J.BMCL.2013.08.059
- JING MIAO ET AL: "Synthesis and antibacterial activity of chalcone and rhodanine derivatives", JOURNAL OF MEDICAL SCIENCE YANBIAN UNIVERSITY, vol. 36, no. 2, 30 June 2013 (2013-06-30), pages 104-109, XP055586082, DOI: 10.16068/j.1000-1824.2013.02.020
- PENG GAO ET AL: "Suppression of Staphylococcus aureus virulence by a small-molecule compound", PROCEEDINGS OF THE NATIONAL ACADEMY OF SCIENCES, vol. 115, no. 31, 16 July 2018 (2018-07-16), pages 8003-8008, XP055659083, US ISSN: 0027-8424, DOI: 10.1073/pnas.1720520115
- MIAO JING et al.: "Synthesis and antibacterial activity of chalcone and rhodanine derivatives", Journal of Medical Science Yanbian University, vol. 36, no. 2, 30 June 2013 (2013-06-30), pages 104-109, XP055586082, DOI: 10.16068/j.1000-1824.2013.02.020
- SONG MINGXIA et al.: "Synthesis and bioactivity evaluation of rhodanine derivatives as potential anti-bacterial agents", European Journal of Medicinal Chemistry, vol. 54, 31 May 2012 (2012-05-31), pages 403-412, XP028932623,
- SUNDARAM KAVERI et al.: "Synthesis, antibacterial activity against MRSA, and in vitro cytotoxic activity against HeLa cell lines of novel 3-a-carboxy ethyl-5-benzylidene rhodanine derivatives", Res Chem Interned, vol. 41, 24 May 2013 (2013-05-24), pages 1011-1021, XP035426214,
- HARDEJ DIANE et al.: "The synthesis of phenylalanine-derived C5-substituted rhodanines and their activity against selected methicillin-resistant Staphylococcus aureus (MRSA) strains", European Journal of Medicinal Chemistry, vol. 45, 25 September 2010 (2010-09-25), pages 5827-5832, XP027526545,

## Description

### FIELD OF THE INVENTION

The invention is generally directed to compositions for use in treating bacterial infections.

### BACKGROUND OF THE INVENTION

Infectious diseases have been the prime cause of death ever since human population existed, and bacterial infection plays a major role in this medical challenge. Nosocomial infection and antibiotic-resistant bacteria related infection has become the major challenge in the medical field for past decades. Attentions were focused on to the ESKAPE pathogens (acronymically for the *Enterococcus faecium, Staphylococcus aureus, Klebsiella pneumoniae, Acinetobacter baumannii, Pseudomonas aeruginosa,* and *Enterobacter* species), their well adaptations in virulence for infections or multidrug resistances for treatment withstanding made therapeutic options very limiting (Khan et al., Front Microbiol., 7:174 (2016)). Rapid developments of resistance mechanisms render new drugs less effective in a short time following use. The shrinking of the pharmaceutical antibiotics pipeline due to the low profit margins only makes this situation worse (Butler et al., The Journal of antibiotics, 66(10):571-591 (2013)). It is foreseeable that in the coming future, more resistant bacteria will emerge, and the lack of potent antimicrobials against bacteria will become a major threat (Tommasi et al., Nat Rev Drug Discov., 14(8): 529-42 (2015)). New antimicrobials/ treatment methods against the bacteria are therefore needed.

Antibiotic resistance is a major problem for bacterial infections. Efforts have been devoted to the development of new compounds for bacterial growth inhibition. However, no major achievements have been made in the past decades. Current treatments option for antibiotics resistant bacteria still rely on combinatory effect of several second or third line antibiotics. There is still a need for better treatment options.

It is therefore an object of the present invention to provide compounds with antibacterial activity.

It is also an object of the present invention to provide compounds that potentiate the antimicrobial effect of existing antibiotics.

It is a further object of the present invention to provide compositions for use in treating bacterial infections.

It is also an object of the present invention to provide compounds with antibacterial activity, which can be used to combat drug resistant bacterial infections.

The following documents disclose general chemical formulas, compounds and compositions useful as antibacterial:
PHILIPPE VILLAIN-GUILLOT ET AL: "Structure-Activity Relationships of Phenyl-Furanyl-Rhodanines as Inhibitors of RNA Polymerase with Antibacterial Activity on Biofilms", JOURNAL OF MEDICINAL CHEMISTRY, vol. 50, no. 17, 1 August 2007 (2007-08-01), pages 4195-4204; and
JING MIAO ET AL: "Synthesis and antibacterial activity of chalcone and rhodanine derivatives", JOURNAL OF MEDICAL SCIENCE YANBIAN UNIVERSITY, vol. 36, no. 2, 30 June 2013 (2013-06-30), pages 104-109.

The document US 2004/006112 A1 (ORCHARD MICHAEL GLEN [GB] ET AL) 8 January 2004 (2004-01-08) discloses similar compounds to the ones of the present application but for use in the treatment of fungal infections.

### SUMMARY OF THE INVENTION

The references to methods of treatment in the summary and detailed description of the invention in this description are to be interpreted as references to the compounds, pharmaceutical compositions and medicaments of the present invention for use in a method for treatment of the human (or animal) body by therapy (or for diagnosis).

Disclosed are compounds, and compositions for use in treating bacterial infections, increasing the efficacy of antibiotics, and reducing virulence of bacteria. The provided compounds with antibacterial properties are compounds 2, 4, 6-7, 10, 13, 15, 17-20, 22, 25, 27-31, 34-37, 42, 44, 48-50, 52-53, 56, 60, 62-63, 69, 80-82 and 87 as further disclosed below. In some forms, the compound potentiates the therapeutic effects of one or more antimicrobial agents when coadministered. In the other forms the compounds have antivirulence properties, measured as repression in *hla* promoter lux reporter assay and suppression of pigment production. Pharmaceutical compositions including the compounds disclosed herein in a pharmaceutically acceptable carrier are provided. The provided compounds are compounds 2-4, 6-7, 10, 13, 15, 17-20, 22, 25, 27-31, 34-37, 42, 44, 48-50, 52-53, 56, 60, 62-63, 69, 80-82 and 87. In this form, the pharmaceutical composition may further comprise at least one antibiotic, such as, Gentamicin, amikacin, kanamycin, neomycin, spectinomycin, neamine or any combination thereof.

Also disclosed are methods of treating or preventing bacterial infection, resulting from microbes such *Staphylococcus aureus, Pseudomonas aeruginosa, Listeria monocytogenes, Burkholderia cepacia, Escherichia coli, Enterococcus faecalis, Streptococcus pneumoniae.*

The method includes administering an effective amount of one or more compound according to any of formulas 2, 4, 6-7, 10, 13, 15, 17-20, 22, 25, 27-31, 34-37, 42, 44, 48-50, 52-53, 56, 60, 62-63, 69, 80-82 and 87 to a subject in need thereof. In some forms, the methods further comprise administering an effective amount of at least one antibiotic, such as, e.g., gentamicin, amikacin, kanamycin, neomycin, spectinomycin, neamine or any combination thereof.

The antibiotics may be administered separately, simultaneously or sequentially. In a preferred form, the amount of a separately, simultaneously, or sequentially administered one or more antimicrobial agents (including one or more antibiotics) may be less than a therapeutically effective or therapeutically optimal dose of the one or more antimicrobial agents (including one or more antibiotics) when administered to the patient or animal that is not in receipt of the disclosed compounds.

The compounds according to the following general formulas are disclosed herein but are not part of the claimed invention.

Disclosed are compounds represented by Formula I 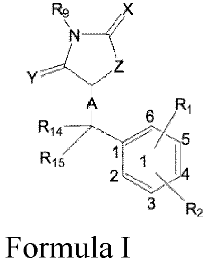
where X and Y are independently O, S, or NR₁₀;where Z is O, S, CR₁₁R₁₂, or NR₁₃;where R₁, R₂, R₉, R₁₀, R₁₁, R₁₂, R₁₃, R₁₄, and R₁₅, are independently absent,
hydrogen, -C(O)R₁₈, -C(W)NR₁₉R₂₀, or substituted or unsubstituted alkyl, alkenyl, alkynyl, alkoxy, cycloalkyl, heterocyclyl, cycloalkenyl, heterocycloalkenyl, aryl, heteroaryl, aroxy, arylalkyl, heteroalkyl, alkylaryl, halogen, alkylheteroaryl, -NR₁₆R₁₇; wherein R₁₆ and R₁₇ are independently hydrogen, substituted or unsubstituted alkyl, alkenyl, alkynyl, cycloalkyl, heterocyclyl, cycloalkenyl, heterocycloalkenyl, aryl, heteroaryl, arylalkyl, heteroalkyl, alkylaryl, or alkylheteroaryl;
where R₁₈ is hydrogen, hydroxyl, or substituted or unsubstituted alkyl, alkenyl, alkynyl, cycloalkyl, alkoxy, heterocyclyl, cycloalkenyl, heterocycloalkenyl, aryl, aroxy, heteroaryl, arylalkyl, heteroalkyl, alkylaryl, or alkylheteroaryl;
where W is O, S, or NR₂₁;
where R₁₉, R₂₀, and R₂₁ are independently hydrogen or substituted or unsubstituted alkyl, alkenyl, alkynyl, cycloalkyl, heterocyclyl, cycloalkenyl, heterocycloalkenyl, aryl, heteroaryl, arylalkyl, heteroalkyl, alkylaryl, or alkylheteroaryl;
where A is a double bond or single bond; and
where R₁₅ is absent when A is a double bond.

In some forms, the disclosed compound can be 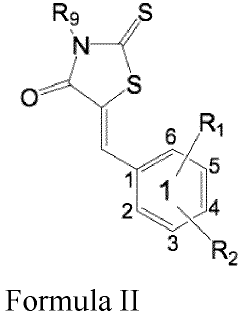
where R₁ is -O-R₄, -NR₅R₈, or -CH₂-R₅; R₂ is halogen, absent, -O-CH₃, or -O-CH₂-CH₃; R₉ is -COOH, -CH₂-COOH, -CH₂-O-CH₃, or -CH₂-CH₂-O-CH₃; R₄ is -CH₂- R₅ or -CO-R₅; R₅ is 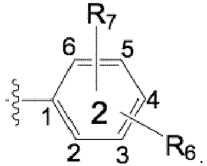
R₆ is a halogen or absent; and R₇ is halogen or absent.

In some forms of the compound, R₁ is at a position selected from the group consisting of 2, 3, 4, 5, and 6. In some forms, R₆ is at position 4. In some forms, R₇ is at position 2 or 6. In some forms, R₂ is at position 3, 4, or 5. In some forms, R₆ is at position 4, R₇ is at position 2 or 6, and R₂ is at position 3 or 5.

In some forms, the disclosed compound is 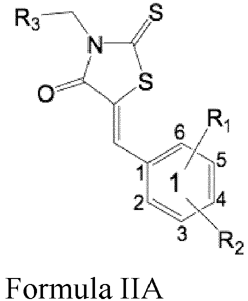
where R₁ is -O-R₄, -NR₅R₈, or -CH₂-R₅; R₂ is halogen, absent, -O-CH₃, or -O- CH₂-CH₃; R₃ is -COOH or -CH₂-COOH, R₄ is -CH₂-R₅ or -COR₅, R₅ is 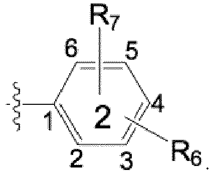
R₆ is halogen or absent; and R₇ is halogen or absent.

In some forms, R₁ is at a position selected from the group consisting of 2, 3, 4, 5, and 6. In some forms, R₆ is at position 4. In some forms, R₇ is at position 2 or 6. In some forms, R₂ is at position 3, 4, or 5. In some forms, R₆ is at position 4, R₇ is at position 2 or 6, and R₂ is at position 3 or 5.

The claimed compounds which are part of the claimed invention are selected from the group consisting of compounds 2, 4, 6-7, 10, 13, 15, 17-20, 22, 25, 27-31, 34-37, 42, 44, 48-50, 52-53, 56, 60, 62-63, 69, 80-82, and 87.

Also disclosed are compositions comprising an effective amount of one or more of the disclosed compounds and a pharmaceutically acceptable carrier. Such compositions are useful for, for example, treating bacterial infections in a subject.

In some forms, the composition further comprises a therapeutically effective amount of an antibiotic. In some forms, the antibiotic is selected from the group consisting of gentamicin, amikacin, kanamycin, neomycin, spectinomycin, neamine and combinations thereof. In some forms, the therapeutically effective amount of the antibiotic is less than a therapeutically effective or therapeutically optimal dose of the antibiotic when administered to a subject in the absence of the composition.

In some forms, at least one of the microorganisms causing the infection is bacteria selected from the group consisting of methicillin-susceptible *Staphyloccous aureus, Pseudomonas aeruginosa, Listeria monocytogenes, Burkholderia cepacia, Escherichia coli, Enterococcus faecalis, Streptococcus pneumoniae* or combinations thereof.

In some forms, at least one of the microorganisms causing the infection is an antibiotic-resistant microorganism selected from the group consisting of a *Streptococcus pneumoniae, Campylobacter, Neisseria gonorrhoeae, Salmonella,* Methicillin-resistant *Staphylococcus aureus* (MRSA), *Shigella,* Vancomycin-resistant *Enterococcus* (VRE), Vancomycin-resistant *Staphylococcus aureus* (VRSA), Erythromycinresistant Group A *Streptococcus*, Clindamycin-resistant Group B *Streptococcus,* Carbapenem-resistant *Enterobacteriaceae* (CRE), drugresistant tuberculosis, Extended spectrum *Enterobacteriaceae* (ESBL), multidrug-resistant *Acinetobacter* (including MRAB), *Clostridium difficile, Enteropathogenic E. coli* (EPEC), *Pseudomonas aeruginosa, H. pylori, Streptococcus anginosus* and *Uropathogenic E. coli* (UPEC).

In some forms, at least one of the microorganisms causing the infection is Methicillin-resistant *Staphylococcus aureus* (MRSA).

In some forms, the microorganism causing the infection is a multidrug-resistant strain of *Listeria monocytogenes, Pseudomonas aeruginosa, Burkholderia cepacia, Enterococcus faecalis* and *Streptococcus pneumoniae.*

In some forms, the infection is selected from the group consisting of impetigo, boils, abscesses, folliculitis, cellulitis, necrotizing fasciitis, pyomyositis, surgical/traumatic wound infection, and infected ulcers and burns), osteomyelitis, device-related osteoarticular infections, impetigo, secondarily infected skin lesions, meningitis, brain abscess, subdural empyema, spinal epidural abscess.

In some forms, the infection is a urinary tract infection. In some forms, the composition is administered parenterally or orally. In some forms, the subject is hospitalized or immunocompromised.

Also disclosed is a method of increasing the efficacy of an antibiotic comprising co-administering the antibiotic and one or more of the disclosed compounds or one or more of the disclosed compositions in amount effective to increase the efficacy of the antibiotic.

Also disclosed is a method of reducing virulence of bacteria selected from the group consisting of methicillin-resistant *Staphyloccous aureus, Pseudomonas aeruginosa, Listeria monocytogenes, Burkholderia cepacia, Escherichia coli, Enterococcus faecalis, Streptococcus pneumoniae* or combinations thereof in a subject comprising administering to the subject an effective amount of one or more of the disclosed compounds or one or more of the disclosed compositions.

In some forms, the compound is selected from the group consisting of compounds 48, 49, and 87.

### BRIEF DESCRIPTION OF THE DRAWINGS

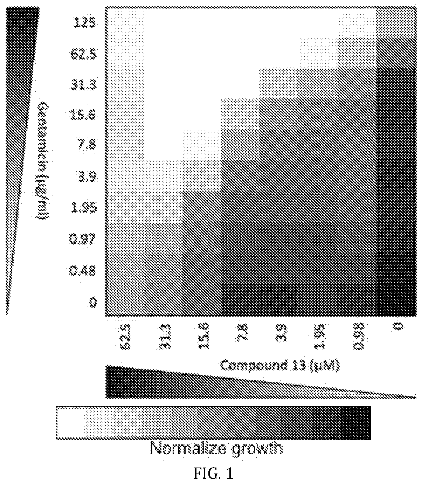

**Figure 1** shows a microdilution checkerboard analysis showing the combined effect of compound 13 with gentamicin. The analysis shows that compound 13 has potentiating effect with gentamicin in inhibiting the growth of *S. aureus* Mu3 strain. Heat Plot shows the average growth from replicated experiments.

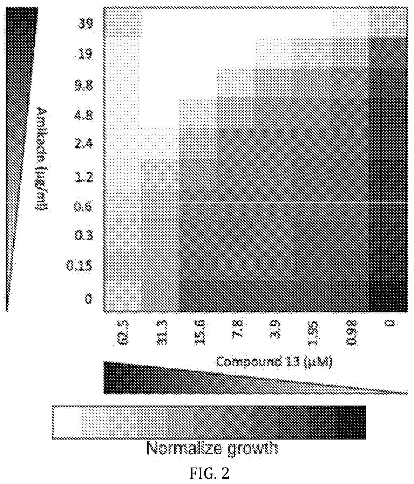

**Figure 2** shows a microdilution checkerboard analysis showing the combined effect of compound 13 with amikacin. The analysis shows that compound 13 has potentiating effect with amikacin in inhibiting the growth of *S. aureus* Mu3 strain. Heat Plot shows the average growth from replicated experiments.

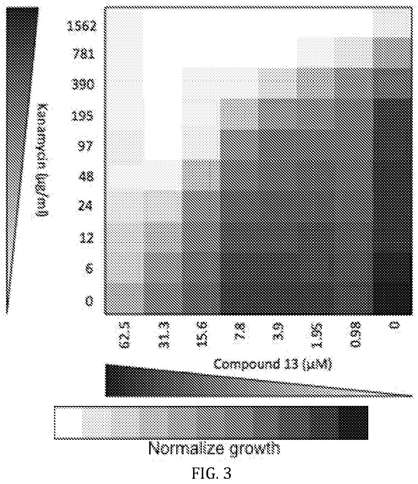

**Figure 3** shows a microdilution checkerboard analysis showing the combined effect of compound 13 with kanamycin. The analysis shows that compound 13 has potentiating effect with kanamycin in inhibiting the growth of *S. aureus* Mu3 strain. Heat Plot shows the average growth from replicated experiments.

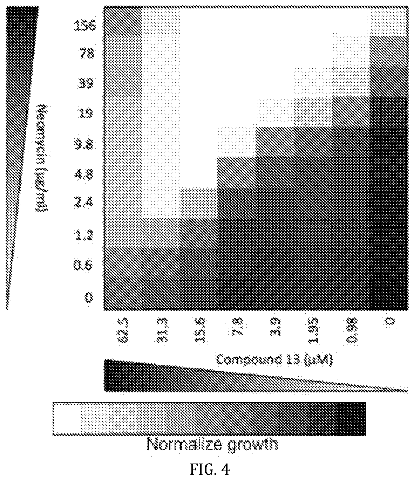

**Figure 4** shows a microdilution checkerboard analysis showing the combined effect of compound 13 with neomycin. The analysis shows that compound 13 has potentiating effect with neomycin in inhibiting the growth of *S. aureus* Mu3 strain. Heat Plot shows the average growth from replicated experiments.

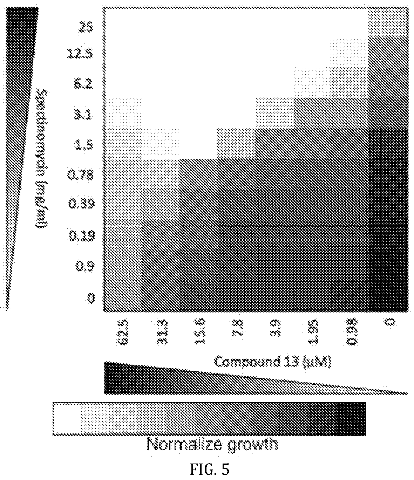

**Figure 5** shows a microdilution checkerboard analysis showing the combined effect of compound 13 with spectinomycin. The analysis shows that compound 13 has potentiating effect with spectinomycin in inhibiting the growth of *S. aureus* Mu3 strain. Heat Plot shows the average growth from replicated experiments.

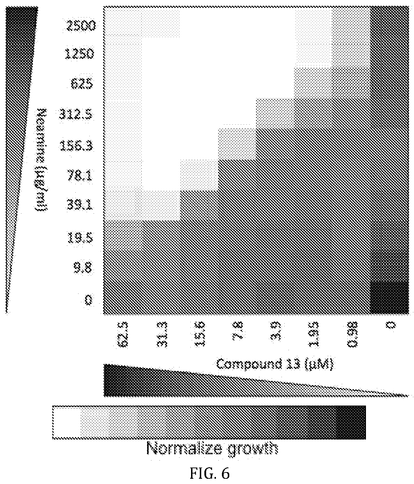

**Figure 6** shows a microdilution checkerboard analysis showing the combined effect of compound 13 with neamine. The analysis shows that compound 13 has potentiating effect with neamine in inhibiting the growth of *S. aureus* Mu3 strain. Heat Plot shows the average growth from replicated experiments.

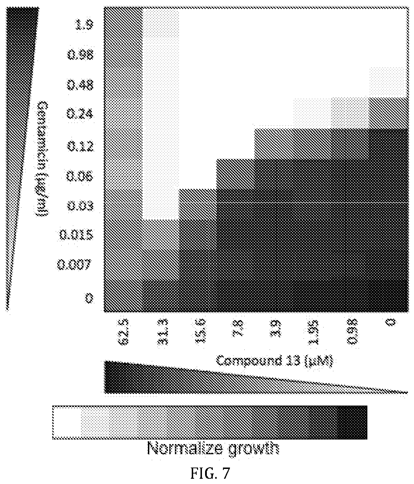

**Figure 7** shows a microdilution checkerboard analysis showing the combined effect of compound 13 with gentamicin. The analysis shows that compound 13 has potentiating effect with gentamicin in inhibiting the growth of *S. aureus* ATCC 29213 strain. Heat Plot shows the average growth from replicated experiments.

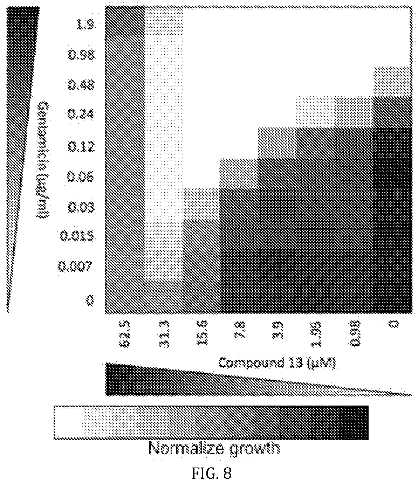

**Figure 8** shows a microdilution checkerboard analysis showing the combined effect of compound 13 with gentamicin. The heat map plot shows normalized growth of checkerboard assay of compound 13 with Gentamicin against Newman.The analysis shows that compound 13 has potentiating effect with gentamicin in inhibiting the growth of *S. aureus* Newman strain. Heat Plot shows the average growth from replicated experiments.

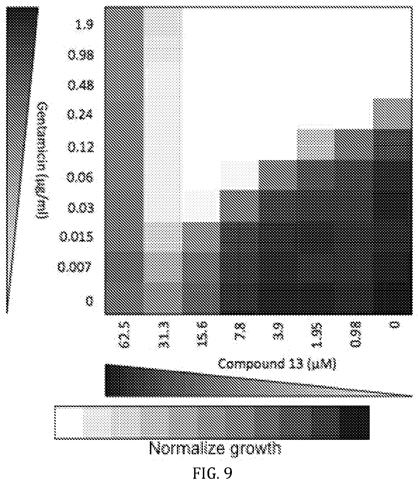

**Figure 9** shows a microdilution checkerboard analysis showing the combined effect of compound 13 with gentamicin. The heat map plot shows normalized growth of checkerboard assay of compound 13 with Gentamicin against RN4220. The analysis shows that compound 13 has potentiating effect with gentamicin in inhibiting the growth of *S. aureus* RN4220 strain. Heat Plot shows the average growth from replicated experiments.

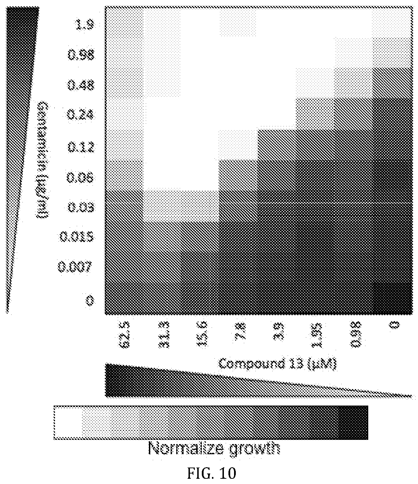

**Figure 10** shows a microdilution checkerboard analysis showing the combined effect of compound 13 with gentamicin. The heat map plot shows normalized growth of checkerboard assay of compound 13 with Gentamicin against USA300.The analysis shows that compound 13 has potentiating effect with gentamicin in inhibiting the growth of *S. aureus* USA300 strain. Heat Plot shows the average growth from replicated experiments.

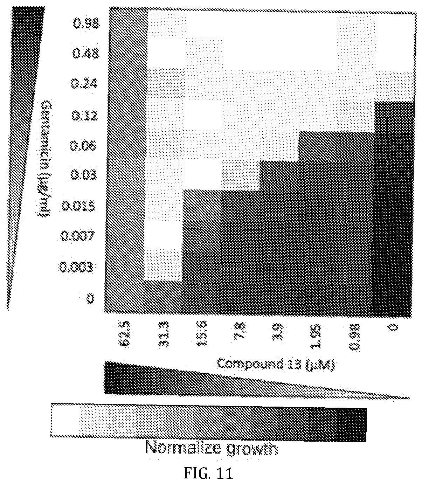

**Figure 11** shows a microdilution checkerboard analysis showing the combined effect of compound 13 with gentamicin. The heat map plot shows normalized growth of checkerboard assay of compound 13 with Gentamicin against COL. The analysis shows that compound 13 has potentiating effect with gentamicin in inhibiting the growth of *S. aureus* COL strain. Heat Plot shows the average growth from replicated experiments.

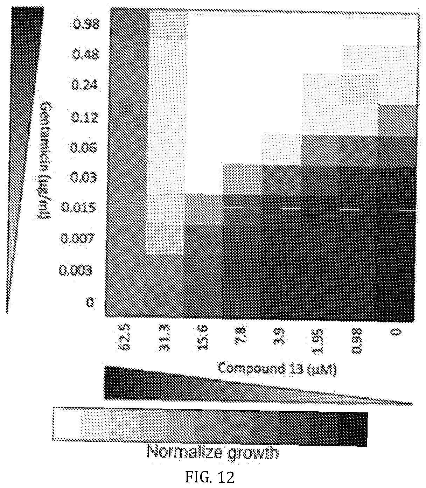

**Figure 12** shows a microdilution checkerboard analysis showing the combined effect of compound 13 with gentamicin. The heat map plot shows normalized growth of checkerboard assay of compound 13 with Gentamicin against RN6390.The analysis shows that compound 13 has potentiating effect with gentamicin in inhibiting the growth of *S. aureus* RN6390 strain. Heat Plot shows the average growth from replicated experiments.

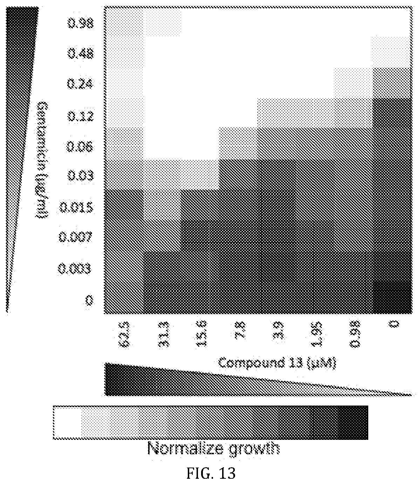

**Figure 13** shows a microdilution checkerboard analysis showing the combined effect of compound 13 with gentamicin. The analysis shows that compound 13 has potentiating effect with gentamicin in inhibiting the growth of *S. aureus* ST45-502 strain. Heat Plot shows the average growth from replicated experiments.

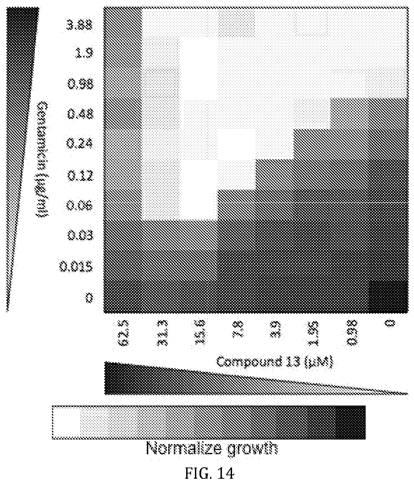

**Figure 14** shows a microdilution checkerboard analysis showing the combined effect of compound 13 with gentamicin. The analysis shows that compound 13 has potentiating effect with gentamicin in inhibiting the growth of *S. aureus* AE-052 strain. Heat Plot shows the average growth from replicated experiments.

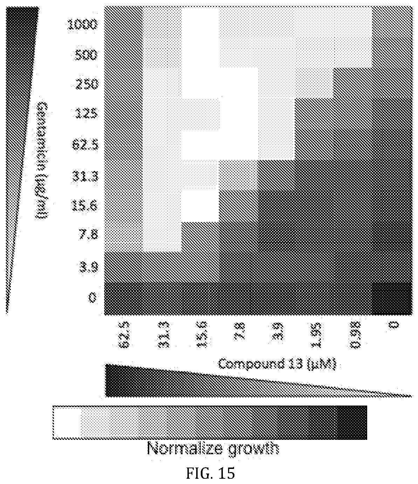

**Figure 15** shows a microdilution checkerboard analysis showing the combined effect of compound 13 with gentamicin. The analysis shows that compound 13 has potentiating effect with gentamicin in inhibiting the growth of *S. aureus* ST239-III-AH-504 strain. Heat Plot shows the average growth from replicated experiments.

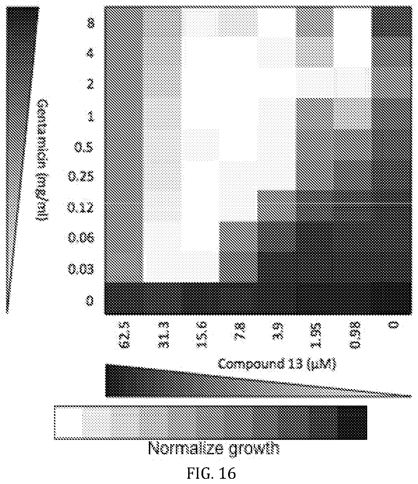

**Figure 16** shows a microdilution checkerboard analysis showing the combined effect of compound 13 with gentamicin. The analysis shows that compound 13 has potentiating effect with gentamicin in inhibiting the growth of *S. aureus* ST239-IIIA-503 strain. Heat Plot shows the average growth from replicated experiments.

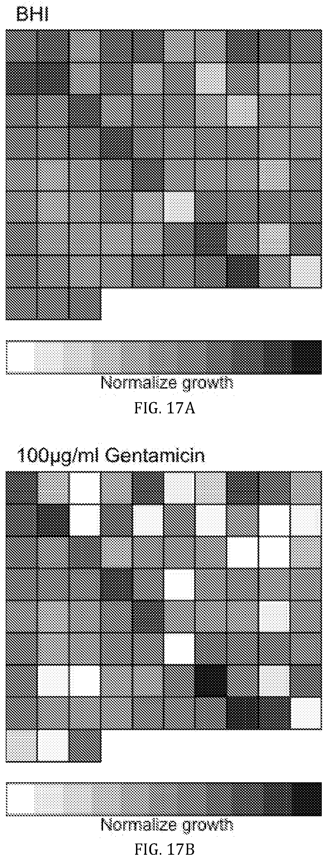

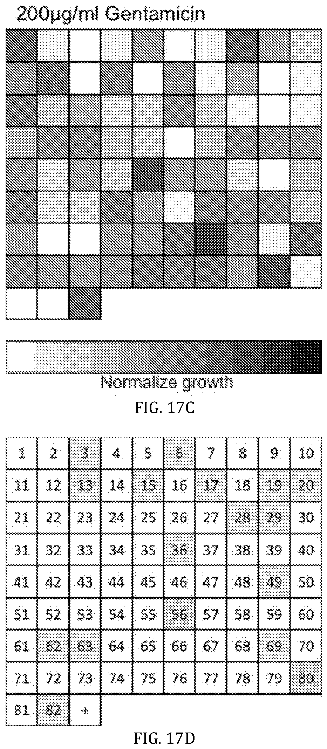

**Figure 17** shows a secondary screening of compound 13 analogues at 50µM in combination of various concentrations of gentamicin against *S. aureus* Mu3. The analysis shows that some structural analogues possess the combinatory inhibitory effect against *S. aureus* Mu3. Heat Plots show the average growth from replicated experiments.

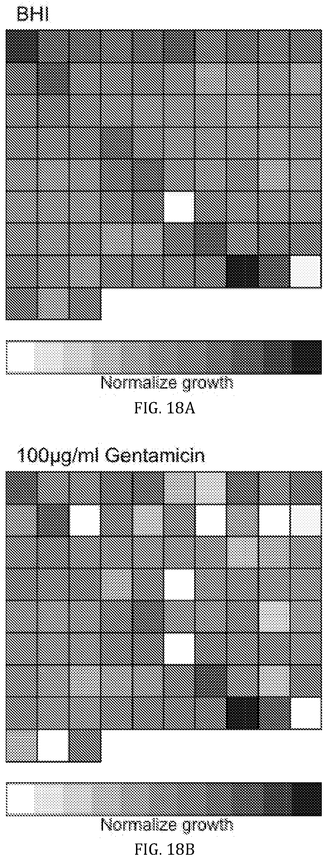

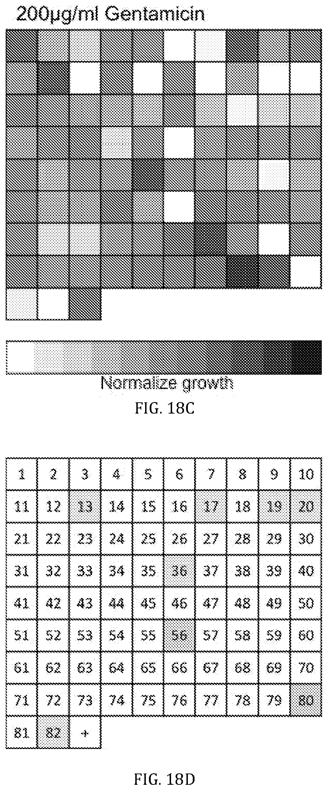

**Figure 18** shows a secondary screening of compound 13 analogues at 20µM in combination of various concentrations of gentamicin against *S. aureus* Mu3. The analysis shows that some structural analogues possess the combinatory inhibitory effect against *S. aureus* Mu3. Heat Plots show the average growth from replicated experiments.

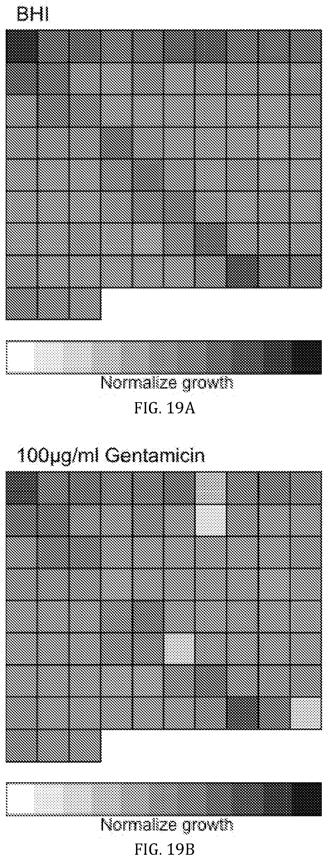

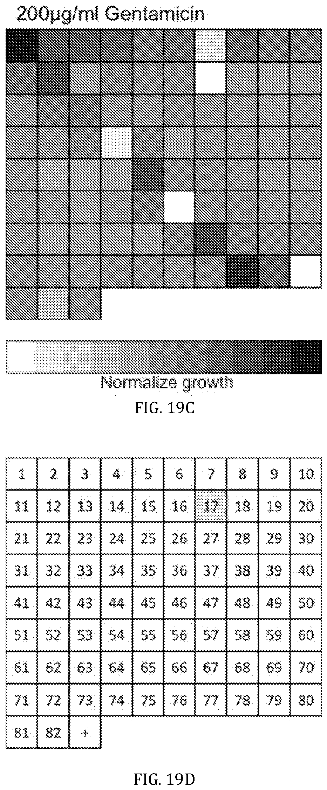

**Figure 19** shows a secondary screening of compound 13 analogues at 5µM in combination of various concentrations of gentamicin against *S*. *aureus* Mu3. The analysis shows that some structural analogues possess the combinatory inhibitory effect against *S. aureus* Mu3. Heat Plots show the average growth from replicated experiments.

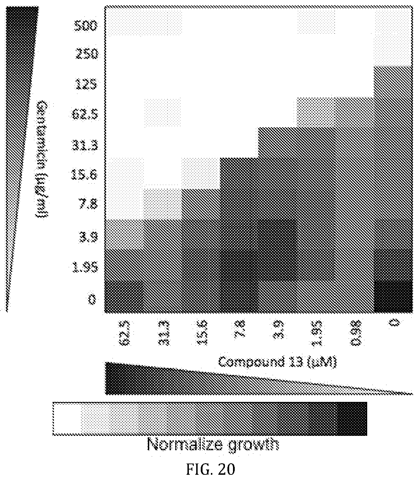

**Figure 20** shows a microdilution checkerboard analysis showing the combined effect of compound 13 with gentamicin. The analysis shows that compound 13 has potentiating effect with gentamicin in inhibiting the growth of *S. aureus* Mu3 strain. Heat Plot shows the average growth from replicated experiments.

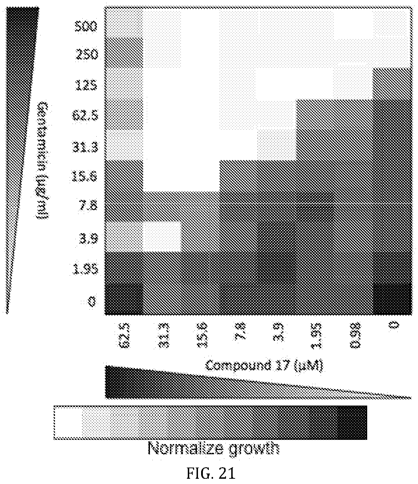

**Figure 21** shows a microdilution checkerboard analysis showing the combined effect of compound 17 with gentamicin. The analysis shows that compound 17 has potentiating effect with gentamicin in inhibiting the growth of *S. aureus* Mu3 strain. Heat Plot shows the average growth from replicated experiments.

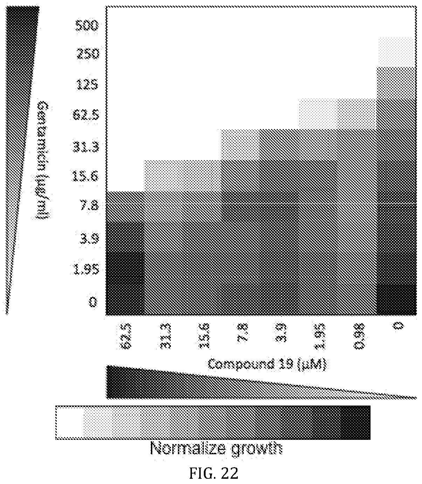

**Figure 22** shows a microdilution checkerboard analysis showing the combined effect of compound 19 with gentamicin. The analysis shows that compound 19 has potentiating effect with gentamicin in inhibiting the growth of *S. aureus* Mu3 strain. Heat Plot shows the average growth from replicated experiments.

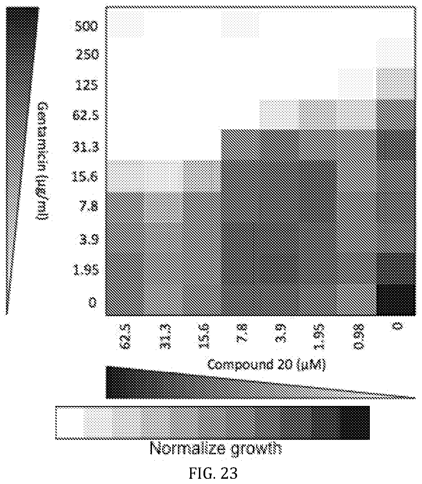

**Figure 23** shows a microdilution checkerboard analysis showing the combined effect of compound 20 with gentamicin. The analysis shows that compound 20 has potentiating effect with gentamicin in inhibiting the growth of *S. aureus* Mu3 strain. Heat Plot shows the average growth from replicated experiments.

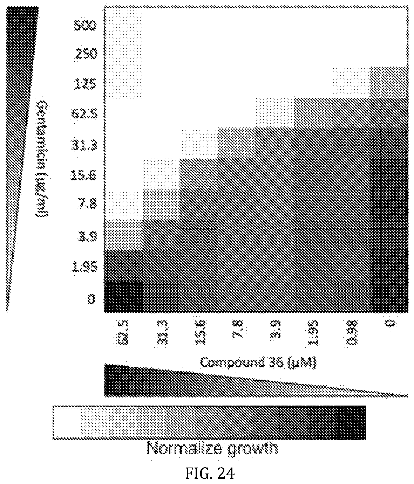

**Figure 24** shows a microdilution checkerboard analysis showing the combined effect of compound 36 with gentamicin. The analysis shows that compound 36 has potentiating effect with gentamicin in inhibiting the growth of *S. aureus* Mu3 strain. Heat Plot shows the average growth from replicated experiments.

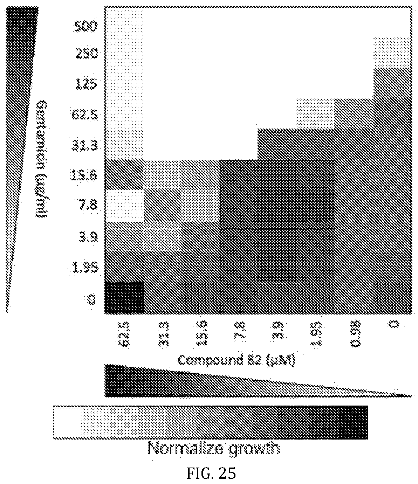

**Figure 25** shows a microdilution checkerboard analysis showing the combined effect of compound 82 with gentamicin. The analysis shows that compound 82 has potentiating effect with gentamicin in inhibiting the growth of *S. aureus* Mu3 strain. Heat Plot shows the average growth from replicated experiments.

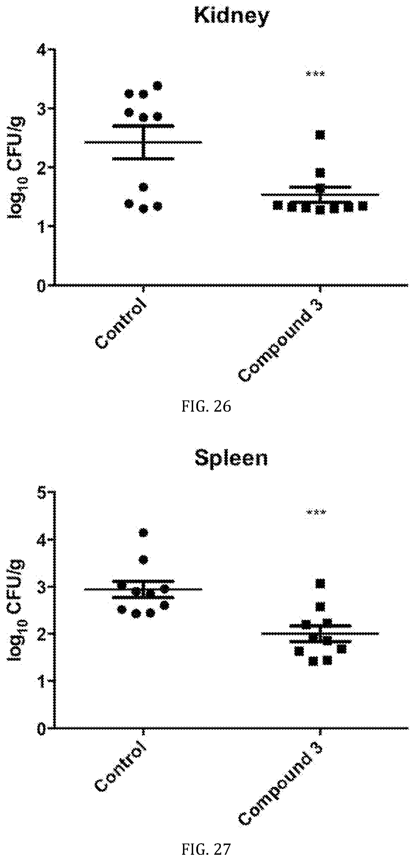

**Figure 26** Treatment of compound 3 reduce kidney bacterial load in BALB/c mice after Mu3 infection. The analysis shows that compound 3 can reduce bacterial infection by itself.
**Figure 27** Treatment of compound 3 reduce spleen bacterial load in BALB/c mice after Mu3 infection. The analysis shows that compound 3 can reduce bacterial infection by itself.

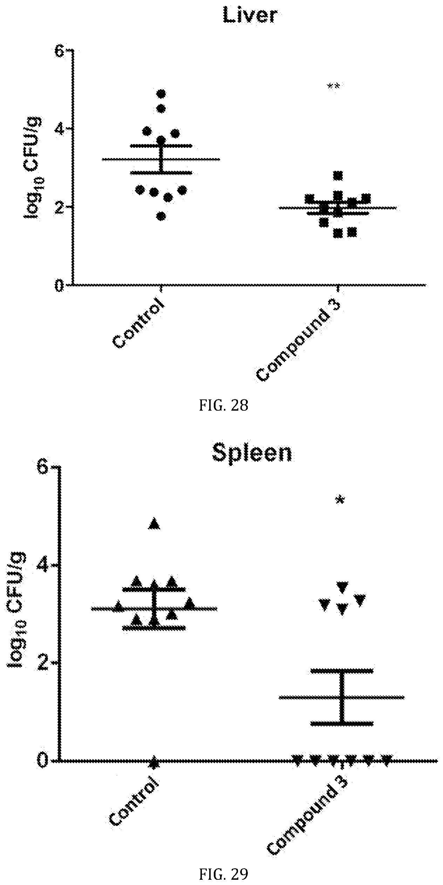

**Figure 28** Treatment of compound 3 reduce liver bacterial load in BALB/c mice after Mu3 infection. The analysis shows that compound 3 can reduce bacterial infection by itself.
**Figure 29** Treatment of compound 3 reduce spleen bacterial load in BALB/c mice after USA300 infection. The analysis shows that compound 3 can reduce bacterial infection by itself.

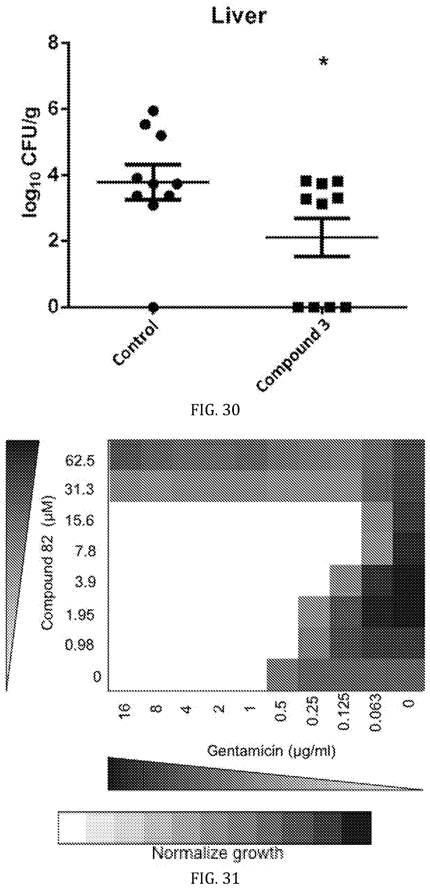

**Figure 30** Treatment of compound 3 reduce liver bacterial load in BALB/c mice after USA300 infection. The analysis shows that compound 3 can reduce bacterial infection by itself.
**Figure 31** Heat map plot showing normalized growth of checkerboard assay of compound 82 with Gentamicin against *L. monocytogenes.* The analysis shows that compound 82 has potentiating effect with gentamicin in inhibiting the growth of *L. monocytogenes* strain. Heat Plot shows the average growth from replicated experiments.

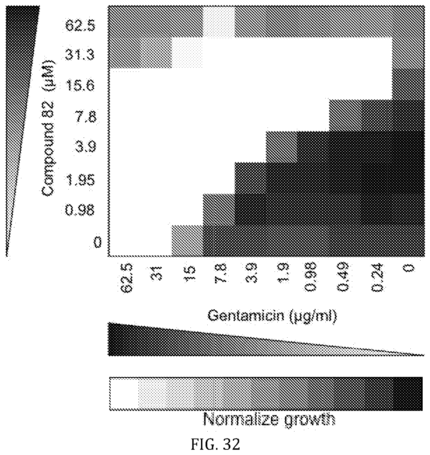

**Figure 32** Heat map plot showing normalized growth of checkerboard assay of compound 82 with Gentamicin against *E. faecalis.* The analysis shows that compound 82 has potentiating effect with gentamicin in inhibiting the growth of *E. faecalis* strain. Heat Plot shows the average growth from replicated experiments.

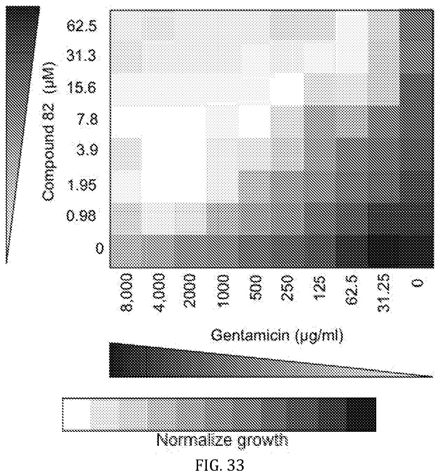

**Figure 33** Heat map plot showing normalized growth of checkerboard assay of compound 82 with Gentamicin against *E. coli.* The analysis shows that compound 82 has potentiating effect with gentamicin in inhibiting the growth of *E. coli* strain. Heat Plot shows the average growth from replicated experiments.

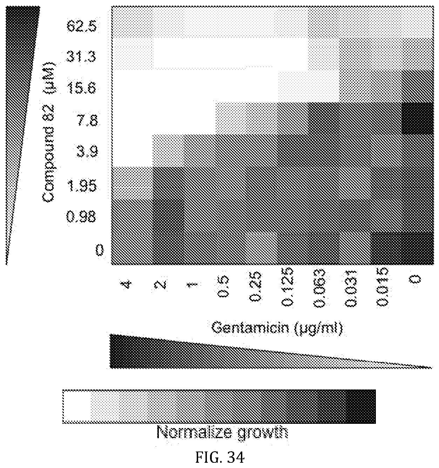

**Figure 34** Heat map plot showing normalized growth of checkerboard assay of compound 82 with Gentamicin against *S. pneumoniae.* The analysis shows that compound 82 has potentiating effect with gentamicin in inhibiting the growth of *S. pneumonia* strain. Heat Plot shows the average growth from replicated experiments.

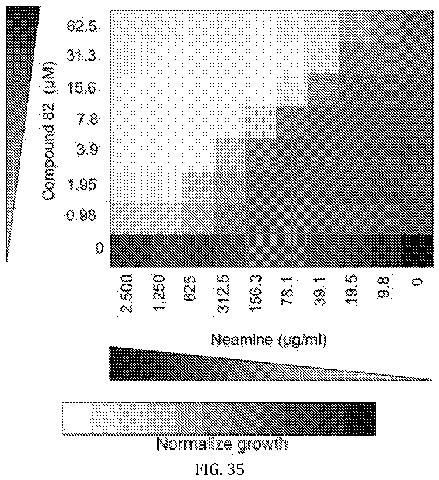

**Figure 35** shows a microdilution checkerboard analysis showing the combined effect of compound 82 with neamine. The analysis shows that compound 82 has potentiating effect with neamine in inhibiting the growth of *S. aureus* Mu3 strain. Heat Plot shows the average growth from replicated experiments.

### DETAILED DESCRIPTION OF THE INVENTION

### I. Definitions

Recitation of ranges of values herein are merely intended to serve as a shorthand method of referring individually to each separate value falling within the range, unless otherwise indicated herein, and each separate value is incorporated into the specification as if it were individually recited herein.

Use of the term "about" is intended to describe values either above or below the stated value in a range of approx. +/- 10%; in other forms the values may range in value either above or below the stated value in a range of approx. +/- 5%; in other forms the values may range in value either above or below the stated value in a range of approx. +/- 2%; in other forms the values may range in value either above or below the stated value in a range of approx. +/- 1%. The preceding ranges are intended to be made clear by context, and no further limitation is implied. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

"Aerosol" as used herein refers to any preparation of a fine mist of particles, which can be in solution or a suspension, whether or not it is produced using a propellant.

The term "alkyl" refers to the radical of saturated aliphatic groups (i.e., an alkane with one hydrogen atom removed), including straight-chain alkyl groups, branched-chain alkyl groups, cycloalkyl (alicyclic) groups, alkyl-substituted cycloalkyl groups, and cycloalkyl-substituted alkyl groups.

"Amphiphilic" as used herein refers to a molecule combining hydrophilic and lipophilic (hydrophobic) properties.

The term "carrier" refers to a diluent, adjuvant, excipient, and/or vehicle with which the compound and/or antibiotic are administered.

"Cleaning formulation", as used herein, means a composition suitable for application to a surface for removing dirt, oils, and the like, for disinfecting, or a combination thereof. Cleaning compositions can be antibacterial, antimicrobial, or both. Cleaning compositions can be suitable for use on the human skin meaning that all components of the composition are present at concentrations that cause no significant signs of irritation when applied to human skin. As used herein, "significant signs of irritation" include erythema, redness, and/or swelling at the site of injection at the site of application, necrosis at the site of application, exfoliative dermatitis at the site of application, and severe pain that prevents daily activity and/or requires medical attention or hospitalization.

A "continuous phase" refers to the liquid in which solids are suspended or droplets of another liquid are dispersed, and is sometimes called the external phase. This also refers to the fluid phase of a colloid within which solid or fluid particles are distributed. If the continuous phase is water (or another hydrophilic solvent), water- soluble or hydrophilic drugs will dissolve in the continuous phase (as opposed to being dispersed). In a multiphase formulation (e.g., an emulsion), the discreet phase is suspended or dispersed in the continuous phase.

A "cream" is a viscous liquid or semi-solid emulsion of either the "oil-in-water" or "water-in-oil type".

An "emulsion" is a composition containing a mixture of non-miscible components homogenously blended together.

"Gel" as used herein is a colloid in which the dispersed phase has combined with the continuous phase to produce a semisolid material, such as jelly.

"Hydrophilic" as used herein refers to substances that have strongly polar groups that readily interact with water.

"Hydrophobic" as used herein refers to substances that lack an affinity for water; tending to repel and not absorb water as well as not dissolve in or mix with water.

"Lipophilic" as used herein refers to compounds having an affinity for lipids.

A "lotion" is a low- to medium-viscosity liquid formulation.

"Oil" as used herein refers to a composition containing at least 95% wt. of a lipophilic substance.

An "ointment" is a semisolid preparation containing an ointment base and optionally one or more active agents.

"Parenteral administration", as used herein, means administration by any method other than through the digestive tract or non-invasive topical or regional routes.

The term "pharmaceutically acceptable", as used herein with regard to pharmaceutical compositions, means approved by a regulatory agency of the Federal or a state government or listed in the U.S. Pharmacopeia or other generally recognized pharmacopeia for use in animals and/or in humans.

"Pharmaceutically acceptable salt", as used herein, refers to derivatives of the compounds defined herein, wherein the parent compound is modified by making acid or base salts thereof.

As used herein, "treatment" or "treating" refers to arresting or inhibiting, or attempting to arrest or inhibit, the development or progression of an infection and/or causing, or attempting to cause, the reduction, suppression, regression, or remission of an infection and/or a symptom thereof. As would be understood by those skilled in the art, various clinical and scientific methodologies and assays may be used to assess the development or progression of an infection, and similarly, various clinical and scientific methodologies and assays may be used to assess the reduction, regression, or remission of an infection or its symptoms. "Treatment" refers to both therapeutic treatment and prophylactic or preventative measures. Those in need of treatment include those already with the infection as well as those prone to have the infection or those in whom the infection is to be prevented. In at least some forms, the infection being treated is *Staphylococcus aureus, Pseudomonas aeruginosa, Listeria monocytogenes, Burkholderia cepacia, Escherichia coli, Enterococcus faecalis, Streptococcus pneumoniae* infection. In other forms, the infection being treated is a bacterial infection.

As used herein, the term "subject" refers to an animal. Typically, the terms "subject" and "patient" may be used interchangeably herein in reference to a subject. As such, a "subject" includes a human that is being treated for a microbial infection as a patient.

The terms "effective amount" and "therapeutically effective amount," used interchangeably, as applied to the compounds, antibiotics, and pharmaceutical compositions described herein, mean the quantity necessary to render the desired therapeutic result. For example, an effective amount is a level effective to treat, cure, or alleviate the symptoms of an infection for which the composition and/or antibiotic, or pharmaceutical composition, is/are being administered. Amounts effective for the particular therapeutic goal sought will depend upon a variety of factors including the infection being treated and its severity and/or stage of development/progression; the bioavailability and activity of the specific compound and/or antibiotic, or pharmaceutical composition, used; the route or method of administration and introduction site on the subject; the rate of clearance of the specific composition and other pharmacokinetic properties; the duration of treatment; inoculation regimen; drugs used in combination or coincident with the specific composition; the age, body weight, sex, diet, physiology and general health of the subject being treated; and like factors well known to one of skill in the relevant scientific art. Some variation in dosage will necessarily occur depending upon the condition of the subject being treated, and the physician or other individual administering treatment will, in any event, determine the appropriate dosage for an individual patient.

### II. COMPOSITIONS

High throughput screening provides a good opportunity for discovery of new small molecules as antibacterial agents.

Through sequential screening processes, a chemical compound library to a lead compound for future chemical optimization for antibiotics potentiators was identified. As shown below, compound 13 demonstrated the potentiating effect in combination with aminoglycoside, gentamicin, kanamycin, amikacin, neomycin, spectinomycin and neamine, which are commonly used in clinical settings. Furthermore, these compounds (at a concentration of around 50 µM) were shown to reduce the MIC of the antibiotic below the corresponding clinical breakpoint, thus making an ineffective antibiotic usable again for MRSA treatment.

In some forms, the compound potentiates the therapeutic effects of one or more antimicrobial agents by at least 2 fold when they are coadministered. Potentiation can be determined *in vitro,* as described in the Examples, by measuring bacterial growth inhibition in the presence of the disclosed compounds when administered with the antibiotic. In some forms, the compound is effective to inhibit bacterial growth by at least 30%, preferably at least 40%, and more preferably at least 60% more than the inhibition obtained with gentamycin alone (gentamycin + DMSO control). In the other forms the compounds have antivirulence properties, measured as repression in *hla* promoter lux reporter assay when compared to gentamycin + DMSO control. Suppressions of pigment production were also observed in some pigment producing bacteria in the presence of the compounds.

It is within the abilities for those of ordinary skill in the art to use the data obtained from cell culture assays and animal studies to formulate a dosage range for use in humans. The dosage of such compounds lies preferably within a range of circulating concentrations that include the ED₅₀ with little or no toxicity. The dosage can vary within his range depending upon the dosage form employed and the route of administration. For any compound used in the disclosed methods, the therapeutically effective dose can be estimated initially from cell culture assays. A dose can be formulated in animal models to achieve a circulating plasma concentration range that includes the IC₅₀ (the concentration of the test compound that achieves a half-maximal inhibition of symptoms) as determined in cell culture. Such information can be used to more accurately determine useful doses in humans. Levels in plasma can be measured, for example, by high performance liquid chromatography (HPLC). In general, the dose equivalent of a modulator is from about 1 ng/kg to 10 mg/kg for a typical subject.

Accordingly, compounds with antibacterial activity are disclosed which can be used in an effective amount in a composition to prevent, treat or ameliorate the symptoms resulting from a microbial infection. In some forms, the compounds potentiate the antimicrobial effects of antibiotics. In another form, the compounds possess antivirulence properties.

The compounds disclosed herein can be utilized in combination with other bioactive agents, for example, antibiotics during treatment. Such combination treatments may further enhance the therapeutic effects of treatment with the disclosed compound(s). The disclosed compounds can provide additive, synergistic or antibiotic potentiating effects with antibiotics.

### A. Compounds

The compounds according to the following general formulas are disclosed herein but are not part of the claimed invention.

The disclosed compounds are represented by the following generic Formula I: 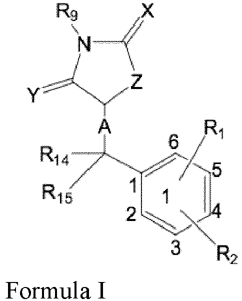
where X and Y are independently O, S, or NR₁₀;
where Z is O, S, CR₁₁R₁₂, or NR₁₃;
where R₁, R₂, R₉, R₁₀, R₁₁, R₁₂, R₁₃, R₁₄, and R₁₅, are independently absent, hydrogen, -C(O)R₁₈, -C(W)NR₁₉R₂₀, or substituted or unsubstituted alkyl, alkenyl, alkynyl, alkoxy, cycloalkyl, heterocyclyl, cycloalkenyl, heterocycloalkenyl, aryl, heteroaryl, aroxy, arylalkyl, heteroalkyl, alkylaryl, halogen, alkylheteroaryl, -NR₁₆R₁₇; wherein R₁, and R₁₇ are independently hydrogen, substituted or unsubstituted alkyl. alkenyl, alkynyl, cycloalkyl, heterocyclyl, cycloalkenyl, heterocycloalkenyl, aryl, heteroaryl, arylalkyl, heteroalkyl, alkylaryl, or alkylheteroaryl;
where R₁₈ is hydrogen, hydroxyl, or substituted or unsubstituted alkyl, alkenyl, alkynyl, cycloalkyl, alkoxy, heterocyclyl, cycloalkenyl, heterocycloalkenyl, aryl, aroxy, heteroaryl, arylalkyl, heteroalkyl, alkylaryl, or alkylheteroaryl;
where W is O, S, or NR₂₁;
where R₁₉, R₂₀, and R₂₁ are independently hydrogen or substituted or unsubstituted alkyl, alkenyl, alkynyl, cycloalkyl, heterocyclyl, cycloalkenyl, heterocycloalkenyl, aryl, heteroaryl, arylalkyl, heteroalkyl, alkylaryl, or alkylheteroaryl;
where A is a double bond or single bond; and
where R₁₅ is absent when A is a double bond.

In some forms, the compound can be 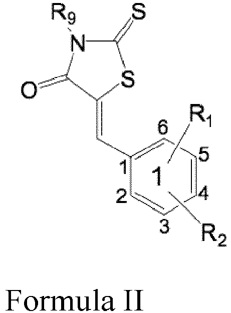
where R₁ is -O-R₄, -NR₅R₈, or -CH₂-R₅; R₂ is halogen, absent, -O-CH₃, or -O-CH₂-CH₃; R₉ is -COOH, -CH₂-COOH, -CH₂-O-CH₃, or -CH₂-CH₂-O-CH₃; R₄ is -CH₂- R₅ or -CO-R₅; R₅ is 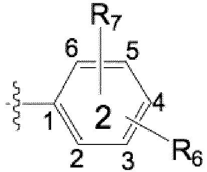
R₆ is a halogen or absent; and R₇ is halogen or absent.

In some forms of the compound, R₁ is at a position selected from the group consisting of 2, 3, 4, 5, and 6. In some forms, R₆ is at position 4. In some forms, R₇ is at position 2 or 6. In some forms, R₂ is at position 3, 4, or 5. In some forms, R₆ is at position 4, R₇ is at position 2 or 6, and R₂ is at position 3 or 5.

In some forms, the disclosed compound is 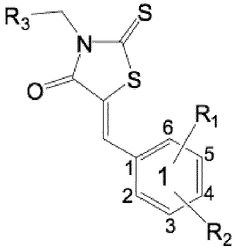
Formula IIA where R₁ is -O-R₄, -NR₅R₈, or -CH₂-R₅; R₂ is halogen, absent, -O-CH₃, or -O-CH₂-CH₃; R₃ is -COOH or -CH₂-COOH, R₄ is - CH₂-R₅ or -CO-R₅; R₅ is 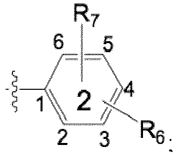
R₆ is halogen or absent; and R₇ is halogen or absent.

In some forms, R₁ is at a position selected from the group consisting of 2, 3, 4, 5, and 6. In some forms, R₆ is at position 4. In some forms, R₇ is at position 2 or 6. In some forms, R₂ is at position 3, 4, or 5. In some forms, R₆ is at position 4, R₇ is at position 2 or 6, and R₂ is at position 3 or 5.

In some forms, R₁ is -O-R₄ and is at position 3; R₂ is not present; R₃ is -COOH; R₄ is -CH₂-R₅; R₅ is 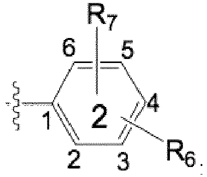 R₆ is -Cl and is at position 4; and R₇ is -Cl and is at position 2.

In some forms, R₁ is -O-R₄ and is at position 3; R₂ is not present; R₃ is -COOH; R₄ is -CH₂-R₅; R₅ is 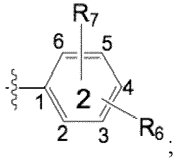 R₆ is -Cl; and R₇ is absent.

In some forms, R₁ is -O-R₄, and is at position 4; R₂ is not present; R₃ is -COOH;
R₄ is -CH₂-R₅; R₅ is 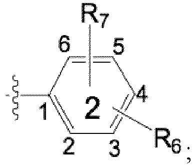
R₆ is -Cl; and R₇ is absent.In some forms, R₁ is -O-R₄, and is at position 4; R₂ is -O-CH₂-CH₃; R₃ is - COOH;
R₄ is -CH₂-R₅; R₅ is 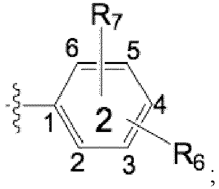
R₆ is -Cl; and R₇ is absent.

These and other groups are listed in Table 8

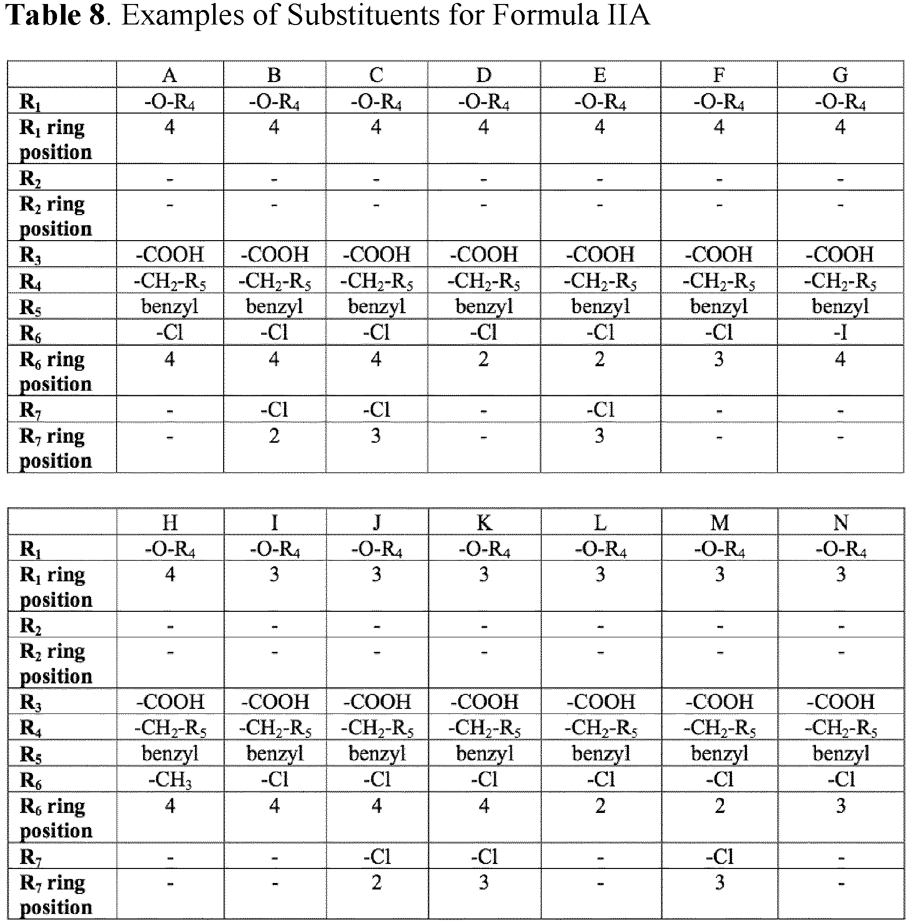

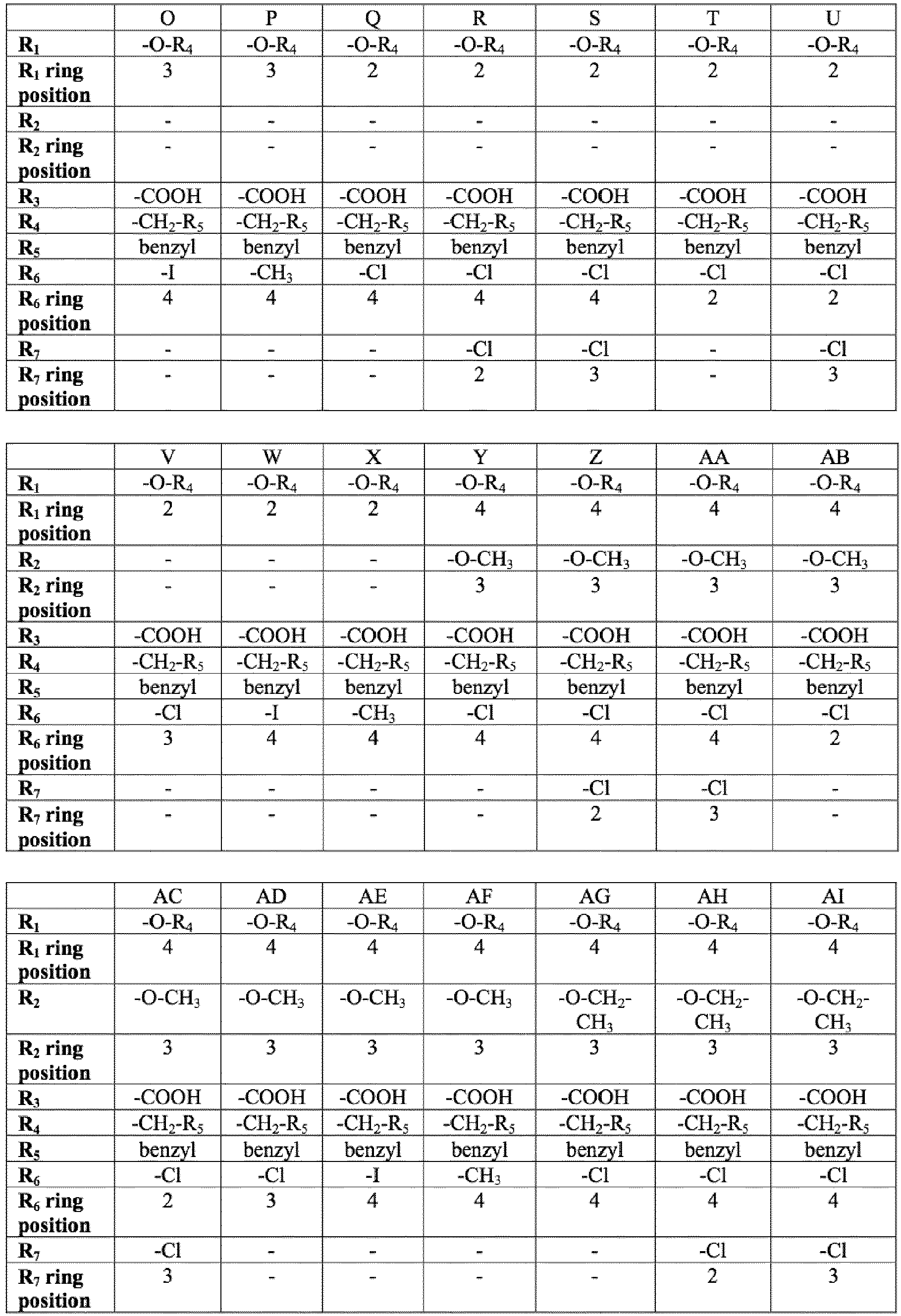

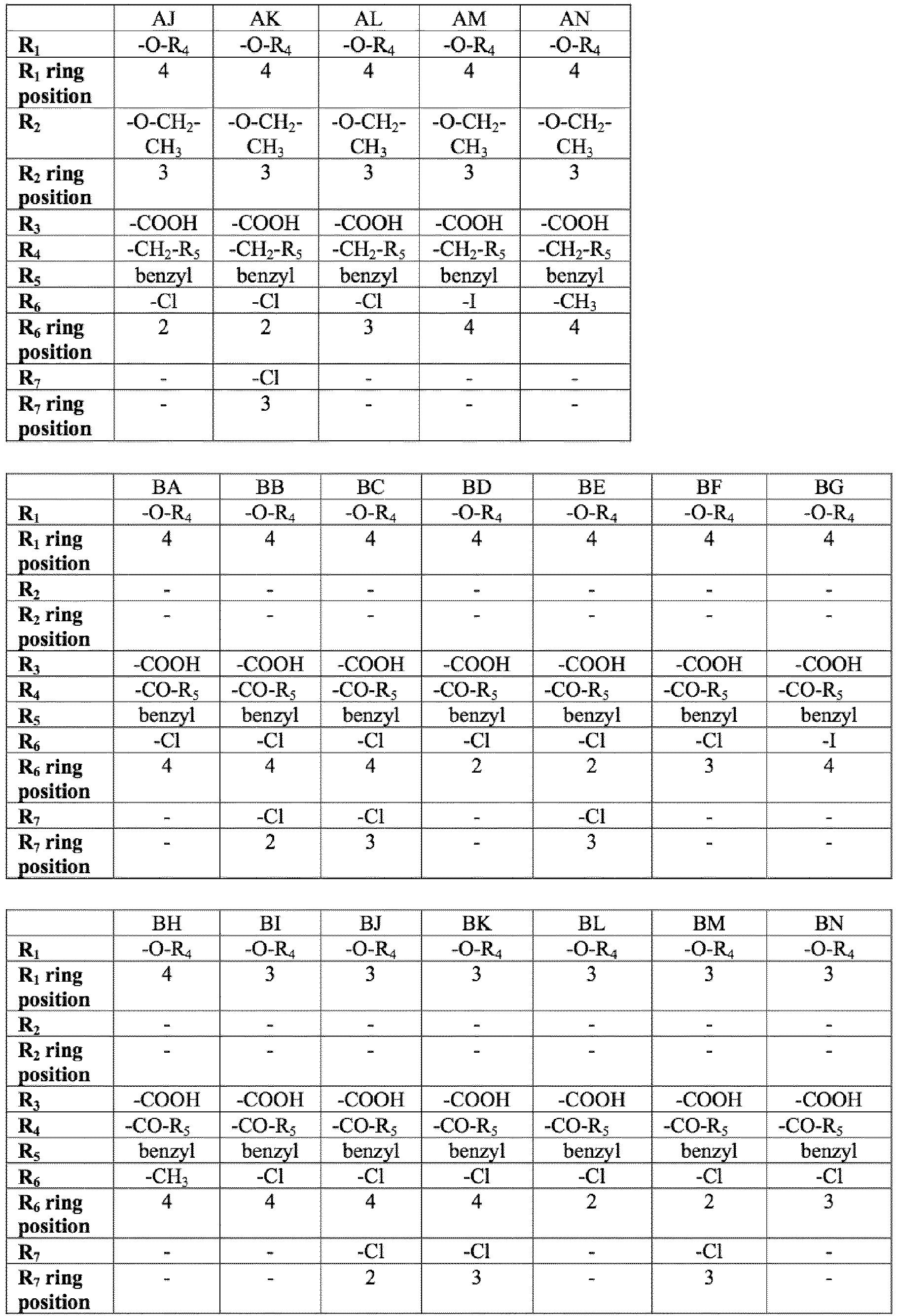

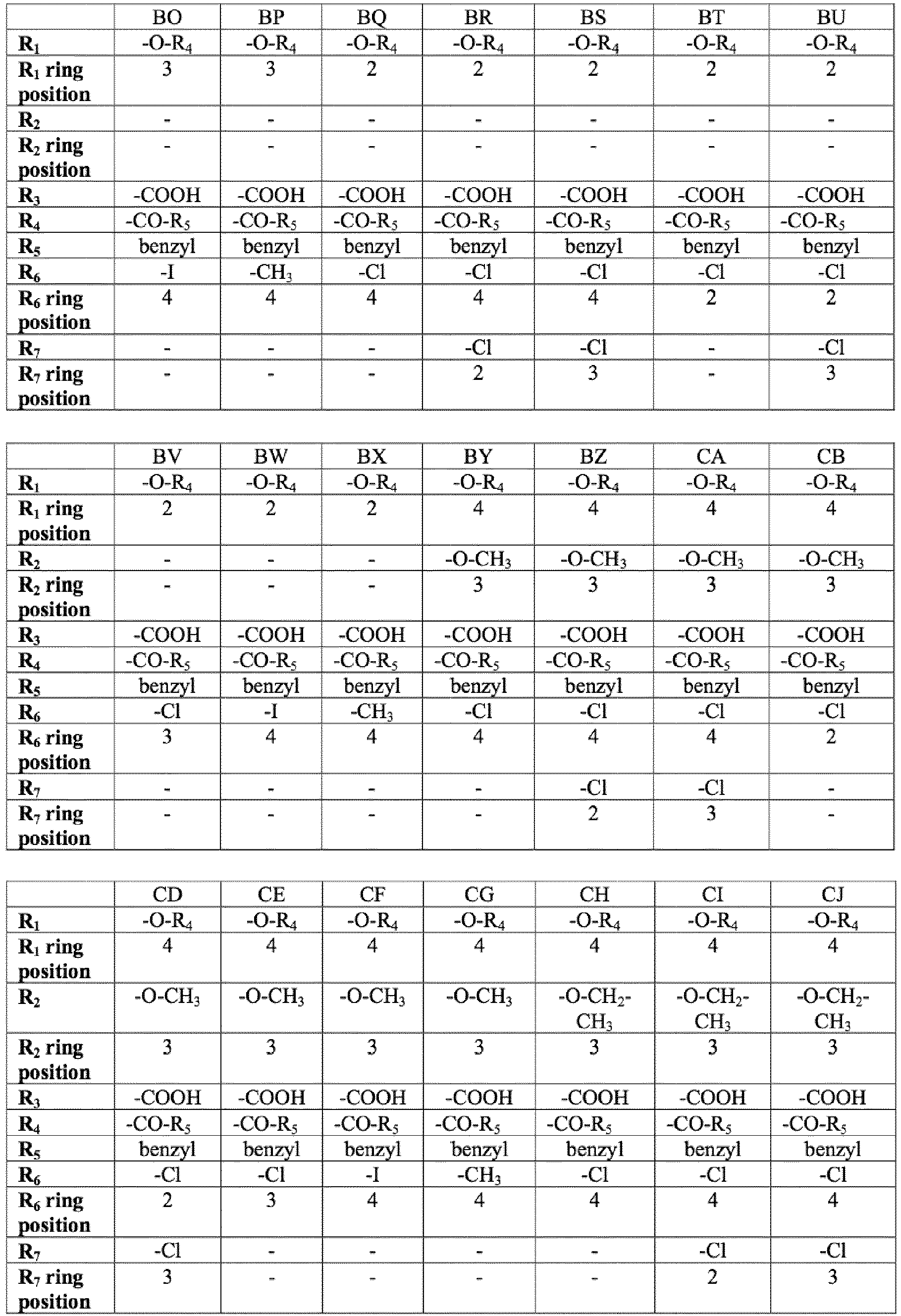

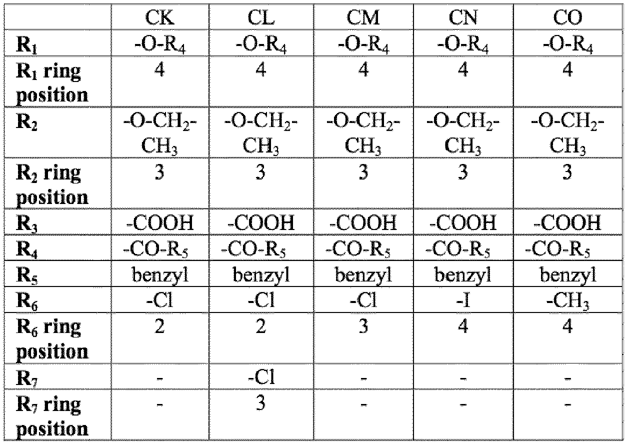

All combinations of R₁-R₇ disclosed above are contemplated.

In some forms, X and Y are independently O, S, or NR₁₀; Z is O, S, CR₁₁R₁₂, or NR₁₃; R₁, R₂, R₉, R₁₀, R₁₁, R₁₂, R₁₃, R₁₄, and R₁₅, are independently absent, hydrogen, substituted or unsubstituted alkyl, alkenyl, alkynyl, alkoxy, cycloalkyl, heterocyclyl, cycloalkenyl, heterocycloalkenyl, aryl, heteroaryl, aroxy, arylalkyl, heteroalkyl, alkylaryl, halogen, alkylheteroaryl, -NR₁₆R₁₇; wherein R₁₆ and R₁₇ are independently hydrogen, substituted or unsubstituted alkyl, alkenyl, alkynyl, cycloalkyl, heterocyclyl, cycloalkenyl, heterocycloalkenyl, aryl, heteroaryl, arylalkyl, heteroalkyl, alkylaryl, alkylheteroaryl, - C(O)R₁₈, or -C(W)NR₁₉R₂₀; R₁₈ is hydrogen, hydroxyl, substituted or unsubstituted alkyl, alkenyl, alkynyl, cycloalkyl, alkoxy, heterocyclyl, cycloalkenyl, heterocycloalkenyl, aryl, aroxy, heteroaryl, arylalkyl, heteroalkyl, alkylaryl, or alkylheteroaryl; W is O, S, NR₂₁; R₁₉, R₂₀, and R₂₁ are independently hydrogen, substituted or unsubstituted alkyl, alkenyl, alkynyl, cycloalkyl, heterocyclyl, cycloalkenyl, heterocycloalkenyl, aryl, heteroaryl, arylalkyl, heteroalkyl, alkylaryl, or alkylheteroaryl; A is a double bond or single bond; and when A is a double bond, R₁₅ is absent.

In some forms of Formula II, R₁ can be -O-R₄ or -NR₅R₈ or -CH₂-R₅; R₂ can be halogen or is absent or -O-CH₃ or -O-CH₂-CH₃; R₉ can be - COOH or -CH₂-COOH or - CH₂-O-CH₃ or -CH₂-CH₂-O-CH₃; R₄ can be -CH₂-R₅ or -CO-R₅; R₅ can be

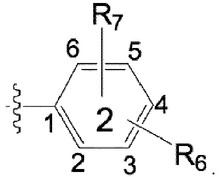

R₆ can be a halogen or is absent; R₇ can be halogen or is absent. In some forms, R₁ is at position 3 or 5; R₁ is at position 4; R₁ is at position 2 or 6; in some forms, R₆ is at position 4. In some forms, R₇ is at position 2 or 6. In some forms, R₂ is at position 3, 4, or 5.

All combinations of R₁-R₇ disclosed above are contemplated.

In some forms the disclosed compounds are represented by the Formula IIA:

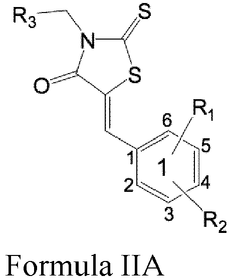

In these forms, R₁ can be -O-R₄ or -NR₅R₈ or -CH₂-R₅, R₂ can be a halogen, is absent or it can be -O-CH₃ or -O-CH₂-CH₃; R₃ can be - COOH or -CH₂-COOH, R₄ can be -CH₂-R₅ or -CO-R₅, R₅ can be

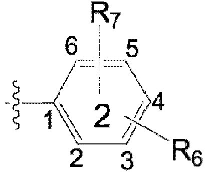

In some forms, R₆ is halogen or absent. In some forms, R₇ is halogen or absent. In some forms, R₁ is at position 3 or 5. In some forms, R₁ is at position 4. In some forms,

R₁ is at position 2 or 6. In some forms, R₆ is at position 4. In some forms, R₇ is at position 2 or 6. In some forms, R₂ is at position 3, 4, or 5.

All combinations of R₁-R₇ disclosed above are contemplated.

The structures and chemical names of the claimed compounds which are part of the claimed invention are shown below.

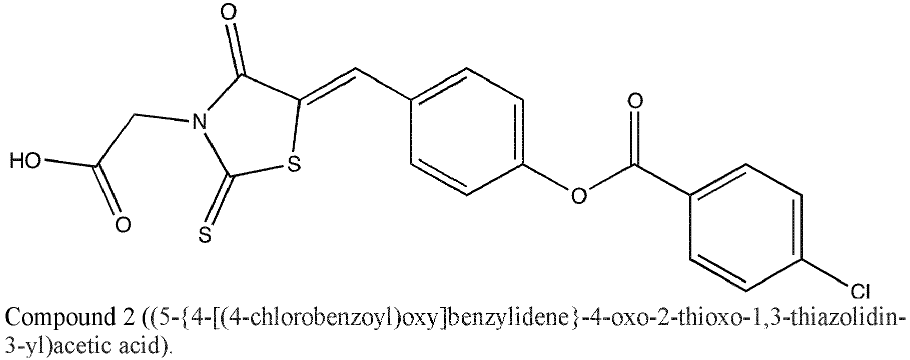

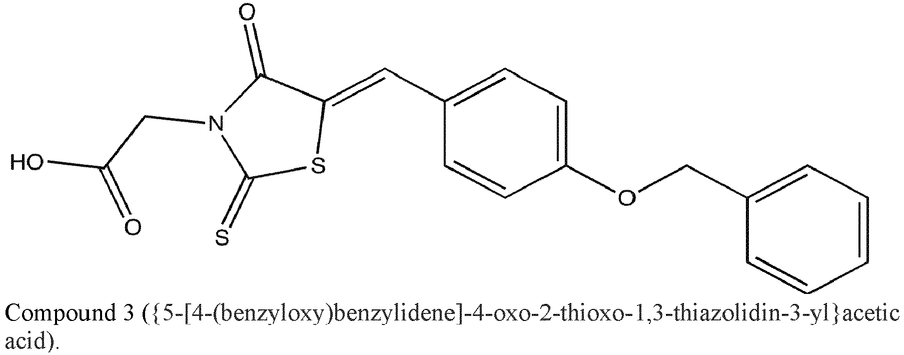

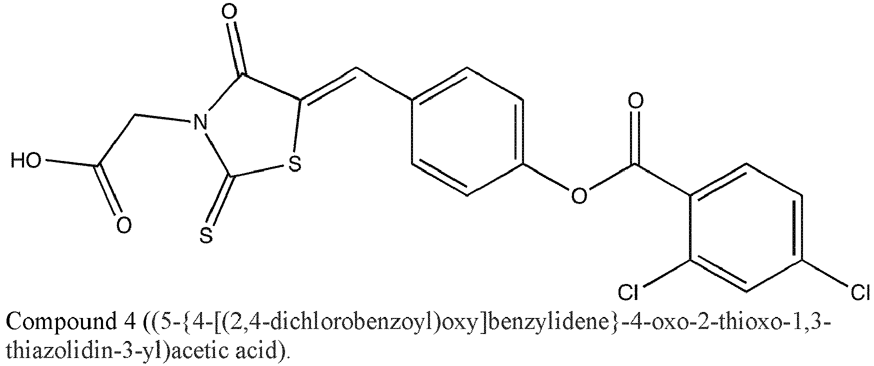

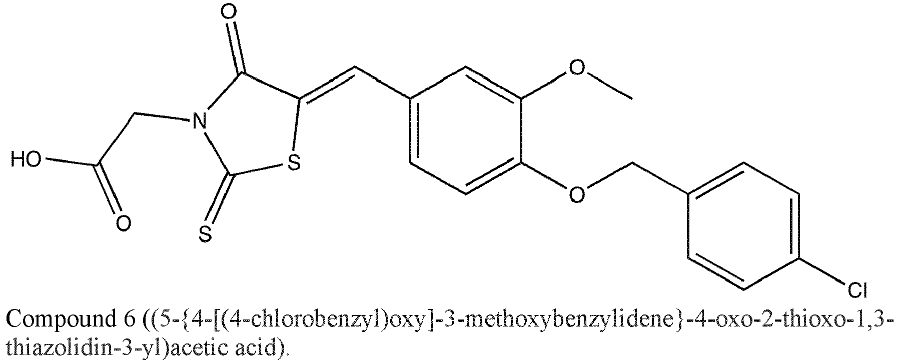

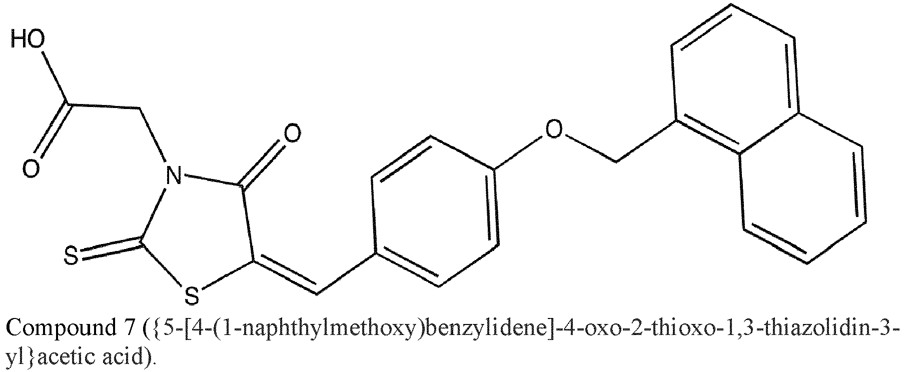

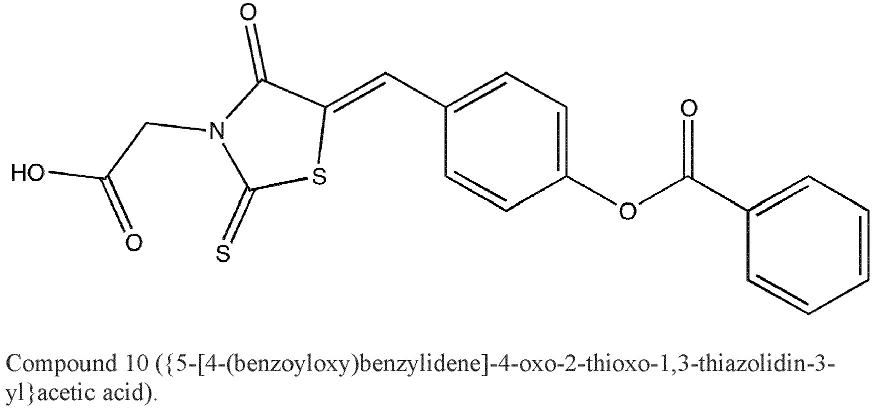

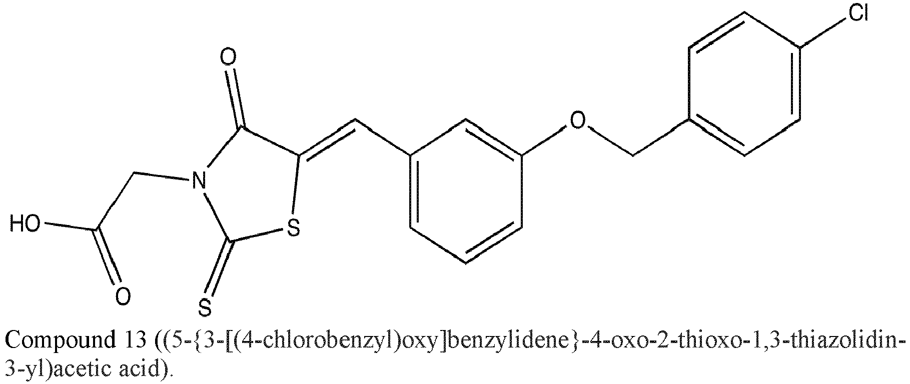

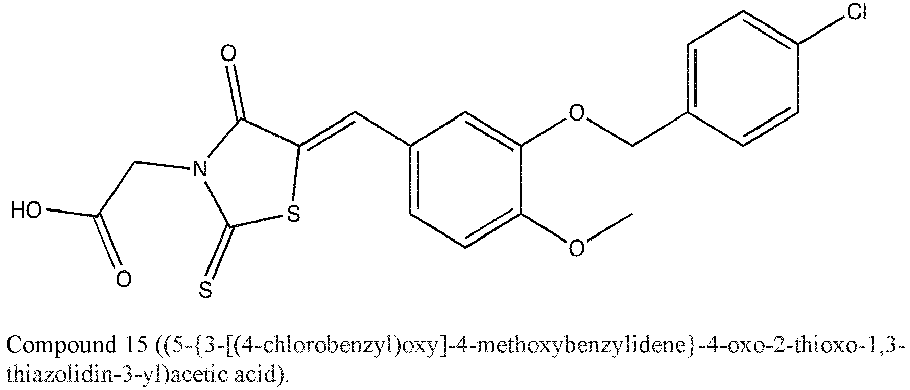

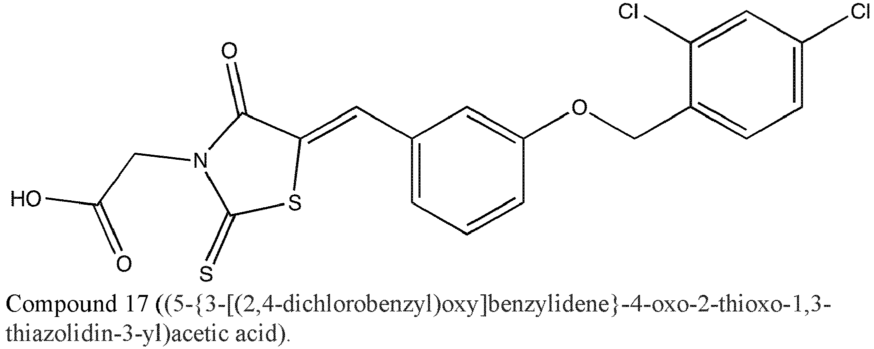

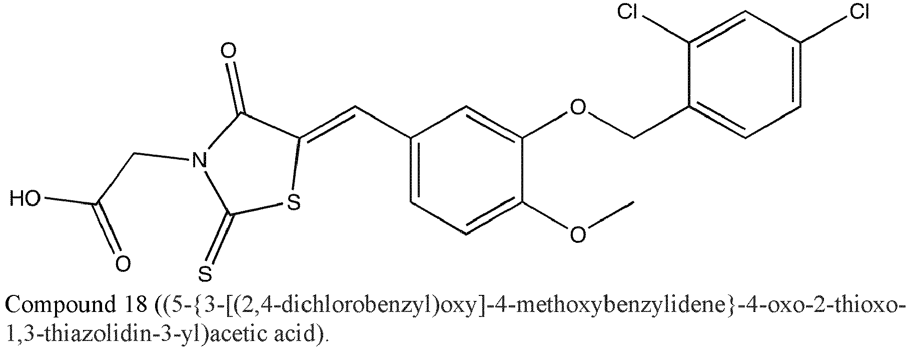

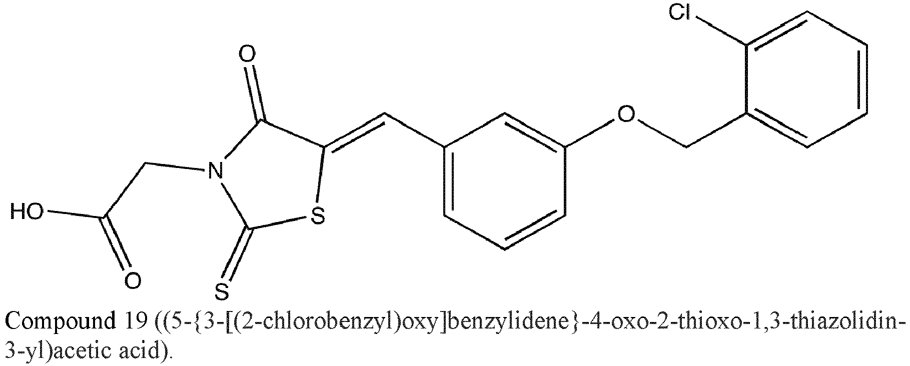

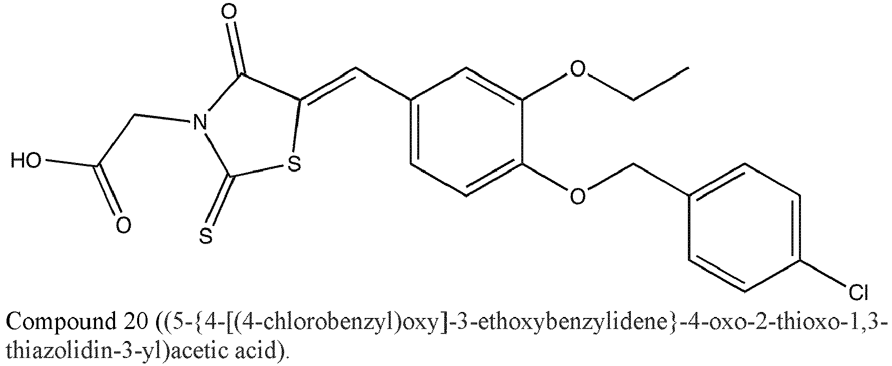

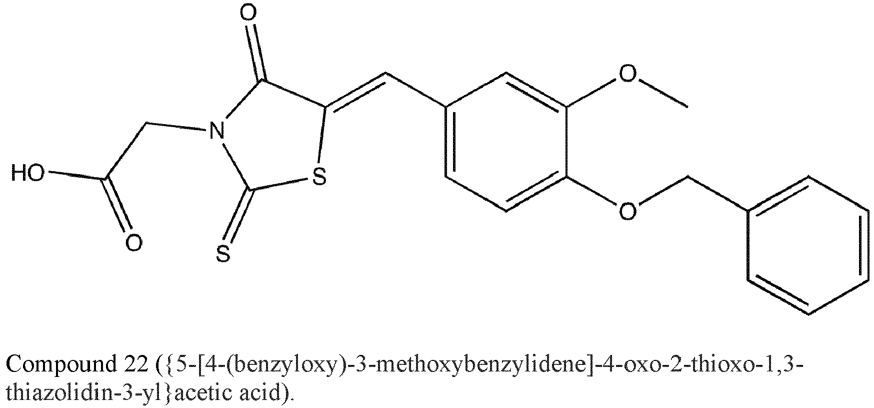

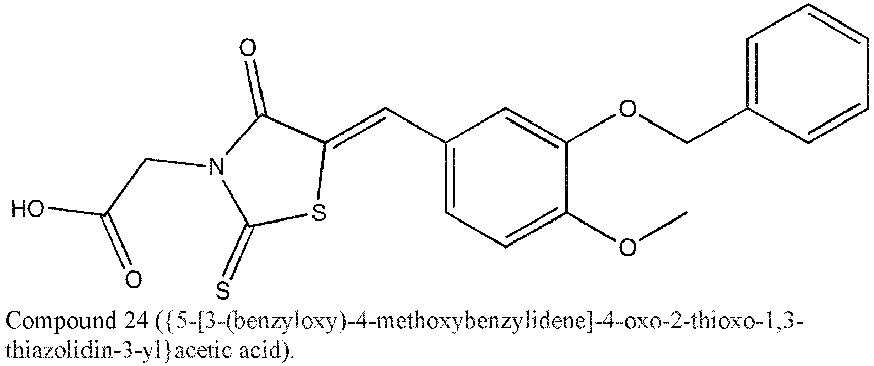

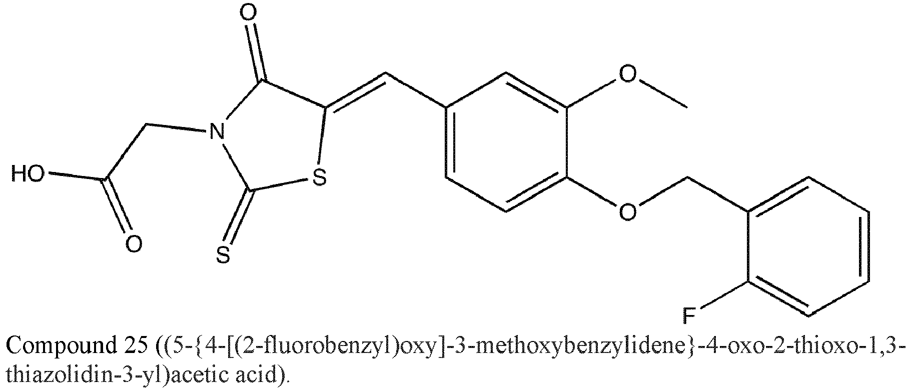

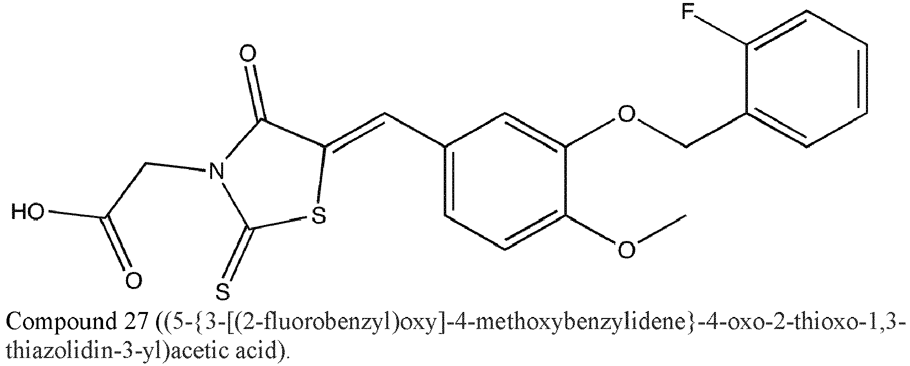

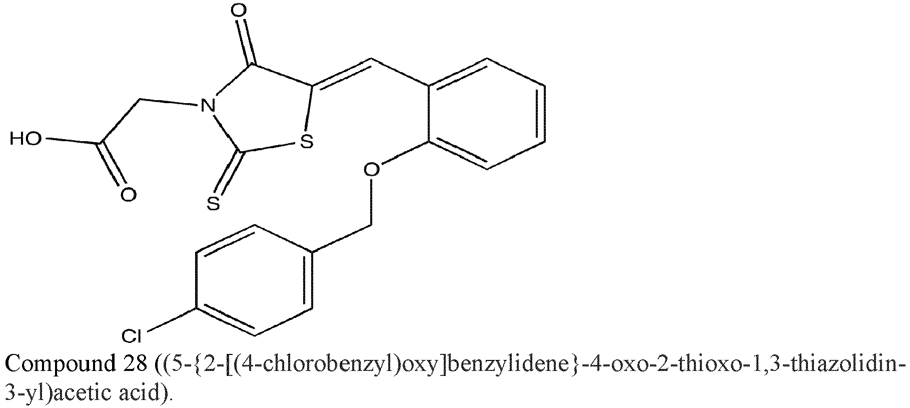

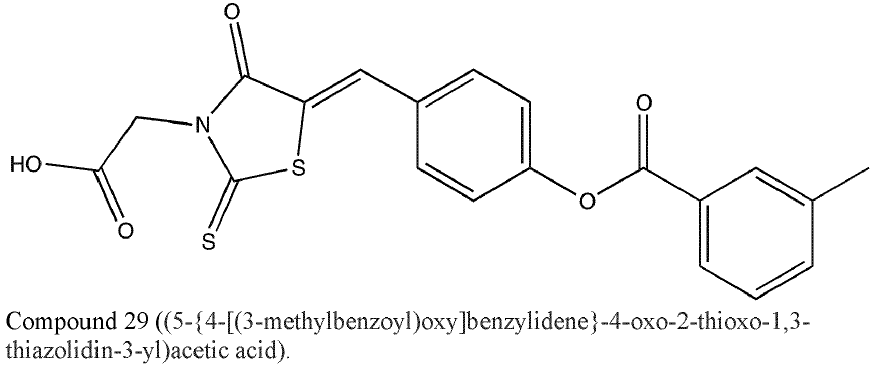

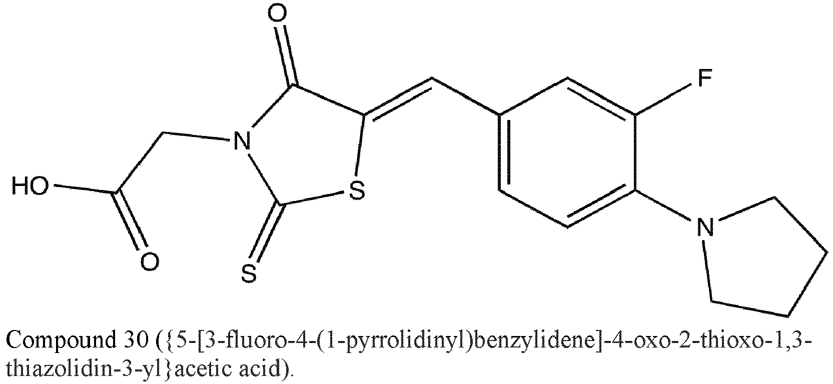

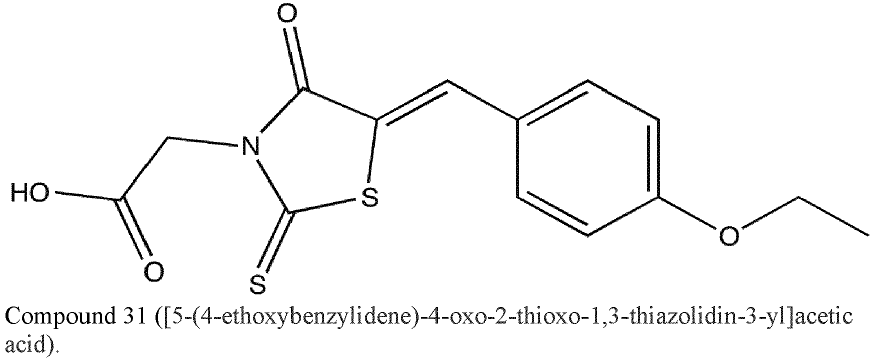

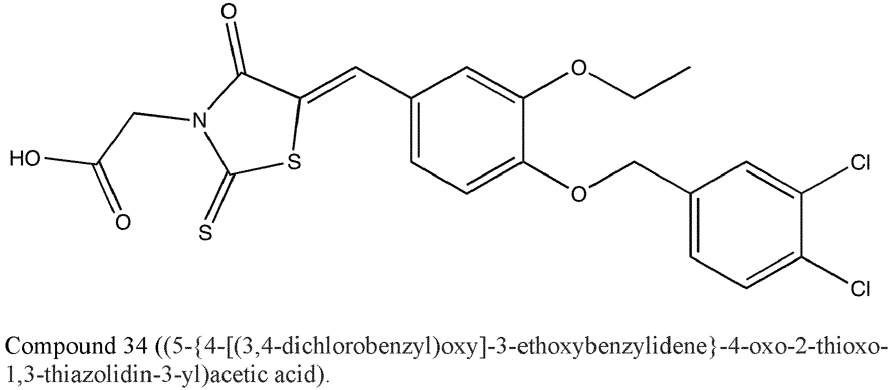

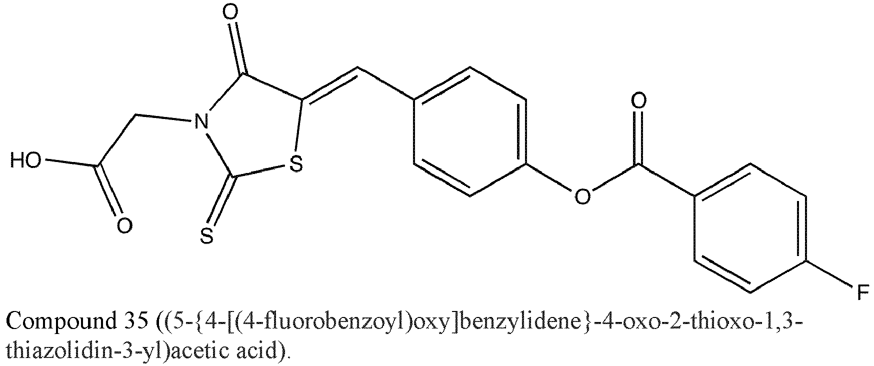

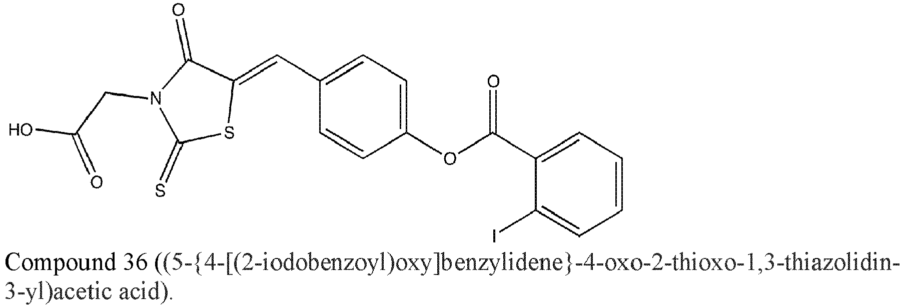

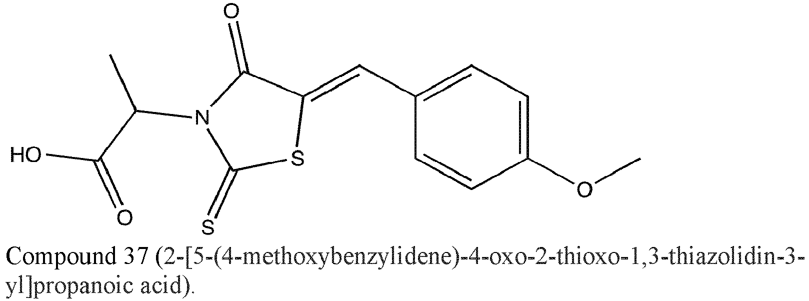

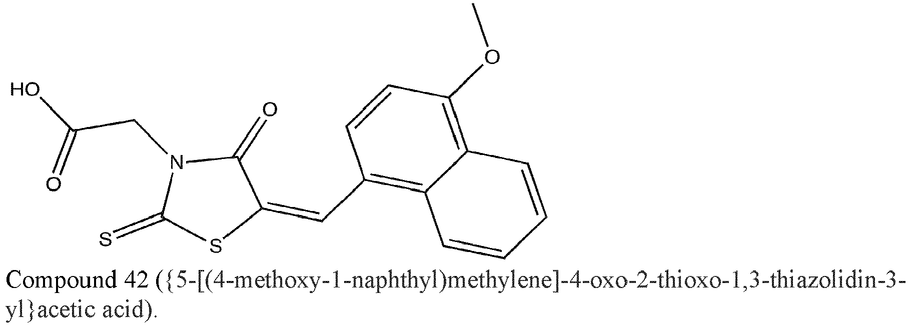

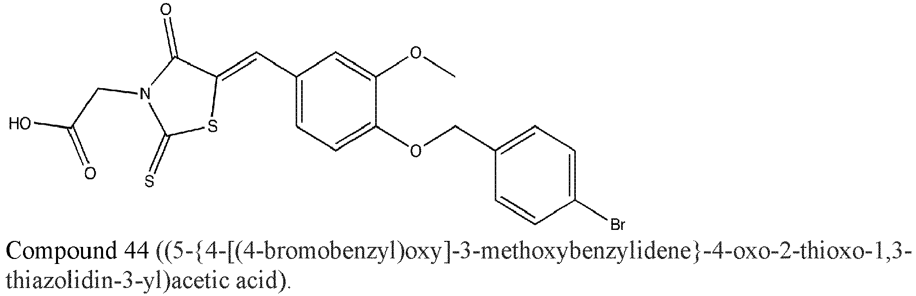

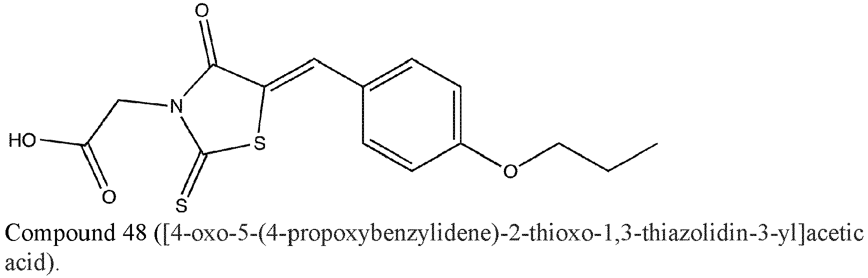

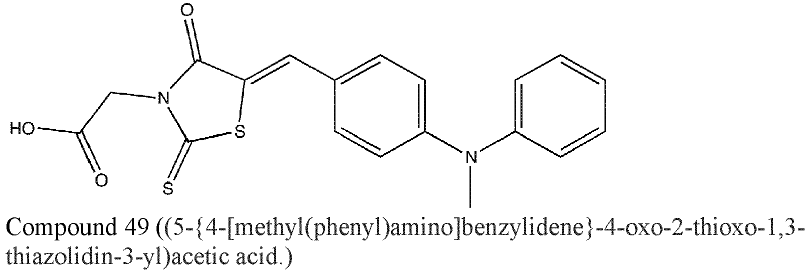

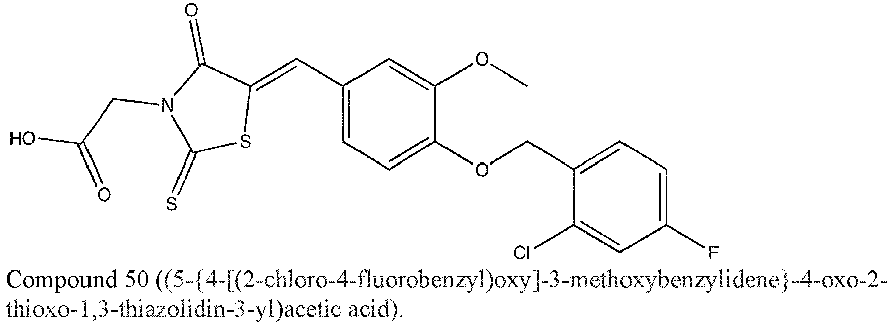

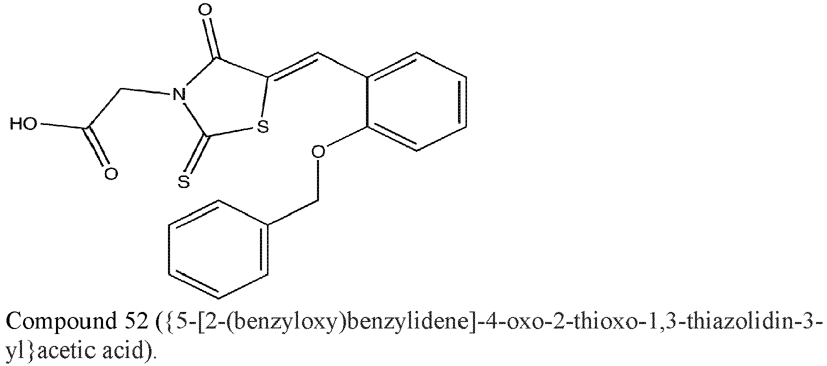

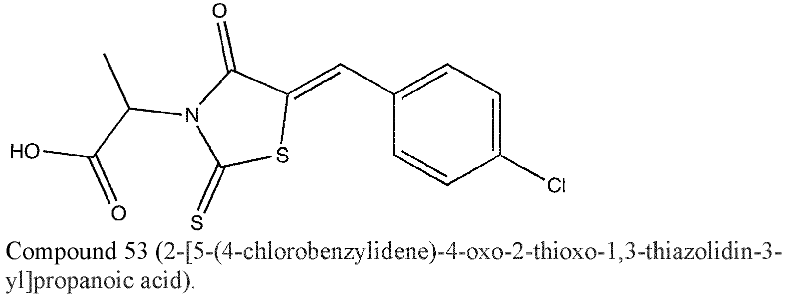

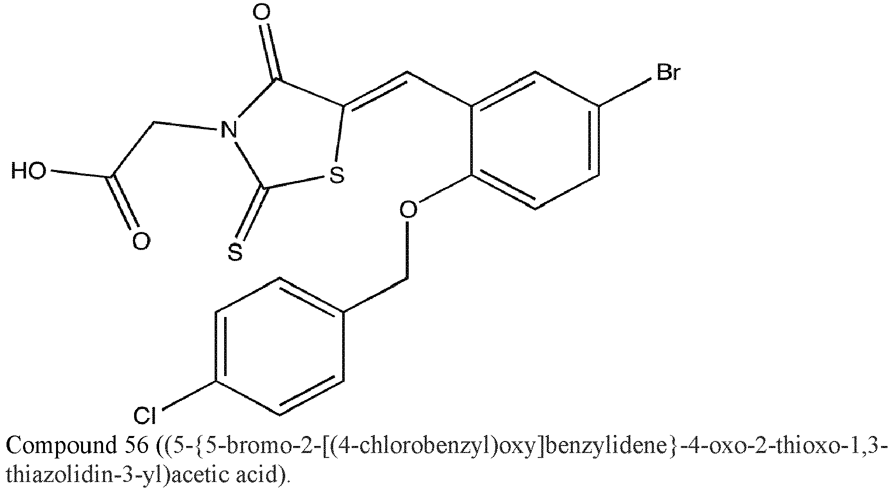

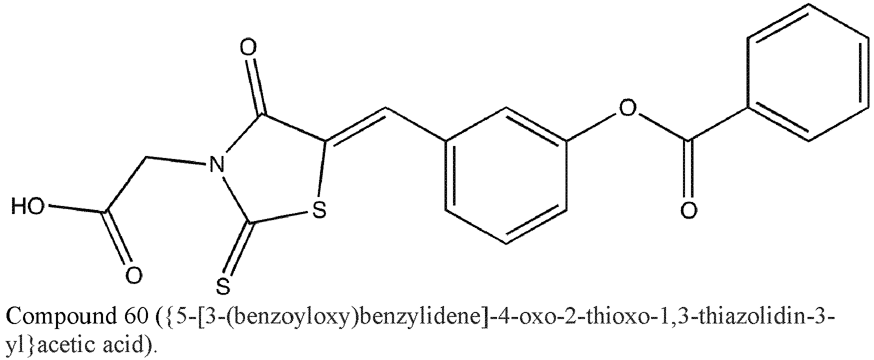

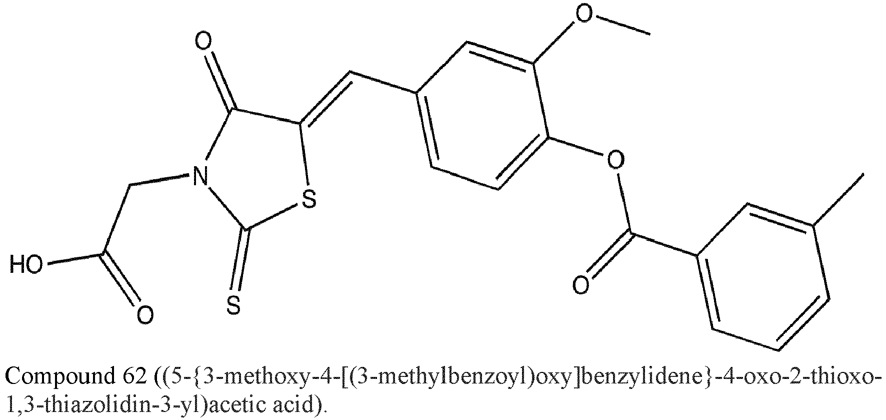

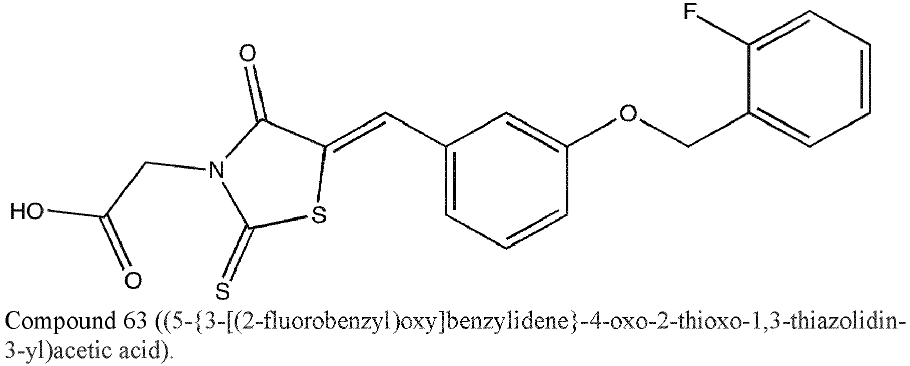

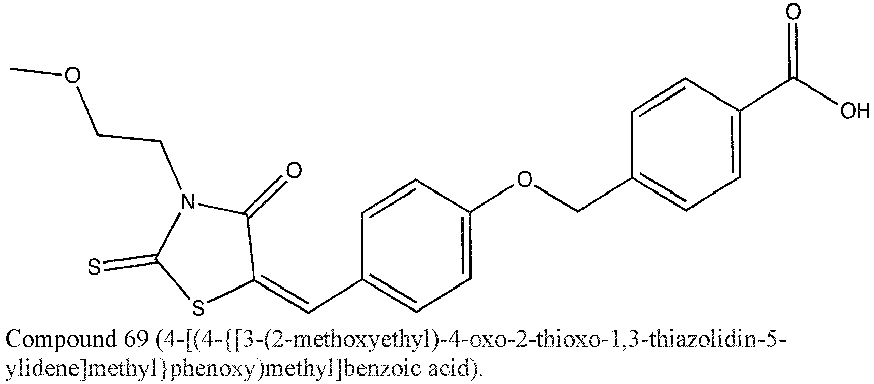

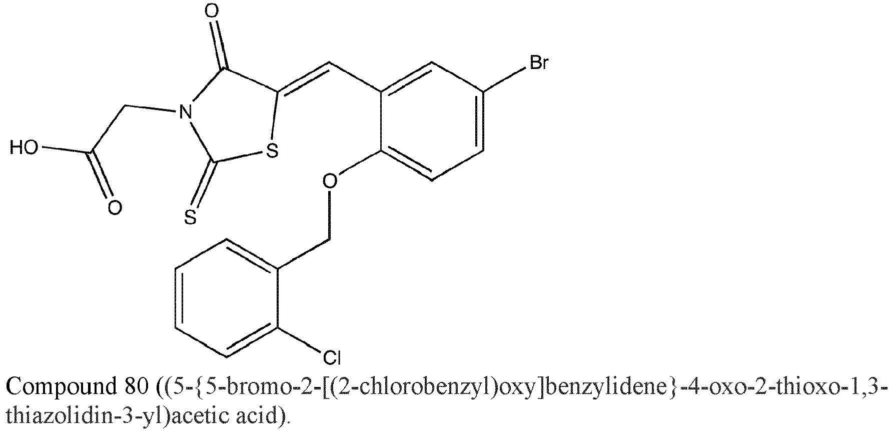

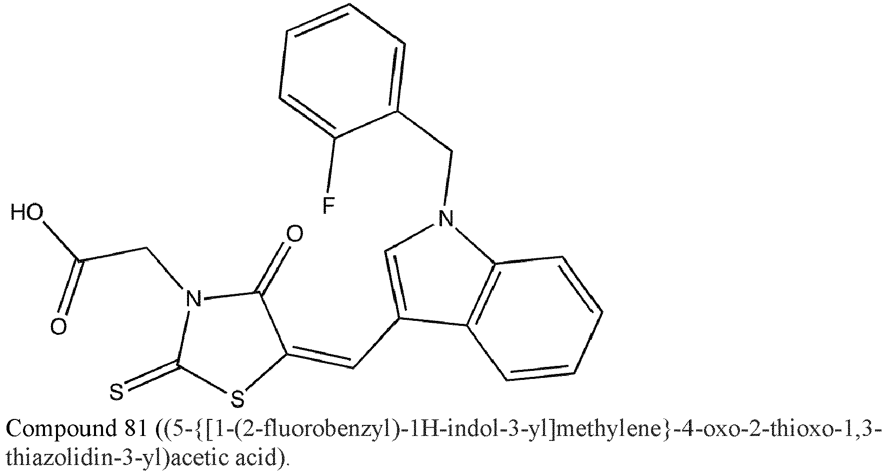

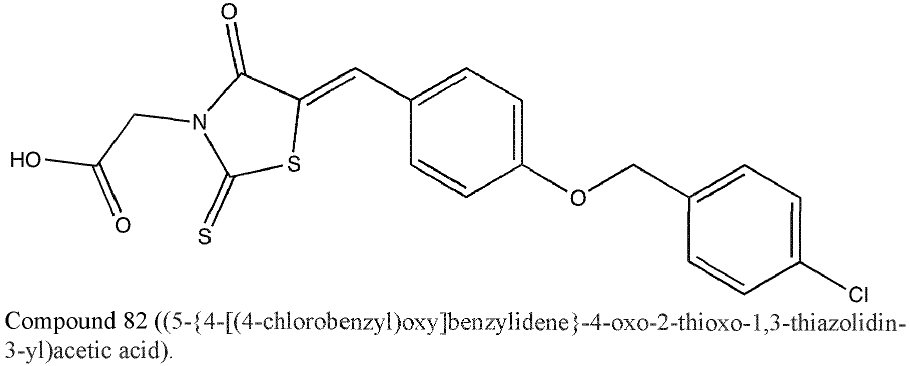

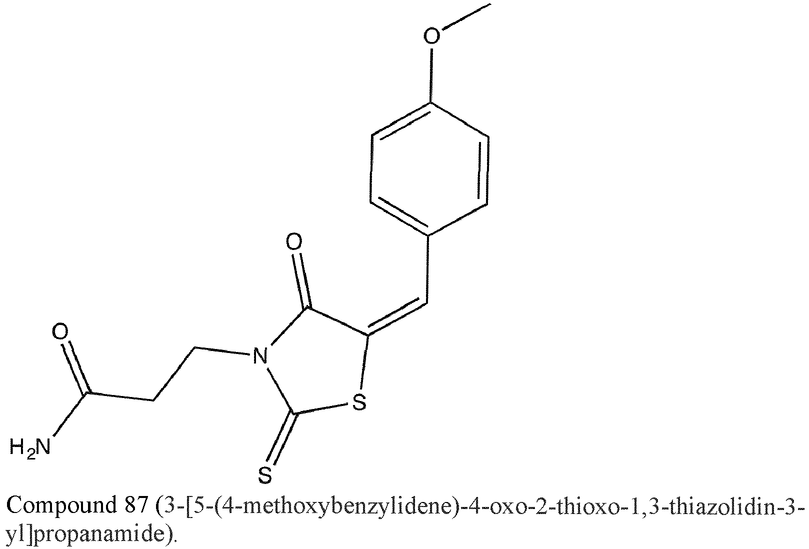

### 1. Pharmaceutically Acceptable Salts

Also disclosed herein are pharmaceutically acceptable salts of the compound represented by Formula I, and the derivatives thereof as well as other compounds that were determined to bind DHFR. Examples of pharmaceutically acceptable salts include, but are not limited to mineral or organic acid salts of basic residues such as amines; and alkali or organic salts of acidic residues such as carboxylic acids. The pharmaceutically acceptable salts include the conventional non-toxic salts or the quaternary ammonium salts of the parent compound formed, for example, from non-toxic inorganic or organic acids. Such conventional non-toxic salts include those derived from inorganic acids such as hydrochloric, hydrobromic, sulfuric, sulfamic, phosphoric, and nitric acids; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, tolunesulfonic, naphthalene sulfonic, methane sulfonic, ethane disulfonic, oxalic, and isethionic salts.

The pharmaceutically acceptable salts of the compounds can be synthesized from the parent compound, which contains a basic or acidic moiety, by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred. Lists of suitable salts are found in Remington's Pharmaceutical Sciences, 20th ed., Lippincott Williams & Wilkins, Baltimore, MD, 2000, p. 704; and "Handbook of Pharmaceutical Salts: Properties, Selection, and Use," P. Heinrich Stahl and Camille G. Wermuth, Eds., Wiley- VCH, Weinheim, 2002.

### 2. Intermediates

The compounds identified herein using virtual ligand screening can be used as a starting compound or intermediate compound to produce a final compound that has increased binding to an enzyme, for example DHFR, or increased inhibition of the enzyme, for example DHFR, relative to starting compound or intermediate compound. The compounds identified herein can be modified to increase bioavailability, increase half-life in the blood stream, increase solubility, increase hydrophilicity, increase hydrophobicity, or a combination thereof using conventional techniques.The compounds can be modified to incorporate polar functional groups, such as the alcohol, amine, amide, carboxylic acid, sulfonic acid and phosphate groups, which either ionize or are capable of relatively strong intermolecular forces of attraction with water (hydrogen bonding), usually resulting in analogues with an increased water solubility. Acidic and basic groups are particularly useful, since these groups can be used to form salts, which would give a wider range of dosage forms for the final product. However, the formation of zwitterions by the introduction of either an acid group into a structure containing a base or a base group into a structure containing an acid group can reduce water solubility. Introduction of weakly polar groups, such as carboxylic acid esters, aryl halides and alkyl halides, will not significantly improve water solubility and can result in enhanced lipid solubility.

The incorporation of acidic residues into the compound is less likely to change the type of activity, but it can result in the analogue exhibiting hemolytic properties. Furthermore, the introduction of an aromatic acid group usually results in anti-inflammatory activity, whilst carboxylic acids with an alpha functional group may act as chelating agents. Basic water solubilizing groups have a tendency to change the mode of action, since bases often interfere with neurotransmitters and biological processes involving amines. However, their incorporation does mean that the analogue can be formulated as a wide variety of acid salts. Non-ionizable groups do not have the disadvantages of acidic and basic groups.

Groups that are bound directly to the carbon skeleton of the lead compound by less reactive C-C, C-O and C-N bonds are likely to be irreversibly attached to the lead structure. Groups that are linked to the compound by ester, amide, phosphate, sulfate and glycosidic bonds are more likely to be metabolized from the resulting analogue to reform the parent compound as the analogue is transferred from its point of administration to its site of action. Compounds with this type of solubilizing group are acting as prodrugs and so their activity is more likely to be the same as the parent compound. However, the rate of loss of the solubilizing group will depend on the nature of the transfer route, and this could affect the activity of the drug.

To preserve the type of activity exhibited by the compound, a water solubilizing group should be attached to a part of the structure that is not involved in the drug- receptor interaction. Consequently, the route used to introduce a new water solubilizing group and its position in the lead compound will depend on the relative reactivities of the compound and the rest of the molecule. Examples of water solubilizing structures and the routes used to introduce them into the lead structures. O-alkylation, N-alkylation, O- acylation and N-acylation reactions are used to introduce both acidic and basic groups. Acetylation methods use both the appropriate acid chloride and anhydride.

Examples of water solubilizing structures and the routes used to introduce them into lead compounds include but are not limited to phosphate acid halides for introducing phosphate groups into compounds. Structures containing hydroxy groups have been introduced by reaction of the corresponding monochlorinated hydrin and the use of suitable epoxides. Sulphonic acid groups may be introduced by either direct sulfonation or the addition of bisulfite to reactive C = C bonds.

### B. Other Bioactive Agents

Other bioactive agents that can be used with the compounds disclosed herein include, but are not limited to antimicrobial agents, in particular antibiotics, disinfectants, anti-scarring agents and anti-inflammatory agents.

Antimicrobial agents that may be incorporated into the perforated collagen coated meshes, include, but are not limited to, antibacterial drugs, antiviral agents, antifungal agents, and antiparisitic drugs. Antimicrobial agents include substances that kill or inhibit the growth of microbes such as microbicidal and microbiostatic agents. Antimicrobial agents that may be incorporated into the perforated collagen coated meshes, include, but are not limited to: rifampin; minocycline and its hydrochloride, sulfate, or phosphate salt; triclosan; chlorhexidine; vancomycin and its hydrochloride, sulfate, or phosphate salt; tetracycline and its hydrochloride, sulfate, or phosphate salt, and derivatives; gentamycin; cephalosporin antimicrobials; aztreonam; cefotetan and its disodium salt; loracarbef; cefoxitin and its sodium salt; cefazolin and its sodium salt; cefaclor; ceftibuten and its sodium salt; ceftizoxime; ceftizoxime sodium salt; cefoperazone and its sodium salt; cefuroxime and its sodium salt; cefuroxime axetil; cefprozil; ceftazidime; cefotaxime and its sodium salt; cefadroxil; ceftazidime and its sodium salt; cephalexin; cefamandole nafate; cefepime and its hydrochloride, sulfate, and phosphate salt; cefdinir and its sodium salt; ceftriaxone and its sodium salt; cefixime and its sodium salt; cefpodoxime proxetil; meropenem and its sodium salt; imipenem and its sodium salt; cilastatin and its sodium salt; azithromycin; clarithromycin; dirithromycin; erythromycin and hydrochloride, sulfate, or phosphate salts, ethylsuccinate, and stearate forms thereof, clindamycin; clindamycin hydrochloride, sulfate, or phosphate salt; lincomycin and hydrochloride, sulfate, or phosphate salt thereof, tobramycin and its hydrochloride, sulfate, or phosphate salt; streptomycin and its hydrochloride, sulfate, or phosphate salt; neomycin and its hydrochloride, sulfate, or phosphate salt; acetyl sulfisoxazole; colistimethate and its sodium salt; quinupristin; dalfopristin; amoxicillin; ampicillin and its sodium salt; clavulanic acid and its sodium or potassium salt; penicillin G; penicillin G benzathine, or procaine salt; penicillin G sodium or potassium salt; carbenicillin and its disodium or indanyl disodium salt; piperacillin and its sodium salt; ticarcillin and its disodium salt; sulbactam and its sodium salt; moxifloxacin; ciprofloxacin; ofloxacin; levofloxacins; norfloxacin; gatifloxacin; trovafloxacin mesylate; alatrofloxacin mesylate; trimethoprim; sulfamethoxazole; demeclocycline and its hydrochloride, sulfate, or phosphate salt; doxycycline and its hydrochloride, sulfate, or phosphate salt; oxytetracycline and its hydrochloride, sulfate, or phosphate salt; chlortetracycline and its hydrochloride, sulfate, or phosphate salt; metronidazole; dapsone; atovaquone; rifabutin; linezolide; polymyxin B and its hydrochloride, sulfate, or phosphate salt; sulfacetamide and its sodium salt; clarithromycin; gentamicin; biguanide; bacitracin; silver, copper, zinc, and gold ions, salts, and complexes. In a preferred form the antimicrobial agents incorporated into the implants are (i) rifampin and (ii) minocycline and its hydrochloride, sulfate, or phosphate salt. In a particularly preferred form the perforated collagen coated meshes comprise rifampin and minocycline or its hydrochloride, sulfate, or phosphate salt.

### C. Formulations

The compounds described herein can be formulated for enteral, parenteral, topical, or pulmonary administration. The compounds can be combined with one or more pharmaceutically acceptable carriers and/or excipients that are considered safe and effective and may be administered to an individual without causing undesirable biological side effects or unwanted interactions. The carrier is all components present in the pharmaceutical formulation other than the active ingredient or ingredients.

Such pharmaceutical carriers can be sterile liquids, such as water and oils, including those of petroleum, animal, vegetable or synthetic origin, such as peanut oil, soybean oil, mineral oil, sesame oil and the like. Saline solutions and aqueous dextrose and glycerol solutions can also be employed as liquid carriers, particularly for injectable solutions.

The compounds disclosed herein can also be formulated for use as a disinfectant, for example, in a hospital environment.

These compositions can take the form of solutions, suspensions, emulsion, tablets, pills, capsules, powders, sustained-release formulations and the like.

Suitable pharmaceutical excipients include starch, glucose, sucrose, gelatin, lactose, malt, rice, flour, chalk, silica gel, sodium stearate, glycerol monostearate, talc, sodium chloride, glycerol, propylene, glycol, water, ethanol and the like. The pharmaceutical composition may also contain wetting or emulsifying agents or suspending/diluting agents, or pH buffering agents, or agents for modifying or maintaining the rate of release of the compounds/antibiotics, all of which are disclosed further herein.

### 1. Parenteral Formulations

The compounds described herein can be formulated for parenteral administration. For example, parenteral administration may include administration to a patient intravenously, intradermally, intraarterially, intraperitoneally, intralesionally, intracranially, intraarticularly, intraprostatically, intrapleurally, intratracheally, intravitreally, intratumorally, intramuscularly, subcutaneously, subconjunctivally, intravesicularly, intrapericardially, intraumbilically, by injection, and by infusion.

Parenteral formulations can be prepared as aqueous compositions using techniques known in the art. Typically, such compositions can be prepared as injectable formulations, for example, solutions or suspensions; solid forms suitable for using to prepare solutions or suspensions upon the addition of a reconstitution medium prior to injection; emulsions, such as water-in-oil (w/o) emulsions, oil-in-water (o/w) emulsions, and microemulsions thereof, liposomes, or emulsomes.

If for intravenous administration, the compositions are packaged in solutions of sterile isotonic aqueous buffer. Where necessary, the composition may also include a solubilizing agent. The components of the composition are supplied either separately or mixed together in unit dosage form, for example, as a dry lyophilized powder or concentrated solution in a hermetically sealed container such as an ampoule or sachet indicating the amount of active agent. If the composition is to be administered by infusion, it can be dispensed with an infusion bottle containing sterile pharmaceutical grade water or saline. Where the composition is administered by injection, an ampoule of sterile water or saline can be provided so that the ingredients may be mixed prior to injection. 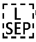 The carrier can be a solvent or dispersion medium containing, for example, water, ethanol, one or more polyols (e.g., glycerol, propylene glycol, and liquid polyethylene glycol), oils, such as vegetable oils (e.g., peanut oil, corn oil, sesame oil, etc.), and combinations thereof. The proper fluidity can be maintained, for example, by the use of a coating, such as lecithin, by the maintenance of the required particle size in the case of dispersion and/or by the use of surfactants. In many cases, it will be preferable to include isotonic agents, for example, sugars or sodium chloride.

Solutions and dispersions of the active compounds as the free acid or base or pharmacologically acceptable salts thereof can be prepared in water or another solvent or dispersing medium suitably mixed with one or more pharmaceutically acceptable excipients including, but not limited to, surfactants, dispersants, emulsifiers, pH modifying agents, viscosity modifying agents, and combination thereof.

Suitable surfactants may be anionic, cationic, amphoteric or nonionic surface- active agents. Suitable anionic surfactants include, but are not limited to, those containing carboxylate, sulfonate and sulfate ions. Examples of anionic surfactants include sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; and alkyl sulfates such as sodium lauryl sulfate. Cationic surfactants include, but are not limited to, quaternary ammonium compounds such as benzalkonium chloride, benzethonium chloride, cetrimonium bromide, stearyl dimethylbenzyl ammonium chloride, polyoxyethylene, and coconut amine. Examples of nonionic surfactants include ethylene glycol monostearate, propylene glycol myristate, glyceryl monostearate, glyceryl stearate, polyglyceryl-4- oleate, sorbitan acylate, sucrose acylate, PEG-150 laurate, PEG-400 monolaurate, polyoxyethylene monolaurate, polysorbates, polyoxyethylene octylphenylether, PEG- 1000 cetyl ether, polyoxyethylene tridecyl ether, polypropylene glycol butyl ether, Poloxamer^{®} 401, stearoyl monoisopropanolamide, and polyoxyethylene hydrogenated tallow amide. Examples of amphoteric surfactants include sodium N-dodecyl-.beta.- alanine, sodium N-lauryl-.beta.-iminodipropionate, myristoamphoacetate, lauryl betaine, and lauryl sulfobetaine.

The formulation can contain a preservative to prevent the growth of 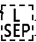 microorganisms. Suitable preservatives include, but are not limited to, parabens, chlorobutanol, phenol, sorbic acid, and thimerosal. The formulation may also contain an antioxidant to prevent degradation of the active agent(s).

The formulation is typically buffered to a pH of 3-8 for parenteral administration upon reconstitution. Suitable buffers include, but are not limited to, phosphate buffers, acetate buffers, and citrate buffers.

Water-soluble polymers are often used in formulations for parenteral administration. Suitable water-soluble polymers include, but are not limited to, polyvinylpyrrolidone, dextran, carboxymethylcellulose, and polyethylene glycol.

Sterile injectable solutions can be prepared by incorporating the active compounds in the required amount in the appropriate solvent or dispersion medium with one or more of the excipients listed above, as required, followed by filtered sterilization. Generally, dispersions are prepared by incorporating the various sterilized active ingredients into a sterile vehicle which contains the basic dispersion medium and the required other ingredients from those listed above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. The powders can be prepared in such a manner that the particles are porous in nature, which can increase dissolution of the particles. Methods for making porous particles are well known in the art.

### (a) Controlled Release Formulations

The parenteral formulations described herein can be formulated for controlled release including immediate release, delayed release, extended release, pulsatile release, and combinations thereof.

### 1. Nano- and microparticles

For parenteral administration, the one or more compounds, and optional one or more additional active agents, can be incorporated into microparticles, nanoparticles, or combinations thereof that provide controlled release of the compounds and/or one or more additional active agents. In forms wherein the formulations contains two or more drugs, the drugs can be formulated for the same type of controlled release (e.g., delayed, extended, immediate, or pulsatile) or the drugs can be independently formulated for different types of release (e.g., immediate and delayed, immediate and extended, delayed and extended, delayed and pulsatile, etc.).

For example, the compounds and/or one or more additional active agents can be incorporated into polymeric microparticles, which provide controlled release of the drug(s). Release of the drug(s) is controlled by diffusion of the drug(s) out of the microparticles and/or degradation of the polymeric particles by hydrolysis and/or enzymatic degradation. Suitable polymers include ethylcellulose and other natural or synthetic cellulose derivatives.

Polymers, which are slowly soluble and form a gel in an aqueous environment, such as hydroxypropyl methylcellulose or polyethylene oxide, can also be suitable as materials for drug containing microparticles. Other polymers include, but are not limited to, polyanhydrides, poly(ester anhydrides), polyhydroxy acids, such as polylactide (PLA), polyglycolide (PGA), poly(lactide-co-glycolide) (PLGA), poly-3- hydroxybutyrate (PHB) and copolymers thereof, poly-4-hydroxybutyrate (P4HB) and copolymers thereof, polycaprolactone and copolymers thereof, and combinations thereof.

Alternatively, the drug(s) can be incorporated into microparticles prepared from materials which are insoluble in aqueous solution or slowly soluble in aqueous solution, but are capable of degrading within the GI tract by means including enzymatic degradation, surfactant action of bile acids, and/or mechanical erosion. As used herein, the term "slowly soluble in water" refers to materials that are not dissolved in water within a period of 30 minutes. Preferred examples include fats, fatty substances, waxes, wax-like substances and mixtures thereof. Suitable fats and fatty substances include fatty alcohols (such as lauryl, myristyl stearyl, cetyl or cetostearyl alcohol), fatty acids and derivatives, including but not limited to fatty acid esters, fatty acid glycerides (mono-, di- and tri-glycerides), and hydrogenated fats. Specific examples include, but are not limited to hydrogenated vegetable oil, hydrogenated cottonseed oil, hydrogenated castor oil, hydrogenated oils available under the trade name Sterotex^{®}, stearic acid, cocoa butter, and stearyl alcohol. Suitable waxes and wax-like materials include natural or synthetic waxes, hydrocarbons, and normal waxes. Specific examples of waxes include beeswax, glycowax, castor wax, carnauba wax, paraffins and candelilla wax. As used herein, a wax-like material is defined as any material, which is normally solid at room temperature and has a melting point of from about 30 to 300°C.

In some cases, it may be desirable to alter the rate of water penetration into the microparticles. To this end, rate-controlling (wicking) agents can be formulated along with the fats or waxes listed above. Examples of rate-controlling materials include certain starch derivatives (e.g., waxy maltodextrin and drum dried corn starch), cellulose derivatives (e.g., hydroxypropylmethyl-cellulose, hydroxypropylcellulose, methylcellulose, and carboxymethyl-cellulose), alginic acid, lactose and talc. Additionally, a pharmaceutically acceptable surfactant (for example, lecithin) may be added to facilitate the degradation of such microparticles.

Proteins, which are water insoluble, such as zein, can also be used as materials for the formation of drug containing microparticles. Additionally, proteins, polysaccharides and combinations thereof, which are water-soluble, can be formulated with drug into microparticles and subsequently cross-linked to form an insoluble network. For example, cyclodextrins can be complexed with individual drug molecules and subsequently cross-linked.

*2. Method of making Nano- and Microparticles* Encapsulation or incorporation of drug into carrier materials to produce drug-containing microparticles can be achieved through known pharmaceutical formulation techniques. In the case of formulation in fats, waxes or wax-like materials, the carrier material is typically heated above its melting temperature and the drug is added to form a mixture comprising drug particles suspended in the carrier material, drug dissolved in the carrier material, or a mixture thereof. Microparticles can be subsequently formulated through several methods including, but not limited to, the processes of congealing, extrusion, spray chilling or aqueous dispersion. In a preferred process, wax is heated above its melting temperature, drug is added, and the molten wax-drug mixture is congealed under constant stirring as the mixture cools. Alternatively, the molten wax- drug mixture can be extruded and spheronized to form pellets or beads. These processes are known in the art.

For some carrier materials it may be desirable to use a solvent evaporation technique to produce drug-containing microparticles. In this case drug and carrier material are co-dissolved in a mutual solvent and microparticles can subsequently be produced by several techniques including, but not limited to, forming an emulsion in water or other appropriate media, spray drying or by evaporating off the solvent from the bulk solution and milling the resulting material.

In some forms, drug in a particulate form is homogeneously dispersed in a water- insoluble or slowly water soluble material. To minimize the size of the drug particles within the composition, the drug powder itself may be milled to generate fine particles prior to formulation. The process of jet milling, known in the pharmaceutical art, can be used for this purpose. In some forms, drug in a particulate form is homogeneously dispersed in a wax or wax like substance by heating the wax or wax like substance above its melting point and adding the drug particles while stirring the mixture. In this case a pharmaceutically acceptable surfactant may be added to the mixture to facilitate the dispersion of the drug particles.

The particles can also be coated with one or more modified release coatings. Solid esters of fatty acids, which are hydrolyzed by lipases, can be spray coated onto microparticles or drug particles. Zein is an example of a naturally water-insoluble protein. It can be coated onto drug containing microparticles or drug particles by spray coating or by wet granulation techniques. In addition to naturally water-insoluble materials, some substrates of digestive enzymes can be treated with cross-linking procedures, resulting in the formation of non-soluble networks. Many methods of cross- linking proteins, initiated by both chemical and physical means, have been reported. One of the most common methods to obtain cross-linking is the use of chemical cross-linking agents. Examples of chemical cross-linking agents include aldehydes (gluteraldehyde and formaldehyde), epoxy compounds, carbodiimides, and genipin. In addition to these cross-linking agents, oxidized and native sugars have been used to cross-link gelatin. Cross-linking can also be accomplished using enzymatic means; for example, transglutaminase has been approved as a GRAS substance for cross-linking seafood products. Finally, cross-linking can be initiated by physical means such as thermal treatment, UV irradiation and gamma irradiation.

To produce a coating layer of cross-linked protein surrounding drug containing microparticles or drug particles, a water-soluble protein can be spray coated onto the microparticles and subsequently cross-linked by the one of the methods described above. Alternatively, drug-containing microparticles can be microencapsulated within protein by coacervation-phase separation (for example, by the addition of salts) and subsequently cross-linked. Some suitable proteins for this purpose include gelatin, albumin, casein, and gluten.

Polysaccharides can also be cross-linked to form a water-insoluble network. For many polysaccharides, this can be accomplished by reaction with calcium salts or multivalent cations, which cross-link the main polymer chains. Pectin, alginate, dextran, amylose and guar gum are subject to cross-linking in the presence of multivalent cations. Complexes between oppositely charged polysaccharides can also be formed; pectin and chitosan, for example, can be complexed via electrostatic interactions.

### (b) Injectable/Implantable formulations

The compounds described herein can be incorporated into injectable/implantable solid or semi-solid implants, such as polymeric implants. In some forms, the compounds are incorporated into a polymer that is a liquid or paste at room temperature, but upon contact with aqueous medium, such as physiological fluids, exhibits an increase in viscosity to form a semi-solid or solid material. Exemplary polymers include, but are not limited to, hydroxyalkanoic acid polyesters derived from the copolymerization of at least one unsaturated hydroxy fatty acid copolymerized with hydroxyalkanoic acids. The polymer can be melted, mixed with the active substance and cast or injection molded into a device. Such melt fabrication requires polymers having a melting point that is below the temperature at which the substance to be delivered and polymer degrade or become reactive. The device can also be prepared by solvent casting where the polymer is dissolved in a solvent and the drug dissolved or dispersed in the polymer solution and the solvent is then evaporated. Solvent processes require that the polymer be soluble in organic solvents. Another method is compression molding of a mixed powder of the polymer and the drug or polymer particles loaded with the active agent.

Alternatively, the compounds can be incorporated into a polymer matrix and molded, compressed, or extruded into a device that is a solid at room temperature. For example, the compounds can be incorporated into a biodegradable polymer, such as polyanhydrides, polyhydroalkanoic acids (PHAs), PLA, PGA, PLGA, polycaprolactone, polyesters, polyamides, polyorthoesters, polyphosphazenes, proteins and polysaccharides such as collagen, hyaluronic acid, albumin and gelatin, and combinations thereof and compressed into solid device, such as disks, or extruded into a device, such as rods.

The release of the one or more compounds from the implant can be varied by selection of the polymer, the molecular weight of the polymer, and/or modification of the polymer to increase degradation, such as the formation of pores and/or incorporation of hydrolyzable linkages. Methods for modifying the properties of biodegradable polymers to vary the release profile of the compounds from the implant are well known in the art.

### 2. Enteral Formulations

Oral formulations can include standard carriers such as pharmaceutical grades of mannitol, lactose, sodium saccharine, starch, magnesium stearate, cellulose, magnesium carbonate, etc. Such compositions will contain a therapeutically effective amount of the compound and/or antibiotic together with a suitable amount of carrier so as to provide the proper form to the patient based on the mode of administration to be used. Suitable oral dosage forms include tablets, capsules, solutions, suspensions, syrups, and lozenges. Tablets can be made using compression or molding techniques well known in the art. Gelatin or non-gelatin capsules can be prepared as hard or soft capsule shells, which can encapsulate liquid, solid, and semi-solid fill materials, using techniques well known in the art.

Formulations may be prepared using a pharmaceutically acceptable carrier. As generally used herein "carrier" includes, but is not limited to, diluents, preservatives, binders, lubricants, disintegrators, swelling agents, fillers, stabilizers, and combinations thereof. 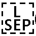 Carrier also includes all components of the coating composition, which may include plasticizers, pigments, colorants, stabilizing agents, and glidants.

Examples of suitable coating materials include, but are not limited to, cellulose polymers such as cellulose acetate phthalate, hydroxypropyl cellulose, hydroxypropyl methylcellulose, hydroxypropyl methylcellulose phthalate and hydroxypropyl methylcellulose acetate succinate; polyvinyl acetate phthalate, acrylic acid polymers and copolymers, and methacrylic resins that are commercially available under the trade name EUDRAGIT^{®} (Roth Pharma, Westerstadt, Germany), zein, shellac, and polysaccharides.

Additionally, the coating material may contain conventional carriers such as plasticizers, pigments, colorants, glidants, stabilization agents, pore formers and surfactants.

"Diluents", also referred to as "fillers," are typically necessary to increase the 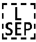 bulk of a solid dosage form so that a practical size is provided for compression of tablets or formation of beads and granules. Suitable diluents include, but are not limited to, dicalcium phosphate dihydrate, calcium sulfate, lactose, sucrose, mannitol, sorbitol, cellulose, microcrystalline cellulose, kaolin, sodium chloride, dry starch, hydrolyzed starches, pregelatinized starch, silicone dioxide, titanium oxide, magnesium aluminum silicate and powdered sugar. "Binders" are used to impart cohesive qualities to a solid dosage formulation, and thus ensure that a tablet or bead or granule remains intact after the formation of the dosage forms. Suitable binder materials include, but are not limited to, starch, pregelatinized starch, gelatin, sugars (including sucrose, glucose, dextrose, lactose and sorbitol), polyethylene glycol, waxes, natural and synthetic gums such as acacia, tragacanth, sodium alginate, cellulose, including hydroxypropylmethylcellulose, hydroxypropylcellulose, ethylcellulose, and veegum, and synthetic polymers such as acrylic acid and methacrylic acid copolymers, methacrylic acid copolymers, methyl methacrylate copolymers, aminoalkyl methacrylate copolymers, polyacrylic acid/polymethacrylic acid and polyvinylpyrrolidone. "Lubricants" are used to facilitate tablet manufacture. Examples of suitable lubricants include, but are not limited to, magnesium stearate, calcium stearate, stearic acid, glycerol behenate, polyethylene glycol, talc, and mineral oil.

"Disintegrants" are used to facilitate dosage form disintegration or "breakup" after administration, and generally include, but are not limited to, starch, sodium starch glycolate, sodium carboxymethyl starch, sodium carboxymethylcellulose, hydroxypropyl cellulose, pregelatinized starch, clays, cellulose, alginine, gums or cross linked polymers, such as cross-linked PVP (Polyplasdone^{®} XL from GAF Chemical Corp).

"Stabilizers" are used to inhibit or retard drug decomposition reactions, which include, by way of example, oxidative reactions. Suitable stabilizers include, but are not limited to, antioxidants, butylated hydroxy toluene (BHT); ascorbic acid, its salts and esters; Vitamin E, tocopherol and its salts; sulfites such as sodium metabisulphite; cysteine and its derivatives; citric acid; propyl gallate, and butylated hydroxyanisole (BHA).

### (a) Controlled Release Enteral Formulations

Oral dosage forms, such as capsules, tablets, solutions, and suspensions, can for formulated for controlled release. For example, the one or more compounds and optional one or more additional active agents can be formulated into nanoparticles, microparticles, and combinations thereof, and encapsulated in a soft or hard gelatin or non-gelatin capsule or dispersed in a dispersing medium to form an oral suspension or syrup. The particles can be formed of the drug and a controlled release polymer or matrix. Alternatively, the drug particles can be coated with one or more controlled release coatings prior to incorporation in to the finished dosage form.

In another form, the one or more compounds and optional one or more additional active agents are dispersed in a matrix material, which gels or emulsifies upon contact with an aqueous medium, such as physiological fluids. In the case of gels, the matrix swells entrapping the active agents, which are released slowly over time by diffusion and/or degradation of the matrix material. Such matrices can be formulated as tablets or as fill materials for hard and soft capsules.

In still another form, the one or more compounds, and optional one or more additional active agents are formulated into a sold oral dosage form, such as a tablet or capsule, and the solid dosage form is coated with one or more controlled release coatings, such as a delayed release coatings or extended release coatings. The coating or coatings may also contain the compounds and/or additional active agents.

### (1) Extended release dosage forms

The extended release formulations are generally prepared as diffusion or osmotic systems, which are known in the art. A diffusion system typically consists of two types of devices, a reservoir and a matrix, and is well known and described in the art. The matrix devices are generally prepared by compressing the drug with a slowly dissolving polymer carrier into a tablet form. The three major types of materials used in the preparation of matrix devices are insoluble plastics, hydrophilic polymers, and fatty compounds. Plastic matrices include, but are not limited to, methyl acrylate-methyl methacrylate, polyvinyl chloride, and polyethylene. Hydrophilic polymers include, but are not limited to, cellulosic polymers such as methyl and ethyl cellulose, hydroxyalkylcelluloses such as hydroxypropyl-cellulose, hydroxypropylmethylcellulose, sodium carboxymethylcellulose, and Carbopol^{®} 934, polyethylene oxides and mixtures thereof. Fatty compounds include, but are not limited to, various waxes such as carnauba wax and glyceryl tristearate and wax-type substances including hydrogenated castor oil or hydrogenated vegetable oil, or mixtures thereof.

In certain preferred forms, the plastic material is a pharmaceutically acceptable acrylic polymer, including but not limited to, acrylic acid and methacrylic acid copolymers, methyl methacrylate, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamine copolymer poly(methyl methacrylate), poly(methacrylic acid)(anhydride), polymethacrylate, polyacrylamide, poly(methacrylic acid anhydride), and glycidyl methacrylate copolymers.

In certain preferred forms, the acrylic polymer is comprised of one or more ammonio methacrylate copolymers. Ammonio methacrylate copolymers are well known in the art, and are described in NF XVII as fully polymerized copolymers of acrylic and methacrylic acid esters with a low content of quaternary ammonium groups.

In one preferred form, the acrylic polymer is an acrylic resin lacquer such as that which is commercially available from Rohm Pharma under the tradename EUDRAGIT^{®}. In further preferred forms, the acrylic polymer comprises a mixture of two acrylic resin lacquers commercially available from Rohm Pharma under the tradenames EUDRAGIT^{®} RL30D and EUDRAGIT ^{®} RS30D, respectively. EUDRAGIT^{®} RL30D and EUDRAGIT ^{®} RS30D are copolymers of acrylic and methacrylic esters with a low content of quaternary ammonium groups, the molar ratio of ammonium groups to the remaining neutral (meth)acrylic esters being 1:20 in EUDRAGIT ^{®} RL30D and 1:40 in EUDRAGIT^{®} RS30D. The mean molecular weight is about 150,000. EUDRAGIT ^{®} S-100 and EUDRAGIT ^{®} L-100 are also preferred. The code designations RL (high permeability) and RS (low permeability) refer to the permeability properties of these agents. EUDRAGIT ^{®} RL/RS mixtures are insoluble in water and in digestive fluids. However, multiparticulate systems formed to include the same are swellable and permeable in aqueous solutions and digestive fluids. 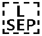

The polymers described above such as EUDRAGIT ^{®} RL/RS may be mixed together in any desired ratio in order to ultimately obtain a sustained-release formulation having a desirable dissolution profile. Desirable sustained-release multiparticulate systems may be obtained, for instance, from 100% EUDRAGIT^{®} RL, 50% EUDRAGIT^{®} RL and 50% EUDRAGIT t^{®} RS, and 10% EUDRAGIT^{®} RL and 90% EUDRAGIT^{®} RS. One skilled in the art will recognize that other acrylic polymers may also be used, such as, for example, EUDRAGIT^{®} L.

Alternatively, extended release formulations can be prepared using osmotic systems or by applying a semi-permeable coating to the dosage form. In the latter case, the desired drug release profile can be achieved by combining low permeable and high permeable coating materials in suitable proportion.

The devices with different drug release mechanisms described above can be combined in a final dosage form comprising single or multiple units. Examples of multiple units include, but are not limited to, multilayer tablets and capsules containing tablets, beads, or granules An immediate release portion can be added to the extended release system by means of either applying an immediate release layer on top of the extended release core using a coating or compression process or in a multiple unit system such as a capsule containing extended and immediate release beads.

Extended release tablets containing hydrophilic polymers are prepared by techniques commonly known in the art such as direct compression, wet granulation, or dry granulation. Their formulations usually incorporate polymers, diluents, binders, and lubricants as well as the active pharmaceutical ingredient. The usual diluents include inert powdered substances such as starches, powdered cellulose, especially crystalline and microcrystalline cellulose, sugars such as fructose, mannitol and sucrose, grain flours and similar edible powders. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders include substances such as starch, gelatin and sugars such as lactose, fructose, and glucose. Natural and synthetic gums, including acacia, alginates, methylcellulose, and polyvinylpyrrolidone can also be used. Polyethylene glycol, hydrophilic polymers, ethylcellulose and waxes can also serve as binders. A lubricant is necessary in a tablet formulation to prevent the tablet and punches from sticking in the die. The lubricant is chosen from such slippery solids as talc, magnesium and calcium stearate, stearic acid and hydrogenated vegetable oils.

Extended release tablets containing wax materials are generally prepared using methods known in the art such as a direct blend method, a congealing method, and an aqueous dispersion method. In the congealing method, the drug is mixed with a wax material and either spray- congealed or congealed and screened and processed.

### (2) Delayed release dosage forms

Delayed release formulations can be created by coating a solid dosage form with a polymer film, which is insoluble in the acidic environment of the stomach, and soluble in the neutral environment of the small intestine.

The delayed release dosage units can be prepared, for example, by coating a drug or a drug-containing composition with a selected coating material. The drug-containing composition may be, e.g., a tablet for incorporation into a capsule, a tablet for use as an inner core in a "coated core" dosage form, or a plurality of drug-containing beads, particles or granules, for incorporation into either a tablet or capsule. Preferred coating materials include bioerodible, gradually hydrolyzable, gradually water-soluble, and/or enzymatically degradable polymers, and may be conventional "enteric" polymers. Enteric polymers, as will be appreciated by those skilled in the art, become soluble in the higher pH environment of the lower gastrointestinal tract or slowly erode as the dosage form passes through the gastrointestinal tract, while enzymatically degradable polymers are degraded by bacterial enzymes present in the lower gastrointestinal tract, particularly in the colon. Suitable coating materials for effecting delayed release include, but are not limited to, cellulosic polymers such as hydroxypropyl cellulose, hydroxyethyl cellulose, hydroxymethyl cellulose, hydroxypropyl methyl cellulose, hydroxypropyl methyl cellulose acetate succinate, hydroxypropylmethyl cellulose phthalate, methylcellulose, ethyl cellulose, cellulose acetate, cellulose acetate phthalate, cellulose acetate trimellitate and carboxymethylcellulose sodium; acrylic acid polymers and copolymers, preferably formed from acrylic acid, methacrylic acid, methyl acrylate, ethyl acrylate, methyl methacrylate and/or ethyl methacrylate, and other methacrylic resins that are commercially available under the tradename Eudragit^{®} (Rohm Pharma; Westerstadt, Germany), including EUDRAGIT^{®} L30D-55 and L100-55 (soluble at pH 5.5 and above), EUDRAGIT^{®} L-100 (soluble at pH 6.0 and above), EUDRAGIT^{®} S (soluble at pH 7.0 and above, as a result of a higher degree of esterification), and EUDRAGITS^{®} NE, RL and RS (water-insoluble polymers having different degrees of permeability and expandability); vinyl polymers and copolymers such as polyvinyl pyrrolidone, vinyl acetate, vinylacetate phthalate, vinylacetate crotonic acid copolymer, and ethylene-vinyl acetate copolymer; enzymatically degradable polymers such as azo polymers, pectin, chitosan, amylose and guar gum; zein and shellac. Combinations of different coating materials may also be used. Multi-layer coatings using different polymers may also be applied.

The preferred coating weights for particular coating materials may be readily 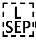 determined by those skilled in the art by evaluating individual release profiles for tablets, beads and granules prepared with different quantities of various coating materials. It is the combination of materials, method and form of application that produce the desired release characteristics, which one can determine only from the clinical studies.

The coating composition may include conventional additives, such as plasticizers, pigments, colorants, stabilizing agents, glidants, etc. A plasticizer is normally present to reduce the fragility of the coating, and will generally represent about 10 wt. % to 50 wt. % relative to the dry weight of the polymer. Examples of typical plasticizers include polyethylene glycol, propylene glycol, triacetin, dimethyl phthalate, diethyl phthalate, dibutyl phthalate, dibutyl sebacate, triethyl citrate, tributyl citrate, triethyl acetyl citrate, castor oil and acetylated monoglycerides. A stabilizing agent is preferably used to stabilize particles in the dispersion. Typical stabilizing agents are nonionic emulsifiers such as sorbitan esters, polysorbates and polyvinylpyrrolidone. Glidants are recommended to reduce sticking effects during film formation and drying, and will generally represent approximately 25 wt. % to 100 wt. % of the polymer weight in the coating solution. One effective glidant is talc. Other glidants such as magnesium stearate and glycerol monostearates may also be used. Pigments such as titanium dioxide may also be used. Small quantities of an anti-foaming agent, such as a silicone (e.g., simethicone), may also be added to the coating composition.

### 3. Topical Formulations

Suitable dosage forms for topical administration include creams, ointments, salves, sprays, gels, lotions, emulsions, and transdermal patches. The formulation may be formulated for transmucosal, transepithelial, transendothelial, or transdermal administration. The compounds can also be formulated for intranasal delivery, pulmonary delivery, or inhalation. The compositions may further contain one or more chemical penetration enhancers, membrane permeability agents, membrane transport agents, emollients, surfactants, stabilizers, buffers, and combination thereof.

In certain forms, it may be desirable to provide continuous delivery of one or more compounds to a patient in need thereof. For topical applications, repeated application can be done or a patch can be used to provide continuous administration of the compounds over an extended period of time.

"Buffers" are used to control pH of a composition. Preferably, the buffers buffer the composition from a pH of about 4 to a pH of about 7.5, more preferably from a pH of about 4 to a pH of about 7, and most preferably from a pH of about 5 to a pH of about 7. In a preferred form, the buffer is triethanolamine."Emollients" are an externally applied agent that softens or soothes skin and are generally known in the art and listed in compendia, such as the "Handbook of Pharmaceutical Excipients", 4th Ed., Pharmaceutical Press, 2003. These include, without limitation, almond oil, castor oil, ceratonia extract, cetostearoyl alcohol, cetyl alcohol, cetyl esters wax, cholesterol, cottonseed oil, cyclomethicone, ethylene glycol palmitostearate, glycerin, glycerin monostearate, glyceryl monooleate, isopropyl myristate, isopropyl palmitate, lanolin, lecithin, light mineral oil, medium-chain triglycerides, mineral oil and lanolin alcohols, petrolatum, petrolatum and lanolin alcohols, soybean oil, starch, stearyl alcohol, sunflower oil, xylitol and combinations thereof. In some forms, the emollients are ethylhexylstearate and ethylhexyl palmitate.

"Emulsifiers" are surface active substances which promote the suspension of one liquid in another and promote the formation of a stable mixture, or emulsion, of oil and water. Common emulsifiers are: metallic soaps, certain animal and vegetable oils, and various polar compounds. Suitable emulsifiers include acacia, anionic emulsifying wax, calcium stearate, carbomers, cetostearyl alcohol, cetyl alcohol, cholesterol, diethanolamine, ethylene glycol palmitostearate, glycerin monostearate, glyceryl monooleate, hydroxpropyl cellulose, hypromellose, lanolin, hydrous, lanolin alcohols, lecithin, medium-chain triglycerides, methylcellulose, mineral oil and lanolin alcohols, monobasic sodium phosphate, monoethanolamine, nonionic emulsifying wax, oleic acid, poloxamer, poloxamers, polyoxyethylene alkyl ethers, polyoxyethylene castor oil derivatives, polyoxyethylene sorbitan fatty acid esters, polyoxyethylene stearates, propylene glycol alginate, self-emulsifying glyceryl monostearate, sodium citrate dehydrate, sodium lauryl sulfate, sorbitan esters, stearic acid, sunflower oil, tragacanth, triethanolamine, xanthan gum and combinations thereof. In some forms, the emulsifier is glycerol stearate.

"Penetration enhancers" are known in the art and include, but are not limited to, fatty alcohols, fatty acid esters, fatty acids, fatty alcohol ethers, amino acids, phospholipids, lecithins, cholate salts, enzymes, amines and amides, complexing agents (liposomes, cyclodextrins, modified celluloses, and diimides), macrocyclics, such as macrocylic lactones, ketones, and anhydrides and cyclic ureas, surfactants, N-methyl pyrrolidones and derivatives thereof, DMSO and related compounds, ionic compounds, azone and related compounds, and solvents, such as alcohols, ketones, amides, polyols (e.g., glycols). Examples of these classes are known in the art.

"Preservatives" can be used to prevent the growth of fungi and microorganisms. Suitable antifungal and antimicrobial agents include, but are not limited to, benzoic acid, butylparaben, ethyl paraben, methyl paraben, propylparaben, sodium benzoate, sodium propionate, benzalkonium chloride, benzethonium chloride, benzyl alcohol, cetylpyridinium chloride, chlorobutanol, phenol, phenylethyl alcohol, and thimerosal.

"Surfactants" are surface-active agents that lower surface tension and thereby increase the emulsifying, foaming, dispersing, spreading and wetting properties of a product. Suitable non-ionic surfactants include emulsifying wax, glyceryl monooleate, polyoxyethylene alkyl ethers, polyoxyethylene castor oil derivatives, polysorbate, sorbitan esters, benzyl alcohol, benzyl benzoate, cyclodextrins, glycerin monostearate, poloxamer, povidone and combinations thereof. In some forms, the non-ionic surfactant is stearyl alcohol.

### (a) Emulsions

An emulsion is a preparation of one liquid distributed in small globules throughout the body of a second liquid. In particular forms, the non-miscible components of the emulsion include a lipophilic component and an aqueous component. The dispersed liquid is the discontinuous phase, and the dispersion medium is the continuous phase. When oil is the dispersed liquid and an aqueous solution is the continuous phase, it is known as an oil-in-water emulsion, whereas when water or aqueous solution is the dispersed phase and oil or oleaginous substance is the continuous phase, it is known as a water-in-oil emulsion. Either or both of the oil phase and the aqueous phase may contain one or more surfactants, emulsifiers, emulsion stabilizers, buffers, and other excipients. Preferred excipients include surfactants, especially nonionic surfactants; emulsifying agents, especially emulsifying waxes; and liquid non-volatile non-aqueous materials, particularly glycols such as propylene glycol. The oil phase may contain other oily pharmaceutically approved excipients. For example, materials such as hydroxylated castor oil or sesame oil may be used in the oil phase as surfactants or emulsifiers.

The oil phase may consist at least in part of a propellant, such as an HFA propellant. Either or both of the oil phase and the aqueous phase may contain one or more surfactants, emulsifiers, emulsion stabilizers, buffers, and other excipients. Preferred excipients include surfactants, especially non-ionic surfactants; emulsifying agents, especially emulsifying waxes; and liquid non-volatile non-aqueous materials, particularly glycols such as propylene glycol. The oil phase may contain other oily pharmaceutically approved excipients. For example, materials such as hydroxylated castor oil or sesame oil may be used in the oil phase as surfactants or emulsifiers.

A sub-set of emulsions are the self-emulsifying systems. These drug delivery systems are typically capsules (hard shell or soft shell) comprised of the drug dispersed or dissolved in a mixture of surfactant(s) and lipophilic liquids such as oils or other water immiscible liquids. When the capsule is exposed to an aqueous environment and the outer gelatin shell dissolves, contact between the aqueous medium and the capsule contents instantly generates very small emulsion droplets. These typically are in the size range of micelles or nanoparticles. No mixing force is required to generate the emulsion as is typically the case in emulsion formulation processes.

### (b) Lotions

A lotion can contain finely powdered substances that are in soluble in the dispersion medium through the use of suspending agents and dispersing agents. Alternatively, lotions can have as the dispersed phase liquid substances that are immiscible with the vehicle and are usually dispersed by means of emulsifying agents or other suitable stabilizers. In some forms, the lotion is in the form of an emulsion having a viscosity of between 100 and 1000 centistokes. The fluidity of lotions permits rapid and uniform application over a wide surface area. Lotions are typically intended to dry on the skin leaving a thin coat of their medicinal components on the skin's surface.

### (c) Creams

Creams may contain emulsifying agents and/or other stabilizing agents. In some forms, the formulation is in the form of a cream having a viscosity of greater than 1000 centistokes, typically in the range of 20,000-50,000 centistokes. Creams are often time preferred over ointments, as they are generally easier to spread and easier to remove. The difference between a cream and a lotion is the viscosity, which is dependent on the amount/use of various oils and the percentage of water used to prepare the formulations. Creams are typically thicker than lotions, may have various uses and often one uses more varied oils/butters, depending upon the desired effect upon the skin. In a cream formulation, the water-base percentage is about 60-75 % and the oil-base is about 20-30 % of the total, with the other percentages being the emulsifier agent, preservatives and additives for a total of 100 %.

### (d) Ointments

Examples of suitable ointment bases include hydrocarbon bases (e.g., petrolatum, white petrolatum, yellow ointment, and mineral oil); absorption bases (hydrophilic petrolatum, anhydrous lanolin, lanolin, and cold cream); water-removable bases (e.g., hydrophilic ointment), and water-soluble bases (e.g., polyethylene glycol ointments). Pastes typically differ from ointments in that they contain a larger percentage of solids. Pastes are typically more absorptive and less greasy that ointments prepared with the same components.

### (e) Gels

Gels are semisolid systems containing dispersions of small or large molecules in a liquid vehicle that is rendered semisolid by the action of a thickening agent or polymeric material dissolved or suspended in the liquid vehicle. The liquid may include a lipophilic component, an aqueous component or both. Some emulsions may be gels or otherwise include a gel component. Some gels, however, are not emulsions because they do not contain a homogenized blend of immiscible components. Suitable gelling agents include, but are not limited to, modified celluloses, such as hydroxypropyl cellulose and hydroxyethyl cellulose; Carbopol homopolymers and copolymers; and combinations thereof. Suitable solvents in the liquid vehicle include, but are not limited to, diglycol monoethyl ether; alklene glycols, such as propylene glycol; dimethyl isosorbide; alcohols, such as isopropyl alcohol and ethanol. The solvents are typically selected for their ability to dissolve the drug. Other additives, which improve the skin feel and/or emolliency of the formulation, may also be incorporated. Examples of such additives include, but are not limited, isopropyl myristate, ethyl acetate, C₁₂-C₁₅ alkyl benzoates, mineral oil, squalane, cyclomethicone, capric/caprylic triglycerides, and combinations thereof.

### (f) Foams

Foams consist of an emulsion in combination with a gaseous propellant. The gaseous propellant consists primarily of hydrofluoroalkanes (HFAs). Suitable propellants include HFAs such as 1,1,1,2-tetrafluoroethane (HFA 134a) and 1,1,1,2,3,3,3-heptafluoropropane (HFA 227), but mixtures and admixtures of these and other HFAs that are currently approved or may become approved for medical use are suitable. The propellants preferably are not hydrocarbon propellant gases, which can produce flammable or explosive vapors during spraying. Furthermore, the compositions preferably contain no volatile alcohols, which can produce flammable or explosive vapors during use.

### III. METHODS OF USE

The methods disclosed herein use two approaches to counter bacterial resistance to antibacterials. First, the methods use the compounds disclosed herein in combination with existing antibiotics. This strategy increases the hurdles for the bacteria to acquire the required resistance factors to survive, simultaneously slowing down the rise of the resistant strains. The methods described herein preserve the use of the existing antibiotics, which will prolong the clinical life span of the use of those antibiotics, providing time for development of a new generation of antibiotics. Not only does this combination treatment mount a greater hurdle for emergence of resistance strains, the compounds disclosed herein potentiate the effect of the co-administered antibiotic. A second approach is targeting virulence factors of the bacteria. Since virulence factors are auxiliary genes for the bacterial survival, having those genes inactivated by chemical genetics methods will not pose direct growth challenges for the bacteria. This in return will reduce the likelihood of developing an escape mutant, therefore reducing the development of resistant strains.

Accordingly, some forms disclose methods of treating or preventing microbial infection, such as but not limited to, *Staphyloccous aureus, Pseudomonas aeruginosa, Listeria monocytogenes, Burkholderia cepacia, Escherichia coli, Enterococcus faecalis, Streptococcus pneumoniae* infection, in a subject, by administering an effective amount of one or more compound according to any of formulas (Compound 2- compound 82) to a subject in need thereof. In some forms, the methods further comprise administering an effective amount of at least one antibiotic, such as, e.g., gentamicin, amikacin, kanamycin, neomycin, spectinomycin, neamine or any combination thereof. The antibiotic can be administered at the same time, before, or after the compound administration. Further, the administration of the antibiotic and disclosed compound(s) can be concurrently in the form of an administered pharmaceutical composition.

Administration may be locally (confined to a single cell or tissue) and/or 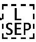 systemically in the subject. It may be desirable to administer the compounds/antibiotics and disclosed pharmaceutical compositions locally to the area in need of treatment, such as areas of infection. This method of administration may be achieved by, for example, and not by way of limitation, local infusion during surgery, topical application (e.g., in conjunction with a wound dressing after surgery), by injection, by catheter, or by means of an implant (e.g., a porous membrane).

In some forms, the compounds/antibiotics or pharmaceutical composition can be delivered in a controlled release system. Such methods may include the use of a pump for administration (e.g., use of an intravenous drip). In another form, a controlled release system can be placed in the proximity of the therapeutic target (e.g., site of infection), requiring only a fraction of the dose required if dosed systemically.

In other forms the disclosed small molecules serve as lead compounds for further modifications to create more potent and specific treatments.

### A. Microbes

Exemplary microbes that can be inhibited with the disclosed compounds include, but are not limited to, *Staphylococcus aureus, Pseudomonas aeruginosa, Burkholderia cepacia Enterococcus faecalis, Enterococcus faecium* and *Streptococcus pneumoniae.* Preferred forms include methicillin-resistant strains of *Staphylococcus aureus* and multidrug resistant *Listeria monocytogenes, Pseudomonas aeruginosa, Burkholderia cepacia, Enterococcus faecalis, Streptococcus pneumoniae.*

Other bacteria that can be treated include, but are not limited to *Acinteobacter, Antinomies, Anabaena, Bacillus, Bacteroides, Bdellovibrio, Bordetella, Borrelia, Campylobacter, Caulobacter, Chlamydia, Chlorobium, Chromatium, Clostridium, Corynebacterium, Cytophaga, Deinococcus, Enterobacter, Enterococcus, Escherichia, Francisella, Halobacterium, Heliobacter, Haemophilus, Hemophilus influenza type B (HIB), Hyphomicrobium, Klebsiella, Legionella, Leptspirosis, Listeria, Meningococcus A, B and C, Methanobacterium, Micrococcus, Myobacterium, Mycoplasma, Myxococcus, Neisseria, Nitrobacter, Oscillatoria, Prochloron, Proteus, Pseudomonas, Phodospirillum, Rickettsia, Salmonella, Shigella, Spirillum, Spirochaeta, Staphylococcus, Streptococcus, Streptomyces, Sulfolobus, Thermoplasma, Thiobacillus, and Treponema, Vibrio, and Yersinia.*

In one preferred form the compositions disclosed herein are used as a topical antibacterial medication for skin infections caused by methicillin-resistant *Staphylococcus aureus.* Methicillin-resistant *Staphylococcus aureus* (MRSA) is a type of bacteria that is resistant to many antibiotics. The CDC encourages clinicians to consider MRSA in the differential diagnosis of skin and soft tissue infections (SSTIs) compatible with *S. aureus* infections, especially those that are purulent (fluctuant or palpable fluid- filled cavity, yellow or white center, central point or "head," draining pus, or possible to aspirate pus with needle or syringe). A patient's presenting complaint of "spider bite" should raise suspicion of an *S. aureus* infection. In the community, most MRSA infections are skin infections. In other forms, the compositions can be used to treat systemic infections of lungs or blood caused by methicillin-resistant *Staphylococcus aureus* in hospitalized or immunocompromised patients. In medical facilities, MRSA causes life-threatening bloodstream infections, pneumonia and surgical site infections.

The spectrum of disease caused by MRSA appears to be similar to that of *Staphylococcus aureus* in the community. Soft tissue infections (SSTIs), specifically furuncles (abscessed hair follicles or "boils"), carbuncles (coalesced masses of furuncles), and abscesses, are the most frequently reported clinical manifestations. The most common manifestations of community associated-MRSA are simple skin infections, such as impetigo, boils, abscesses, folliculitis, and cellulitis. Rarer, but more serious manifestations can occur, such as necrotizing fasciitis and pyomyositis (most commonly found in the tropics), necrotizing pneumonia, infective endocarditis (which affects the valves of the heart), and bone and joint infections. Additional conditions include severe or extensive disease (e.g., involving multiple sites of infection) or rapid progression in presence of associated cellulitis, signs and symptoms of systemic illness, associated comorbidities or immunosuppression, extremes of age, abscess in an area difficult to drain (e.g., face, hand, and genitalia), associated septic phlebitis, and lack of response to incision and drainage alone, purulent cellulitis, hospitalized patients with complicated SSTI (cSSTI; defined as patients with deeper soft-tissue infections, surgical/traumatic wound infection, and infected ulcers and burns), osteomyelitis, device-related osteoarticular infections, children with minor skin infections (such as impetigo) and secondarily infected skin lesions (such as eczema, ulcers, or lacerations). The compositions disclosed herein can also be used to treat MRSA infections of the CNS, which include, but are not limited to Meningitis, Brain abscess, subdural empyema, spinal epidural abscess (reviewed in Liu, et al., Clin Infect Dis., 52(3):e18-55 (2011)).

The compositions disclosed herein can also be used to treat infections cause by strains of *Escherichia coli* (drug resistant or otherwise) in subjects especially in immunocompromised patients. *Escherichia coli* is one of the most frequent causes of many common bacterial infections, including cholecystitis, bacteremia, cholangitis, urinary tract infections other clinical infections such as neonatal meningitis and pneumonia. For example, the compositions can be used treat conditions caused by community- and/or hospital-acquired urinary tract infections (UTI's) caused by strains of *Escherichia coli* (drug resistant or otherwise) in immunocompromised patients.

Other antibiotic-resistant microorganisms that can be targeted with the compounds disclosed herein include *Streptococcus pneumoniae, Campylobacter*, *Neisseria gonorrhoeae*, *Salmonella* (including drug-resistant non-typhoidal *Salmonella* and drug-resistant *Salmonella* serotype typhi), *Shigella*, Vancomycin-resistant *Enterococcus* (VRE), Vancomycin-resistant *Staphylococcus aureus* (VRSA), Erythromycin-resistant Group A *Streptococcus,* Clindamycin-resistant Group B *Streptococcus,* Carbapenem-resistant *Enterobacteriaceae* (CRE), drug-resistant tuberculosis, Extended spectrum *Enterobacteriaceae* (ESBL), multidrug-resistant *Acinetobacter* (including MRAB), *Clostridium difficile*, *Enteropathogenic E. coli* (EPEC), *Pseudomonas aeruginosa*, *H. pylori*, *Streptococcus anginosus* and *Uropathogenic E. coli* (UPEC).

### B. Method of Treatment 1. Infections

The disclosed compounds and derivatives thereof and combinations thereof can be administered as described above in an amount effective to ameliorate or treat symptoms resulting from infections caused by microbes discussed above, infecting a subject, preferably a human subject. The infection can be systemic or local. The infection can be an infection of the skin, eye, throat, nose, or ear, or of any organ including but not limited to lungs, intestine, heart, brain, and liver. The infection can be an abscess or a wound.

In certain forms, it may be desirable to provide continuous delivery of one or 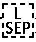 more compounds to a patient in need thereof. For intravenous or intra-arterial routes, this can be accomplished using drip systems, such as by intravenous administration. For topical applications, repeated application can be done or a patch can be used to provide continuous administration of the compounds over an extended period of time.

Because of the potentiating effect of the disclosed compounds, in some form, the amount of a separately, simultaneously, or sequentially administered one or more antimicrobial agents (including one or more antibiotics) may be less than a therapeutically effective or therapeutically optimal dose of the one or more antimicrobial agents (including one or more antibiotics) when administered to the patient or animal that is not in receipt of the disclosed compounds. In some forms, administered one or more antimicrobial agents may be 5%, 10%, 15%, 20%, 30%, 40%, 50%, 60%, 70%, 80% or more, less than a therapeutically effective or therapeutically optimal dose of the one or more antibiotics when administered to the patient or animal that is not in receipt of the disclosed compounds. In another form, the treatment duration of the patient receiving the treatment or prophylaxis of the second form may be 5%, 10%, 15%, 20%, 30%, 40%, 50%, 60%, 70%, 80% or more, less than the treatment duration required when the patient or animal is not in receipt of the disclosed compound.

### 2. Additional methods of use

Furthermore, it would be understood by those skilled in the art that the therapeutic methods described would not only apply to treatment in a subject, but could be applied to cell cultures, organs, tissues, or individual cells *in vivo*, *ex vivo* or *in vitro.*

### IV . KITS

Moreover, if a packaging material is utilized to package the pharmaceutical, compositions it may be biologically inert or lack bioactivity, such as plastic polymers, silicone, etc. and may be processed internally by the subject without affecting the effectiveness of the compound/antibiotic packaged and/or delivered therewith.

Additionally, the compounds and/or pharmaceutical compositions may be packaged with antibiotics as disclosed herein, preferred antibiotics of which include, but are not limited to, gentamicin, amikacin, kanamycin, neomycin, spectinomycin, neamine or any combination thereof.

Without further elaboration, it is believed that one skilled in the art can, using the preceding description, utilize the disclosed compounds, compositions, and methods to their fullest extent. The following examples are offered by way of illustration, not by way of limitation. While specific examples have been provided, the above description is illustrative and not restrictive. Any one or more of the features of the described forms can be combined in any manner with one or more features of any other described forms. Furthermore, many variations of the disclosed compounds, compositions, and methods will become apparent to those skilled in the art upon review of the specification. 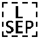 The methods, compounds, and compositions herein described are further illustrated in the following examples, which are provided by way of illustration and are not intended to be limiting.

Theoretical aspects are presented with the understanding that Applicants do not seek to be bound by the theory presented. All parts or amounts, unless otherwise specified, are by weight.

### Examples

### Materials and Methods

*Bacterial strains*: The Mu3 (ATCC 700698), USA300, COL, are methicillin- resistant strains of *Staphylococcus aureus.* The Newman, ATCC29213, RN4220, RN6390 are methicillin-sensitive strains. The AE-052, ST-45-502, ST-239-IIIA-503, ST-239-III-AH-504 are clinical isolates from Queen Mary Hospital, Hong Kong. *Listeria monocytogenes (L. monocytogenes)*, *Pseudomonas aeruginosa (P. aeruginosa)* PA01, *Streptococcus pneumoniae (S. pneumonia) R6* are reference strain, *Burkholderia cepacia*, *Enterococcus faecalis*, *Escherichia coli* (NDM-1) are multi drug resistant clinical isolates from Queen Mary Hospital, Hong Kong., *Escherichia coli* (IMP) is a laboratory strain [remove]. All the bacteria are culture in corresponding growth medium Brain Heart Infusion (BHI) Broth (OXOID), Mueller Hinton (MH) broth (BD), or Cation Adjusted Mueller Hinton (CAMH) broth (BD) at 37 °C.

*Drug libraries and chemicals*: Structurally diverse small-molecule compound library is used for the high throughput screening. Small molecule compounds were purchased from Chembridge. Gentamicin (Sigma-Aldrich), kanamycin (Sigma-Aldrich), amikacin (Sigma-Aldrich), spectinomycin (Sigma-Aldrich), neomycin (Sigma-Aldrich), neamine (Sigma-Aldrich) are used in the minimum inhibitory concentration (MIC) test, high throughput screening, paper disc assays and checkerboard assay.

*Minimal inhibitory concentration (MIC) test.* Assays to determine the MIC of bacteria to the antibiotics are performed with a broth micro-dilution method in 96-wells microtitre plates with appropriate growth medium broths. For the determination of MIC of small-molecule compounds, the compounds are first subjected to two-fold serial dilution in DMSO, 1µL of each diluent is added to the 96 well microtitre plates to give the desired concentration. All tests are performed in triplicates and repeated.

*High throughput screening (HTS).* Primary HTS screening is performed with customized robotic screening platform, fully automated Beckman Coulter Core system (Fullerton), integrated with liquid handler, washer, multidrop liquid dispenser (Thermo Scientific), Kendro robotics CO₂ incubator (Thermo Fisher Scientific), and DTX 880 multimode plate reader (Beckman coulter). The chemical library is pre-aliquoted into 384- well microtiter plates and upon screening, will give a final concentration of 20µg/mL of compound in 50µL BHI medium per well. All screening is done in 384-well polystyrene microtiter plates (Greiner Bio-One). Overnight culture of Mu3 is washed with BHI medium 3 times, diluted and plated to give 5 × 10⁵ CFU/mL per well. The plates are incubated at 37 °C for 16 hours and growth is monitored by optical density reading at 595nm using an automated DTX 880 multimode detector. Screening using BHI medium is designated as the background screen. Potential chemical compounds showing synergistic properties with various antibiotics are screened using sub-MIC concentration antibiotic containing medium. Sub MIC concentration of antibiotics is selected for screening. BHI containing a final concentration for gentamicin 156.25µg/mL is used correspondingly for individual experiments. Data analysis is done by custom designed data analysis software programmed by Jason Madar, Capilano University. Screening results with various antibiotics is normalized with the background screening to calculate the percentage of growth inhibition. Compounds with potential antibiotic activity enhancement effects are selected for secondary screening to validate the hits.

Selected compounds are prepared in different concentrations similar to the primary screening preparation. Identical experimental procedures are performed as primary screening procedures, except different concentrations of selected compound are tested. Compounds demonstrating bacterial growth inhibition in the presence of sub-MIC antibiotics are selected for more detailed characterization.

*Checkerboard assay.* The Checkerboard assay is performed in 96-well microtitre plates. Chemical compound MIC is first determined as previously described herein. The antibiotics are 2 fold serially diluted along the ordinate, the compounds are added along the abscissa to generate the checkerboard concentration gradients between the compounds and the antibiotics. Bacterial culture are topped and incubated overnight. Bacterial growth is monitored by the OD absorbance at 595nm.

*Cytotoxicity assay.* MTT assays are performed for selected compounds on Vero cells according to product instructions.

*Paper disc diffusion assay.* The paper disc diffusion assays are performed by impregnating 10µL of 5mM compounds or appropriate amount of antibiotics in to the 8mm diameter filter paper disc. The discs are then placed on a uniform swabbed lawn of bacteria on BHI agar and grown overnight at 37°C. The larger zone of inhibition correlates with smaller MIC.

*Paper disc diffusion and lux assays.* A single colony of bioluminescent *S. aureus* carrying the hla-promoter lux reporting system was resuspended in 200µl of sterile water, diluted 1000-fold into 0.7% (w/v) BHI agar and overlaid onto BHI agar plates. 10µl of the 50mM analogues compounds dissolved in DMSO were impregnated to paper disc and placed on the lawn of bacteria. The plates were incubated overnight at 37°C. The luminescence was detected with Xenogen IVIS 100 in vivo imaging system (Xenogen, Alameda, CA).

*Compound selectivity optimization.* The lead compound from the primary screens is selected and analogues of the compound are searched and ordered for better performing compounds. The MIC and MTT for the analogues are determined. The selective index is calculated for each compound by comparing the compound toxicity versus the compound efficacy.

*In vivo experiments.* All animal experiments were carried out following the Laboratory Animal Unit (LAU) of the University of Hong Kong's regulation. 6-8 weeks old female BALB/c mice were infected with 4×10⁸ CFU of *S. aureus.* Compound 3 or injection buffer control (PBS with 5% DMSO and 2% Tween 80), were pretreated one day before infection, two dosages were given per day post infection. 3 days after infection, the animals were euthanized; kidneys, livers and spleens were homogenized in PBS, and plated on BHI agar for quantitative bacterial culture.

### Results

Preliminary testing of the MIC value of three antibiotics (Gentamicin, Ceftriaxone, and Levofloxacin) against MRSA Mu3 was performed according to microbroth dilution methods. This was to establish the reference for the appropriate amount of antibiotics to be used for HTS for hit identification of compounds that could have synergistic activities or potentiate the antibiotic's antimicrobial effect. The MIC value of Gentamicin against MRSA Mu3 was determined to be 1000µg/mL. Adjusted sub MIC concentration of antibiotics was used for the screenings; a final concentration for gentamicin 156.25µg/mL was used for individual screening experiment.

High throughput screening of the structurally diverse small molecule library at a final concentration of 20µg/mL was carried out against the Mu3 strain in plain BHI. Growth was monitored by the OD readings. This screening was to initiate the background antimicrobial activities of each compound against the Mu3 strain. This was later compared against the individual screenings containing the sub MIC concentration of antibiotic. Antibiotic potentiating compounds ideally have no background antimicrobial activities in the background screen and exhibit growth inhibition in the presence of sub MIC concentration of antibiotic. Primary hit compounds were isolate through the initial screenings after the normalization of the sub MIC screens with the background screen. The selected compounds were subjected to the secondary screening to refine and validate the combinatory effect of the small molecules with the antibiotic.

Among which Compound 13 was finalized for more in depth studies. This compound shows no sign of MIC, up to an undetermined concentration, which the compound solubility limits the higher concentrations to be tested.

Checkerboard assays were performed with the lead compound, compound 13, against gentamicin (Figure 1) and other aminoglycosides, amikacin (Figure 2), kanamycin (Figure 3), neomycin (Figure 4), spectinomycin (Figure 5), neamine (6). All of the antibiotics tested can be potentiated by compound 13 and show a combinatory improvement.

Compound 13 can also extend its combinatory effect with gentamicin in other *staphylococcus aureus (S. aureus)* strains. The effects could be seen with gentamicin sensitive strains such as ATCC 29213 (Figure 7), Newman (Figure 8), RN4220 (Figure 9), USA300 (Figure 10), COL (Figure 11), RN6390 (Figure 12), ST-45-502 (Figure 13) and AE-052 (Figure 14), as well as well as in gentamicin resistant strains such as ST- 239-III-AH-504 (Figure 15), ST-239-III-AH-503 (Figure 16).

Compound 13 demonstrated its aminoglycoside potentiating properties in *S. aureus,* structural analogues of compound 13 were purchased for lead compound optimization. Secondary like screening was performed with different concentrations of each analogue compounds were tested with different gentamicin concentration, 50µM, 20µM and 5µM compound concentration were used against, BHI containing, 0µg/ml, 100µg/ml and 200µg/ml gentamicin. Analogues showing activities in the screenings were selected (Figure 17, 18, 19), summarized in Table 1.

**Table 1. Summarizing the numerical relative growth of the analogues secondary screening at various concentrations with various concentration of gentamicin.**

| [Cpd] [Genta] | 5µM | | | 20µM | | | 50µM | | | Virulence |
|---|---|---|---|---|---|---|---|---|---|---|
| | BHI only | 100ug /ml | 200ug /ml | BHI only | 100ug /ml | 200ug /ml | BHI only | 100ug /ml | 200ug /ml | *hla* promoter |
| 1 | 1.38 | 1.29 | 1.45 | 1.22 | 1.1 | 1.05 | 1 | 1.11 | 1.01 | - |
| 2 | 1.15 | 1.1 | 1.2 | 1.08 | 0.93 | 0.65 | 1.07 | 0.62 | *0.24 | + |
| 3 | 1.18 | 1.12 | 1.18 | 1.09 | 0.94 | 0.4 | 0.88 | *0.09 | *0.08 | --- |
| 4 | 1.1 | 1.07 | 1.16 | 1.05 | 1.04 | 0.89 | 1.05 | 0.76 | 0.2 | - |
| 5 | 1.13 | 1.08 | 1.06 | 1.08 | 1.06 | 0.92 | 1.07 | 1.1 | 0.8 | - |
| 6 | 1.2 | 1.13 | 1.11 | 1.14 | 0.61 | *0.1 | 0.79 | *0.2 | *0.11 | - |
| 7 | 1.22 | 0.67 | *0.34 | 1.02 | 0.4 | 0.15 | 0.87 | 0.45 | *0.23 | - |
| 8 | 1.12 | 1.06 | 1.13 | 1.07 | 1.07 | 1.09 | 1.1 | 1.12 | 1.02 | -- |
| 9 | 1.15 | 1.09 | 1.06 | 1 | 0.92 | 0.78 | 1.06 | 1.02 | 0.76 | + |
| 10 | 1.11 | 1.04 | 1.06 | 1.03 | 1.03 | 0.96 | 1 | 0.73 | 0.41 | + |
| 11 | 1.2 | 1.12 | 1.09 | 1.04 | 0.84 | 0.82 | 1.12 | 1.07 | 0.89 | + |
| 12 | 1.14 | 1.12 | 1.27 | 1.12 | 1.11 | 1.1 | 1.14 | 1.16 | 0.98 | + |
| 13 | 0.99 | 1 | 0.89 | 0.97 | *0.09 | *0.09 | 0.88 | *0.09 | *0.09 | - |
| 14 | 0.95 | 0.94 | 1.03 | 0.95 | 0.97 | 0.96 | 1.02 | 1.04 | 0.93 | ++ |
| 15 | 1.02 | 1.01 | 1 | 0.97 | 0.52 | *0.09 | 0.79 | *0.18 | *0.11 | + |
| 16 | 0.94 | 0.95 | 0.99 | 0.94 | 0.95 | 0.94 | 0.96 | 0.95 | 0.88 | ++ |
| 17 | 0.99 | *0.35 | *0.1 | 0.74 | *0.09 | *0.09 | 0.43 | *0.14 | *0.13 | - |
| 18 | 0.98 | 0.97 | 0.9 | 0.85 | 0.8 | 0.76 | 0.97 | 0.88 | 0.7 | - |
| 19 | 0.99 | 0.96 | 0.83 | 0.91 | *0.1 | *0.09 | 0.76 | *0.09 | *0.08 | -- |
| 20 | 1.01 | 0.96 | 0.88 | 0.78 | *0.14 | *0.09 | 0.91 | *0.17 | *0.14 | - |
| 21 | 0.97 | 1 | 0.94 | 0.96 | 0.96 | 0.95 | 0.99 | 1 | 0.96 | + |
| 22 | 1.08 | 1.08 | 1.05 | 1.01 | 0.99 | 0.88 | 0.94 | 0.91 | 0.52 | - |
| 23 | 1.07 | 1.09 | 1.08 | 0.97 | 0.94 | 0.79 | 1.1 | 1.05 | 0.71 | ++ |
| 24 | 0.96 | 0.94 | 0.95 | 0.93 | 0.92 | 0.73 | 0.92 | 0.72 | *0.32 | ++ |
| 25 | 0.96 | 0.95 | 0.92 | 0.91 | 0.89 | 0.62 | 0.95 | 0.91 | 0.42 | ++ |
| 26 | 0.94 | 0.94 | 0.99 | 0.89 | 0.91 | 0.92 | 0.95 | 0.91 | 0.9 | +++ |
| 27 | 0.92 | 0.93 | 0.91 | 0.87 | 0.88 | 0.55 | 0.81 | 0.88 | 0.53 | ++ |
| 28 | 0.94 | 0.93 | 0.91 | 0.81 | 0.56 | *0.14 | 0.51 | *0.09 | *0.19 | - |
| 29 | 0.96 | 0.94 | 0.89 | 0.9 | 0.67 | *0.39 | 0.89 | *0.11 | *0.08 | + |
| 30 | 0.98 | 0.98 | 0.95 | 0.88 | 0.89 | 0.51 | 0.83 | 0.6 | *0.18 | -- |
| 31 | 0.99 | 0.98 | 0.95 | 0.96 | 0.98 | 0.77 | 0.98 | 0.98 | 0.61 | -- |
| 32 | 0.99 | 1 | 0.96 | 0.95 | 0.96 | 0.93 | 0.97 | 0.96 | 0.91 | + |
| 33 | 0.99 | 1 | 1.02 | 1 | 0.97 | 0.97 | 1.01 | 0.97 | 0.93 | + |
| 34 | 1.12 | 0.9 | *0.24 | 1.09 | 0.67 | *0.33 | 1.13 | 1.16 | 0.57 | + |
| 35 | 0.98 | 0.96 | 1.01 | 0.96 | 0.95 | 0.9 | 0.97 | 0.96 | 0.45 | +++ |
| 36 | 0.95 | 0.92 | 0.83 | 0.9 | *0.09 | *0.09 | 0.92 | *0.11 | *0.11 | +++ |
| 37 | 0.93 | 0.94 | 0.94 | 0.92 | 0.91 | 0.83 | 0.96 | 0.9 | 0.57 | - |
| 38 | 0.93 | 0.95 | 0.96 | 0.91 | 0.93 | 0.94 | 0.96 | 0.94 | 0.9 | + |
| 39 | 0.91 | 0.89 | 0.9 | 0.82 | 0.89 | 0.9 | 0.89 | 0.86 | 0.85 | ++ |
| 40 | 0.91 | 0.91 | 0.92 | 0.88 | 0.89 | 0.91 | 0.91 | 0.89 | 0.85 | + |
| 41 | 0.94 | 0.92 | 0.93 | 0.9 | 0.9 | 0.93 | 0.9 | 0.92 | 0.87 | - |
| DMSO | 1 | 1 | 1 | 1 | 1 | 1 | 1 | 1 | 1 | +++ |
| 42 | 0.93 | 0.93 | 0.78 | 0.81 | 0.82 | 0.63 | 0.77 | 0.7 | *0.33 | + |
| 43 | 0.94 | 0.96 | 0.8 | 0.88 | 0.89 | 0.9 | 0.89 | 0.87 | 0.82 | ++ |
| 44 | 1.03 | 1.02 | 0.83 | 1.03 | 0.99 | 0.83 | 0.98 | 0.97 | 0.47 | ++ |
| 45 | 1.11 | 1.11 | 1.24 | 1.1 | 1.09 | 1.11 | 1.09 | 1.12 | 1.08 | +++ |
| 46 | 0.98 | 0.95 | 1 | 0.93 | 0.9 | 0.9 | 0.9 | 0.87 | 0.81 | +++ |
| 47 | 0.96 | 0.94 | 0.98 | 0.87 | 0.91 | 0.89 | 0.91 | 0.85 | 0.8 | +++ |
| 48 | 0.98 | 0.97 | 0.96 | 0.95 | 0.97 | 0.64 | 0.87 | 0.8 | *0.31 | - |
| 49 | 0.95 | 0.95 | 0.86 | 0.7 | 0.42 | ^{∗}0.13 | 0.61 | *0.2 | *0.1 | - |
| 50 | 0.93 | 0.91 | 0.87 | 0.82 | 0.82 | 0.64 | 0.96 | 0.9 | 0.6 | ++ |
| 51 | 0.91 | 0.92 | 0.96 | 0.9 | 0.93 | 0.94 | 0.93 | 0.99 | 0.94 | - |
| 52 | 0.95 | 0.96 | 0.93 | 0.86 | 0.89 | 0.69 | 0.76 | 0.74 | *0.36 | - |
| 53 | 0.93 | 0.92 | 0.89 | 0.89 | 0.9 | 0.76 | 0.88 | 0.84 | *0.38 | + |
| 54 | 0.99 | 1.01 | 0.96 | 0.97 | 0.98 | 0.99 | 0.96 | 0.95 | 0.94 | ++ |
| 55 | 1.05 | 1.04 | 1.04 | 1.05 | 1 | 0.74 | 0.77 | 0.95 | 0.73 | - |
| 56 | 1.08 | 0.52 | **0.1 | **0.11 | **0.09 | **0.09 | **0.32 | **0.12 | **0.14 | + |
| 57 | 0.97 | 0.96 | 0.99 | 0.98 | 0.99 | 0.98 | 1 | 1.01 | 0.93 | +++ |
| 58 | 0.96 | 0.95 | 0.97 | 0.95 | 0.95 | 0.95 | 0.97 | 0.98 | 0.9 | +++ |
| 59 | 0.98 | 0.96 | 0.95 | 0.94 | 0.95 | 0.92 | 0.98 | 0.97 | 0.91 | +++ |
| 60 | 0.96 | 0.95 | 0.95 | 0.93 | 0.94 | 0.81 | 0.98 | 0.94 | 0.52 | ++ |
| 61 | 0.96 | 0.94 | 0.93 | 0.94 | 0.94 | 0.93 | 0.98 | 0.98 | 0.77 | +++ |
| 62 | 0.93 | 0.92 | 0.83 | 0.91 | 0.8 | *0.37 | 0.88 | *0.14 | *0.09 | +++ |
| 63 | 0.97 | 0.97 | 0.88 | 0.93 | 0.67 | *0.35 | 0.84 | *0.11 | *0.08 | - |
| 64 | 0.91 | 0.89 | 0.87 | 0.8 | 0.76 | 0.81 | 0.83 | 0.81 | 0.78 | +++ |
| 65 | 0.9 | 0.9 | 0.85 | 0.82 | 0.83 | 0.89 | 0.86 | 0.82 | 0.8 | +++ |
| 66 | 1.04 | 1.02 | 1.02 | 1.03 | 0.99 | 1 | 1.01 | 0.98 | 0.95 | + |
| 67 | 1.16 | 1.14 | 1.26 | 1.14 | 1.16 | 1.14 | 1.13 | 1.25 | 1.13 | ++ |
| 68 | 1 | 1 | 1.02 | 0.95 | 0.94 | 0.92 | 0.94 | 0.95 | 0.88 | +++ |
| 69 | 0.97 | 0.97 | 0.98 | 1.02 | 0.56 | *0.11 | 0.55 | *0.27 | ^{∗}0.23 | - |
| 70 | 0.97 | 0.97 | 0.99 | 1 | 0.98 | 0.94 | 1.02 | 1.04 | 0.97 | +++ |
| 71 | 0.97 | 0.98 | 1 | 0.98 | 0.95 | 0.96 | 0.97 | 0.97 | 0.92 | ++ |
| 72 | 0.95 | 0.95 | 0.94 | 0.93 | 0.86 | 0.84 | 0.93 | 0.85 | 0.81 | +++ |
| 73 | 0.94 | 0.93 | 0.92 | 0.85 | 0.88 | 0.9 | 0.87 | 0.85 | 0.81 | +++ |
| 74 | 0.97 | 0.96 | 0.97 | 0.97 | 0.95 | 0.94 | 0.91 | 0.85 | 0.85 | ++ |
| 75 | 0.96 | 0.96 | 0.98 | 0.98 | 0.98 | 0.95 | 0.99 | 0.97 | 0.95 | +++ |
| 76 | 0.95 | 0.96 | 0.93 | 1 | 0.97 | 0.95 | 0.95 | 0.95 | 0.92 | ++ |
| 77 | 1.02 | 1.01 | 1.01 | 1.01 | 1 | 1 | 1.02 | 1 | 0.95 | - |
| 78 | 1.26 | 1.24 | 1.38 | 1.28 | 1.28 | 1.21 | 1.18 | 1.21 | 0.74 | + |
| 79 | 1.1 | 1.09 | 1.14 | 1.13 | 1.11 | 1.09 | 0.91 | 1.15 | 1.07 | ++ |
| 80 | 1.1 | 0.49 | **0.12 | **0.16 | **0.09 | **0.09 | **0.33 | **0.15 | **0.13 | - |
| 81 | 1.05 | 1.03 | 0.98 | 1.03 | 0.68 | *0.24 | 0.98 | 0.4 | *0.1 | - |
| 82 | 1.03 | 0.98 | 0.61 | 0.78 | *0.09 | *0.09 | 0.99 | *0.13 | *0.11 | - |
| DMSO | 1 | 1 | 1 | 1 | 1 | 1 | 1 | 1 | 1 | +++ |

| | | | | | | | | | | |
|---|---|---|---|---|---|---|---|---|---|---|
| ^{∗}indicates the relative growth smaller than 40%, ** indicate compound oriented growth inhibition. +/- indicates induction or repression on *hla* promoter. | | | | | | | | | | |

Structure and function analysis was carried out to narrow down the functional core structure (Table 2, 3, 4, 5).

**Table 2. Structure function analysis of compounds with over 30% growth inhibitions under 50µM compound concentration and 200µg/ml gentamicin**

| 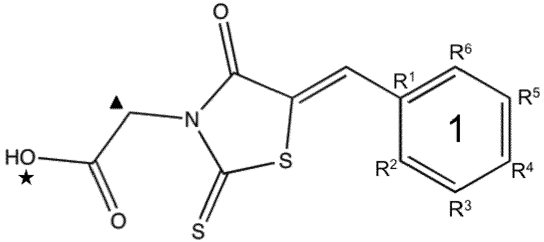 | | | |
|---|---|---|---|
| Compound No. | Side Chain | 50µM + 200µg/ml Gentamicin Growth inhibition Percentage | 20µM + 200µg/ml Gentamicin Growth inhibition Percentage |
| 2 | -Ring1 R4 addition | 76 | 35 |
| | 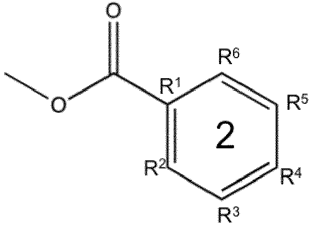 -Ring2 R4 addition Cl | | |
| 4 | -Ring1 R4 addition | 80 | 11 |
| | 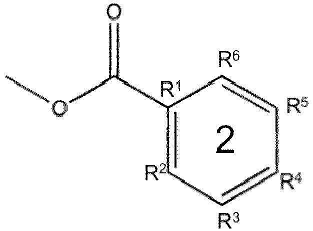 -Ring2 R2 addition | | |
| | Cl | | |
| | -Ring2 R4 addition Cl | | |
| 10 | 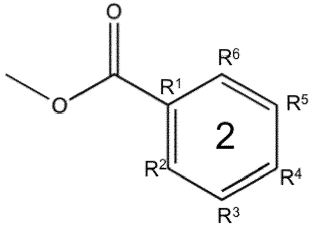 -Ring1 R4 addition | 59 | 4 |
| 28 | -Ring1 R2 addition | 81 | 86 |
| | 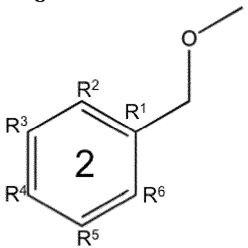 -Ring2 R4 addition -Cl | | |
| 29 | - Ring1 R4 addition | 92 | 61 |
| | 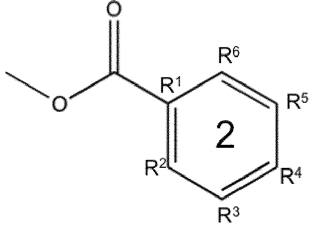 -Ring2 R5 addition CH₃ | | |
| 30 | - Ring1 R4 addition | 82 | 49 |
| | 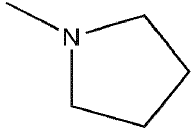 - Ring1 R5 addition F | | |
| 31 | - Ring1 R4 addition | 39 | 23 |
| | 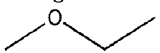 | | |
| 35 | - Ring1 R4 addition | 55 | 10 |
| | 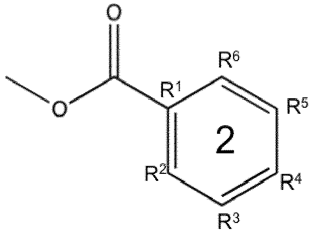 -Ring2 R4 addition F | | |
| 36 | - Ring1 R4 addition | 89 | 91 |
| | 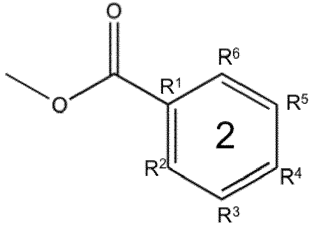 -Ring2 R4 addition I | | |
| 37 | -▲ addition CH₃ | 43 | 17 |
| | - Ring1 R4 addition | | |
| | 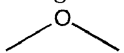 | | |
| 48 | -Ring1 R4 addition | 69 | 36 |
| | 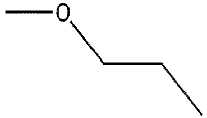 | | |
| 49 | -Ring1 R4 addition | 90 | 87 |
| | 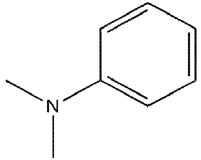 | | |
| 52 | -Ring1 R2 addition | 64 | 31 |
| | 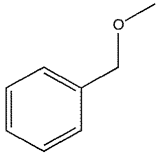 | | |
| 53 | -▲ addition -CH₃ | 62 | 24 |
| | - Ring1 R4 addition -Cl | | |
| 56 | -Ring1 R2 addition | 86 | 91 |
| | 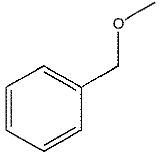 -Ring1 R5 addition Br | | |
| 60 | -Ring2 R5 addition | 48 | 19 |
| | 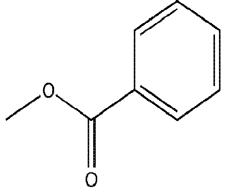 | | |
| 62 | - Ring1 R5 addition | 91 | 63 |
| | 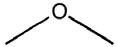 | | |
| | - Ring1 R4 addition | | |
| | 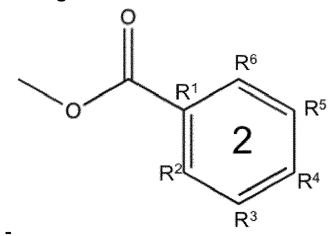 -Ring2 R3 addition -CH₃ | | |
| 80 | -Ring1 R2 addition | 87 | 91 |
| | 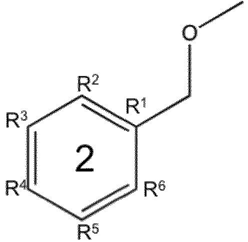 -Ring1 R5 addition | | |
| | -Br | | |
| | - Ring2 R6 addition -Cl | | |

Table 2 shows a structure function analysis of selected analogues with over 30% growth inhibitions with compound concentration of 50µM and 200µg/ml gentamicin. The analysis shows that some functional groups improves the combinatory growth inhibition effect and retains growth inhibition of *S. aureus* Mu3 at a lower gentamicin concentration.

**Table 3. Structure function analysis of compounds with over 30% growth inhibitions under 50µM compound concentration and 200µg/ml gentamicin**

| 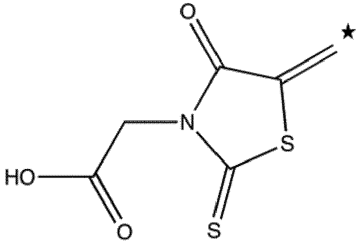 | | | |
|---|---|---|---|
| Compound No. | Side Chain | 50µM + 200µg/ml Gentamicin Growth inhibition Percentage | 20µM + 200µg/ml Gentamicin Growth inhibition Percentage |
| 42 | -★ addition | 67 | 37 |
| | 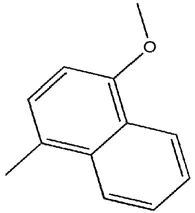 | | |
| 81 | -★ addition | 90 | 76 |
| | 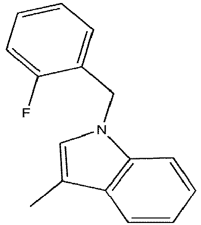 | | |

Table 3 shows a structure function analysis of selected analogues with over 30% growth inhibitions with compound concentration of 50µM and 200µg/ml gentamicin. The analysis shows that some functional groups improves the combinatory growth inhibition effect and retains growth inhibition of *S. aureus* Mu3 at a lower gentamicin concentration.

**Table 4. Structure function analysis of compounds with over 30% growth inhibitions under 50µM compound concentration and 200µg/ml gentamicin**

| 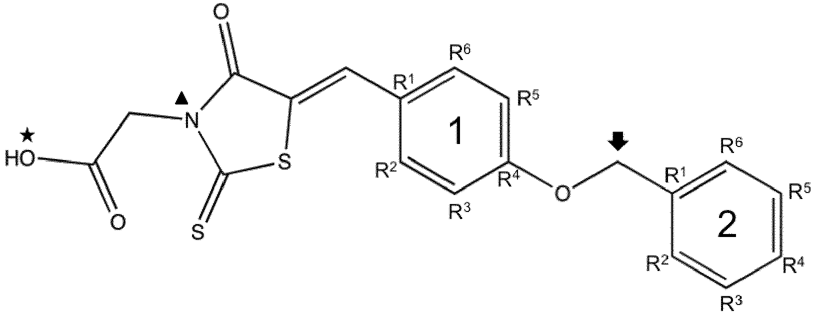 | | | |
|---|---|---|---|
| Compound No. | Side Chain | 50µM + 200µg/ml Gentamicin Growth inhibition Percentage | 20µM + 200µg/ml Gentamicin Growth inhibition Percentage |
| 3 | - | 92 | 60 |
| 6 | -Ring1 R5 addition | 89 | 90 |
| | 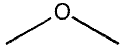 -Ring2 R4 addition -Cl | | |
| 7 | - 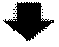 replaced with | 77 | 85 |
| | 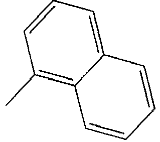 | | |
| 20 | -Ring1 R5 addition | 86 | 91 |
| | 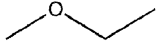 -Ring2 R4 addition -Cl | | |
| 22 | -Ring1 R5 addition | 48 | 12 |
| | 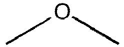 | | |
| 25 | -Ring1 R5 addition | 58 | 38 |
| | 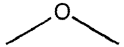 -Ring2 R2 addition -F | | |
| 34 | -Ring1 R5 addition | 43 | 67 |
| | 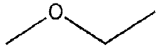 -Ring2 R4 addition -Cl | | |
| | -Ring2 R5 addition -Cl | | |
| 44 | -Ring1 R5 addition | 53 | 17 |
| | 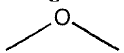 -Ring2 R4 addition -Br | | |
| 50 | Ring1 R5 addition | 40 | 36 |
| | 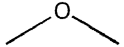 -Ring2 R2 addition -Cl | | |
| | -Ring2 R4 addition -F | | |
| 69 | -★ ester-linkage -CH₃ | 77 | 89 |
| 82 | -Ring2 R4 addition -Cl | 89 | 91 |

Table 4 shows a structure function analysis of selected analogues with over 30% growth inhibitions with compound concentration of 50µM and 200µg/ml gentamicin. The analysis shows that some functional groups improves the combinatory growth inhibition effect and retains growth inhibition of *S. aureus* Mu3 at a lower gentamicin concentration.

**Table 5. Structure function analysis of compounds with over 30% growth inhibitions under 50µM compound concentration and 200µg/ml gentamicin**

| 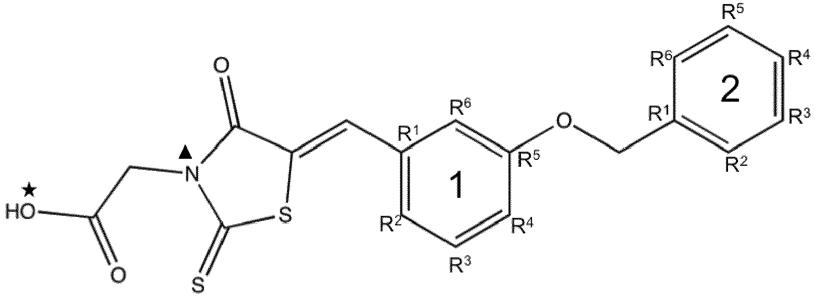 | | | |
|---|---|---|---|
| Compound No. | Side Chain | 50µM + 200µg/ml Gentamicin Growth inhibition Percentage | 20µM + 200µg/ml Gentamicin Growth inhibition Percentage |
| 13 | -Ring2 R4 addition | 91 | 91 |
| | -Cl | | |
| 15 | -Ring 1 R4 addition | 89 | 91 |
| | 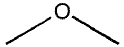 | | |
| | -Ring2 R4 addition | | |
| | -Cl | | |
| 17 | -Ring2 R4 addition | 87 | 91 |
| | -Cl | | |
| | -Ring2 R6 addition | | |
| | Cl | | |
| 18 | -Ring 1 R4 addition | 30 | 24 |
| | 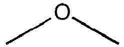 | | |
| | -Ring2 R4 addition | | |
| | -Cl | | |
| | -Ring2 R6 addition | | |
| | -Cl | | |
| 19 | -Ring2 R6 addition | 92 | 91 |
| | -Cl | | |
| 24 | -Ring1 R4 addition | 68 | 27 |
| | 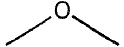 | | |
| 27 | -Ring1 R4 addition | 47 | 45 |
| | 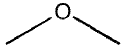 | | |
| | -Ring2 R6 addition | | |
| | -F | | |
| 63 | -Ring2 R6 addition | 92 | 65 |
| | -F | | |

Table 5 shows a structure function analysis of selected analogues with over 30% growth inhibitions with compound concentration of 50µM and 200µg/ml gentamicin. The analysis shows that some functional groups improves the combinatory growth inhibition effect and retains growth inhibition of *S. aureus* Mu3 at a lower gentamicin concentration.

Paper disc assays were used for direct assess of the potentiating effect of the selected compounds with potent activities. Checkerboard assays were carried out for the selected compounds, compound 13 (Figure 20), compound 17 (Figure 21), compound 19 (Figure 22), compound 20 (Figure 23), compound 36 (Figure 24) and compound 82 (Figure 25). The cytotoxicity of the 6 compounds was tested with MTT assays in Vero cells, results were summarized in Table 6.

**Table 6. Summary table of selected compound and their attributes.**

| Compound | Effective at <20µM with 100µM gentamicin | Combinatory effect with gentamicin | Cytotoxicity |
|---|---|---|---|
| 2 | | | |
| 3 | | | Low |
| 4 | | | |
| 6 | + | | |
| 7 | +++ | | |
| 13 | +++++ | Potentiating | High |
| 15 | ++ | | |
| 17 | +++++ | Potentiating | High |
| 19 | +++++ | Potentiating | Medium |
| 20 | +++++ | Potentiating | Medium/Low |
| 24 | | | |
| 28 | ++ | | |
| 29 | + | | |
| 30 | | | |
| 36 | +++++ | Potentiating | High |
| 42 | | | |
| 48 | | | |
| 49 | +++ | | |
| 52 | | | |
| 53 | | | |
| 56 | NA | | |
| 62 | | | |
| 63 | + | | |
| 69 | ++ | | |
| 80 | NA | | |
| 81 | ++ | | |
| 82 | +++++ | Potentiating | Low |

Paper disc diffusion and lux assays of the tested analogues compounds reveal the anti-virulence properties of some analogues, summarized on Table 1. Testing of other analogues of compound 3 identified some potential virulence factor inhibitor, Table 3.

**Table 7. Relative lux signal of selected compounds on the hla promoter, each compound are used at 25µM.**

| Compound No. | MIC (µM) | Relative lux signal |
|---|---|---|
| 3 | 500 | 0.00439 |
| 83 | 500 | 0.83975 |
| 84 | 500 | 0.7261 |
| 85 | 500 | 0.80121 |
| 86 | 500 | 0.6369 |
| 87 | 500 | 0.2657 |
| 88 | 500 | 0.97768 |
| 89 | 500 | 0.9655 |
| 90 | 500 | 1.1926 |
| 91 | 500 | 0.97015 |
| 92 | 500 | 0.83975 |

The selected compound 3 as treatment showed reduction of kidney (Figure 26), spleen (Figure 27), and liver (Figure 28) bacterial load in BALB/c mice infection model with Mu3 strains. Similar results were observed in BALB/c mice infection model with USA300 strains, reduction in spleen (Figure 29), and liver (Figure 30) bacterial load.

Activities of the selected Compound 82 on other bacteria were tested. Checkerboard assays were conducted to evaluate the activities for other Gram positive bacteria, *L. monocytogenes* (Figure 31), *E. faecalis* (Figure 32), and *S. pneumoniae* (Figure 34). A gentamicin potentiating effect in the presence of Compound 82 was observed in, *L. monocytogenes, E. faecalis* and *S. pneumoniae.* Furthermore, Compound 82 also demonstrated the synergistic effect with Gram negative bacteria, *E. coli* (Figure 33). Compound 82 can also mediate virulence suppression on other Gram negative bacteria, by repressing the pigment formation in *P. aeruginosa* and *B. cepacia. Finally,* Compound 82 (Figure 35) retained its potentiating effect against *S*. *aureus* in the presence of neamine.

### Discussion

Previously, through a high-throughput screening platform, Applicants successfully isolated a small molecule, nucleozin, which targets the influenza A nucleoprotein (Kao et al., Nature biotechnology, 28(6):600-605 (2010)). The same library of structurally diverse small molecule compounds was screened. By performing the background screening (screening with BHI medium, without antibiotics) at a final concentration of 20µg/mL, small molecule compounds with direct bacterial inhibitions were excluded from the screening. Screenings with the presence of sub MIC antibiotics allow the identification of small molecules with a potential bacterial growth inhibition effect in combination with antibiotics. The top 10% of compounds showing a combinatorial effect with antibiotics were selected for further studies. Dual concentration secondary screens were conducted; compounds were added at a final concentration of 100µg/mL and 10µg/mL. This method refined the screening result by reducing false positives from the primary screening, as well as fortifying the reproducibility of success.

The top compound from the secondary screen was subjected to further analysis. MIC of the compound (compound 13) in Mu3 was first characterized. The concentration tested range was from 500µM to 0.98µM. MICs higher than 500µM were not determined. Followed by the MIC determination, the checkerboard assay of compound 13 against different aminoglycoside was performed to assess the aminoglycoside combinatory effect. The Fractional inhibitory concentration (FIC) index was normally used to assess the combinatory effect (Meletiadis et al., Antimicrobial agents and chemotherapy, 54(2):602-609 (2010)). FIC index of less than or equal to 0.5 is considered as synergistic, but for compound's MIC higher than 500µM, which the actual MIC cannot be determined, are considered to be potentiating if the antibiotic concentration required for growth inhibition is decreased by at least 4 fold antibiotic MIC.

Through sequential screening processes, Applicants were able to narrow down a chemical compound library to a lead compound for future chemical optimization for antibiotics potentiator. As shown, compound 13 demonstrated the potentiating effect in combination with aminoglycoside, gentamicin, kanamycin, amikacin, neomycin and spectinomycin, which are commonly used in clinical settings. Furthermore, at compound concentration of around 50µM, it can reduce the antibiotic MIC below the corresponding clinical breakpoint, making an ineffective drug usable again for MRSA treatment. Neamine is represents the core structure common to all aminoglycosides being used in the clinical settings. Furthermore, neamine derivative libraries were constructed for novel aminoglycosides antibiotics screening. Compound 13 showed potentiating effect with neamine further indicated that the potentiating effect is a broad spectrum characteristic among the aminoglycoside family.

Applicant validated that other strains of *S. aureus* are exhibiting similar observation of antibiotic potentiating effect by compound 13 and gentamicin. Regardless of gentamicin sensitivity, all tested strains demonstrated the enhanced susceptibility to gentamicin in the presence of compound 13. This provides a useful antibiotic cocktail treatment for *S. aureus* infection.

Applicants identified several potent analogue compounds providing potentiating effect in combinatorial use with gentamicin in the lead compound optimization screening. Compound 13, 17, 36 showed potentiating activities for gentamicin, but is accompany by high cytotoxicity from Vero cell MTT assays. Compound 19, 20 were found to have less cytotoxicity while retaining promising potentiating effect with gentamicin. Finally compound 82 is found to exhibit low cytotoxicity as well as maintaining good level of potentiating effect with gentamicin against *S. aureus.*

Applicants expanded the effective host range of the Compound 82 to other tested species of bacteria, including Gram positive *L*. *monocytogenes*, *E. faecalis* and *S. pneumoniae*, Gram negative bacteria, *E. coli.* Applicants also demonstrated that Compound 82 could elicit virulence suppression on other Gram negative bacteria, by repressing the pigment formation in *P. aeruginosa* and *B. cepacia*, which are important for colonization in host.

Applicants also identified apart from combinatory effect with current antibiotics as a potential therapeutic option, some of the compounds by themselves can repress virulence activities. Compound 3, 48, 49 shows significant repression in *hla* promoter lux reporter assay. Applicants have demonstrated the concept of using Compound 3 as anti-virulence can reduce organs' bacterial loads in *In vivo* mice model, indicating the compounds analogues themselves can be a therapeutic method for bacterial infection. Analogues of Compound 3 like Compound 87 also demonstrate the *hla* promoter lux repression, suggesting the anti-virulence property of the compound.

Applicants demonstrate the success of using HTS to isolate small molecule compound having potentiating effect with aminoglycoside against bacteria. The family of compound could also by themselves act as anti-virulence agent in reducing infectivity of bacteria. The compounds are useful for downstream chemical optimizations to increase the combinatory effect with antibiotics and enhance the anti-virulence property, while providing a reduction in cytotoxicity. The biological activities of the disclosed compounds are novel, and they provide commercial applications for potentiating the activities of commonly used antibiotics along with anti-virulence use.

## Claims

1. A composition for use in treating a bacterial infection in a subject comprising a pharmaceutically acceptable carrier and an effective amount of a compound represented by any one of the following formulae, or a pharmaceutically acceptable salt thereof:

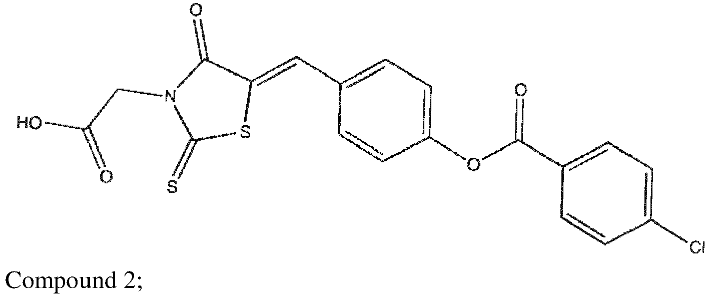

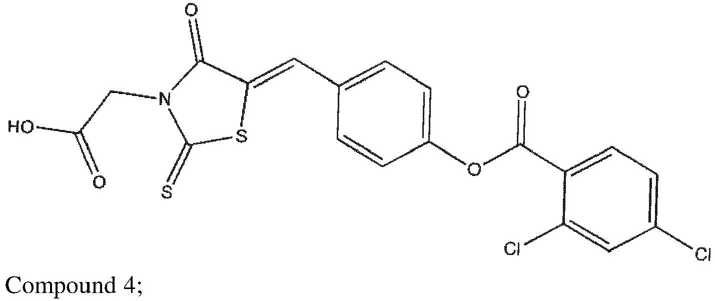

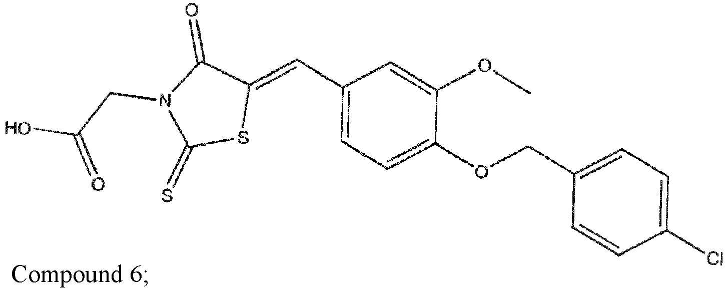

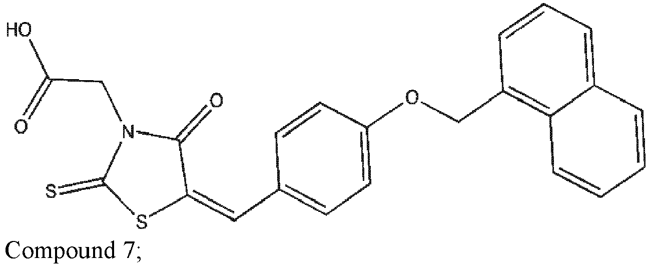

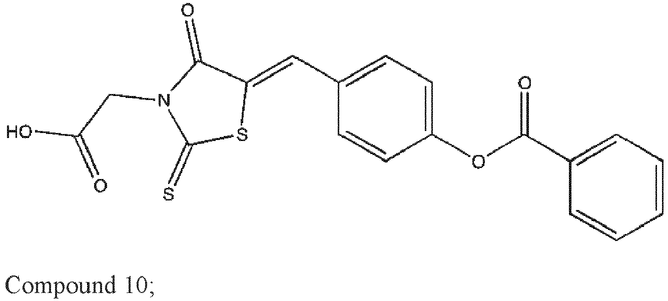

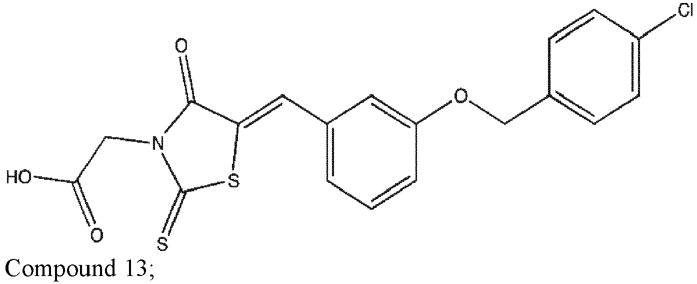

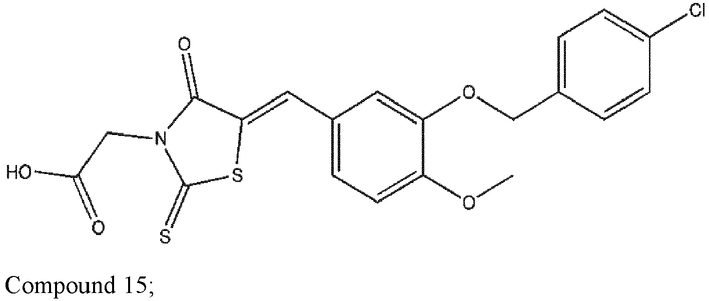

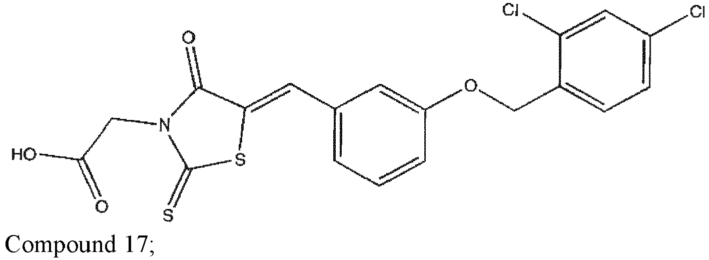

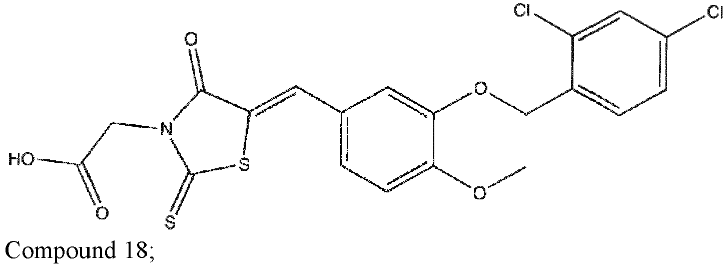

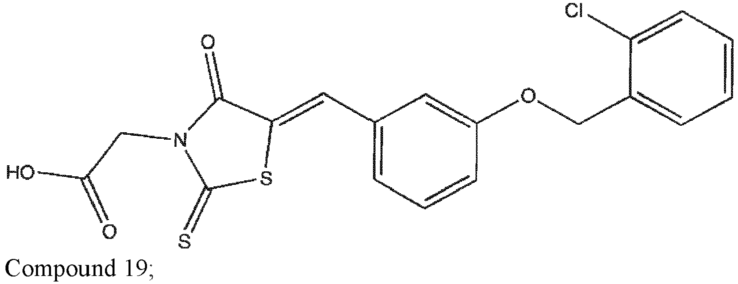

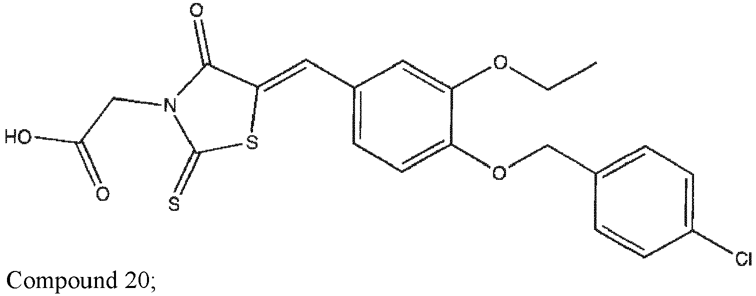

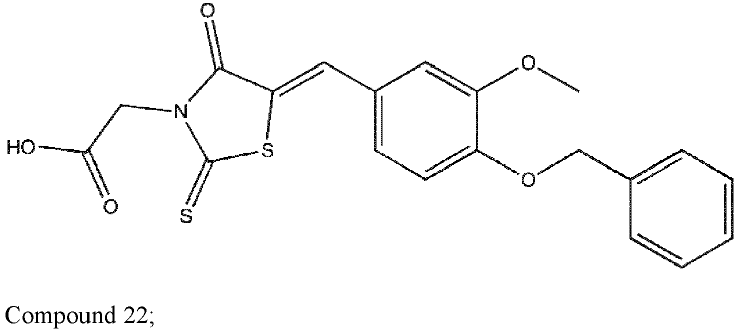

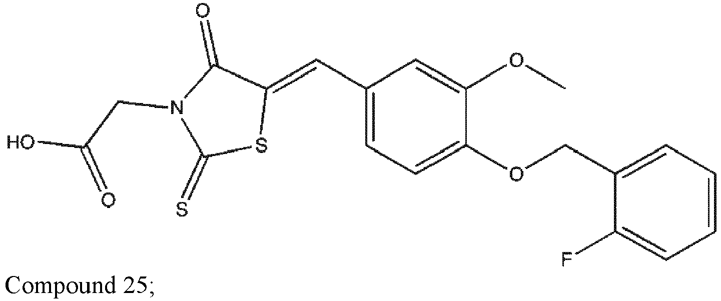

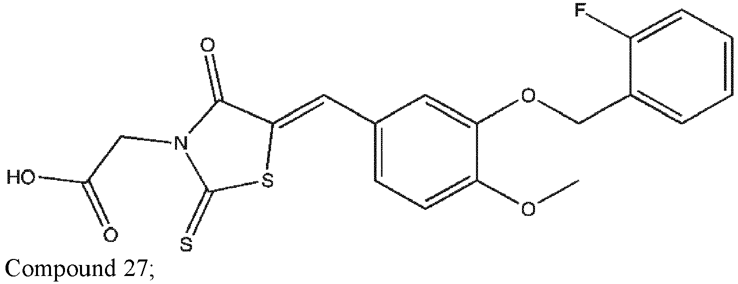

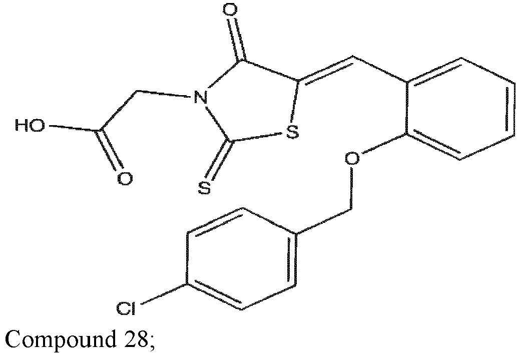

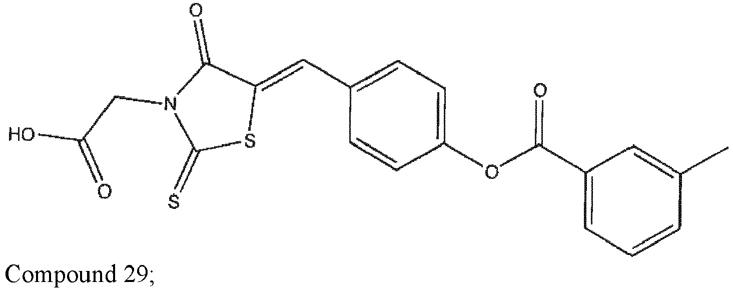

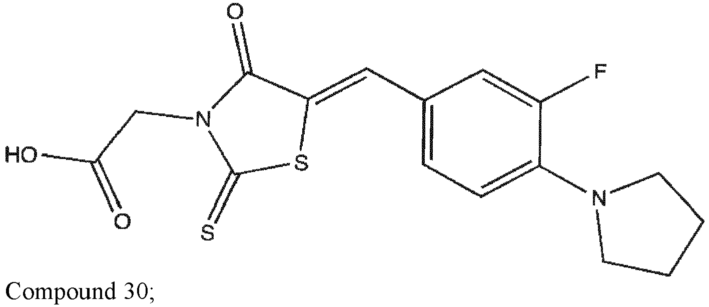

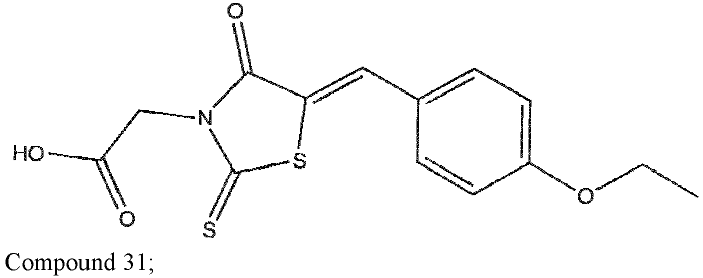

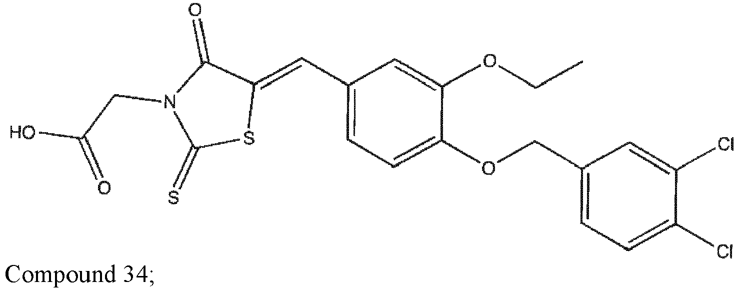

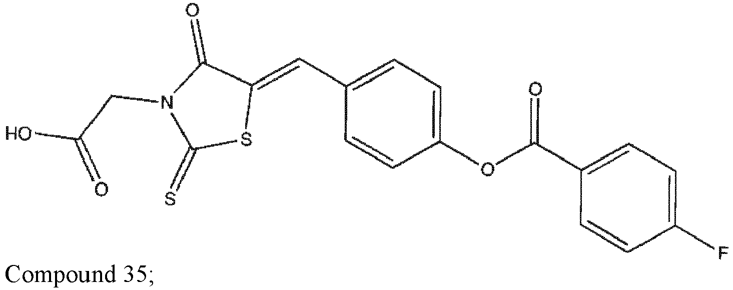

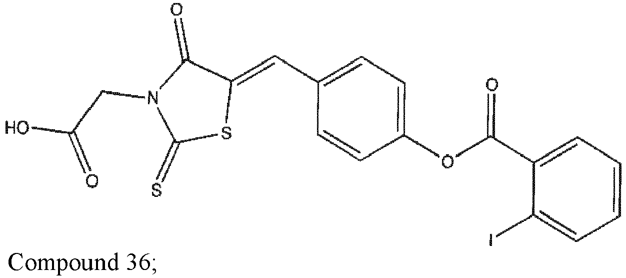

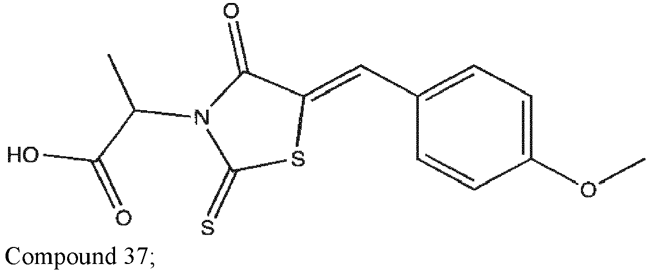

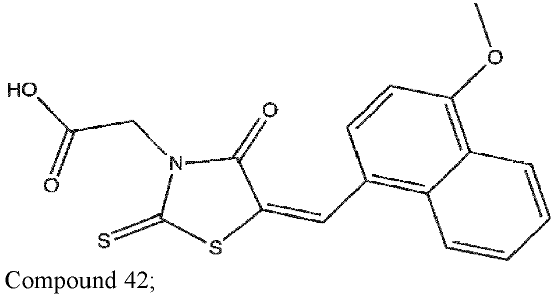

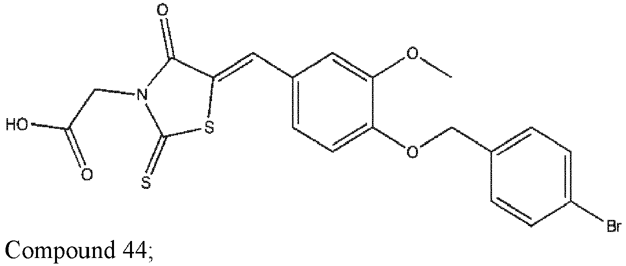

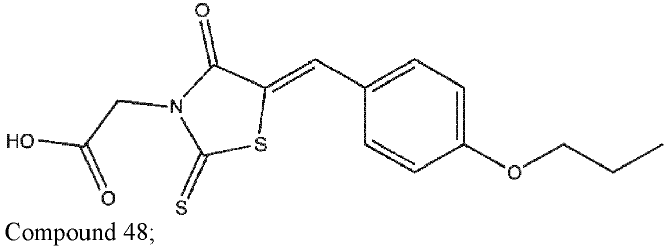

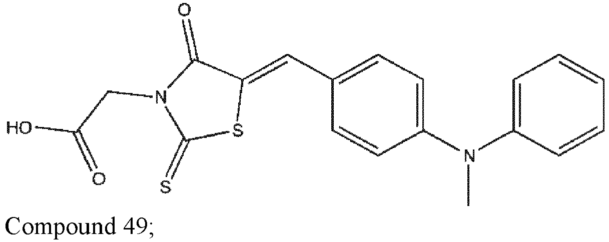

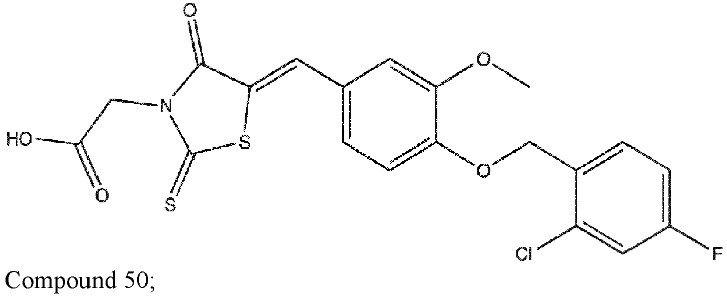

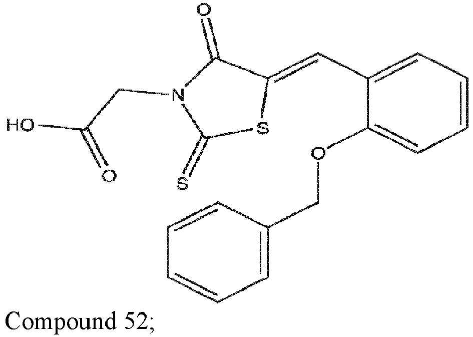

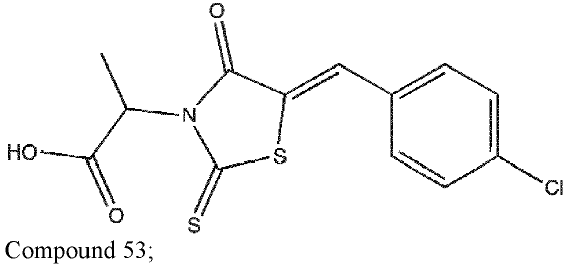

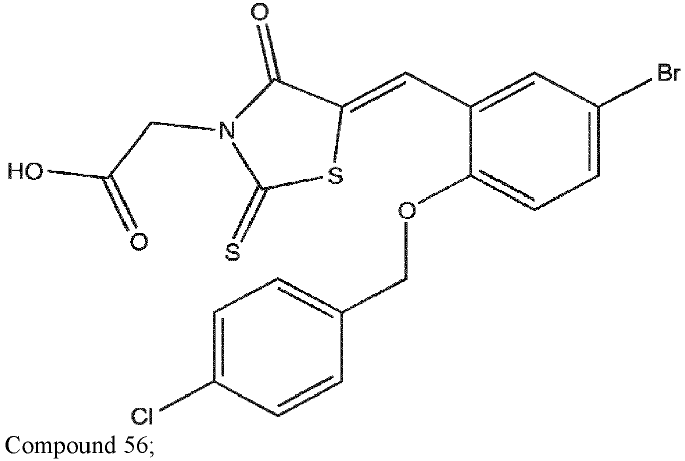

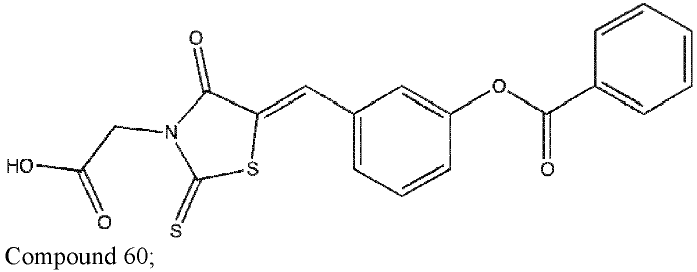

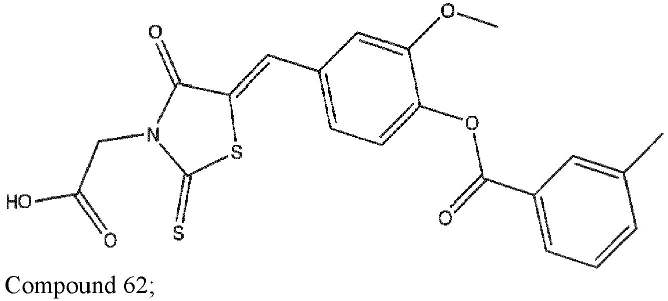

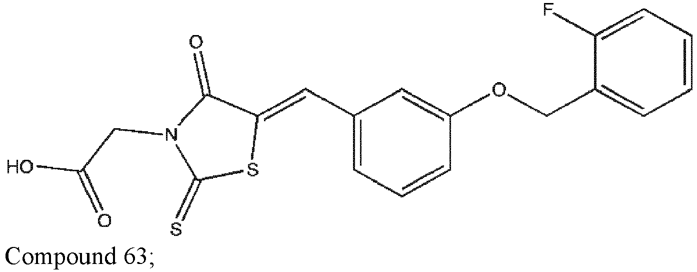

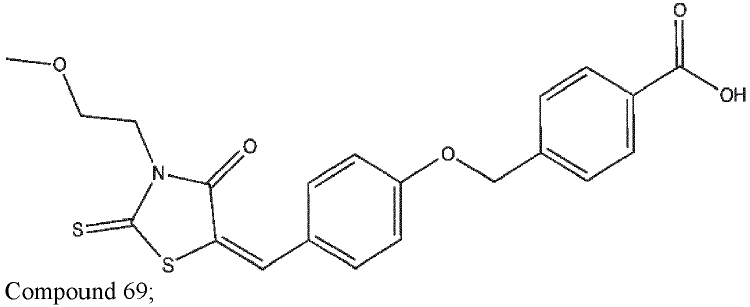

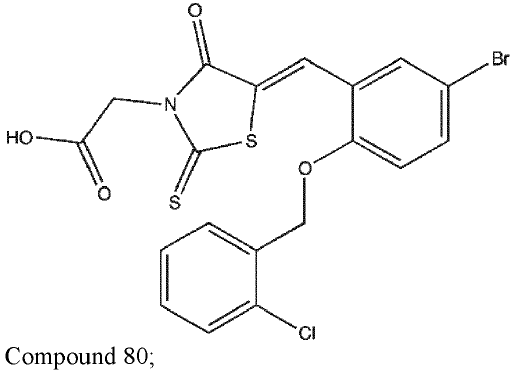

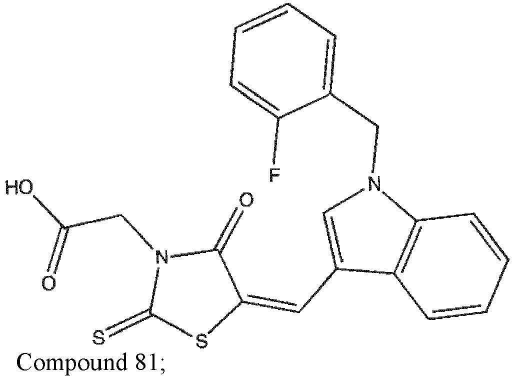

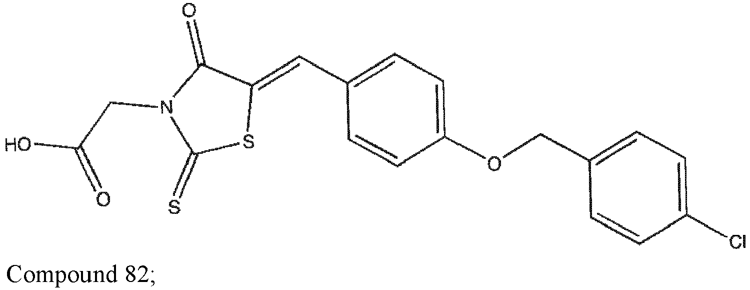

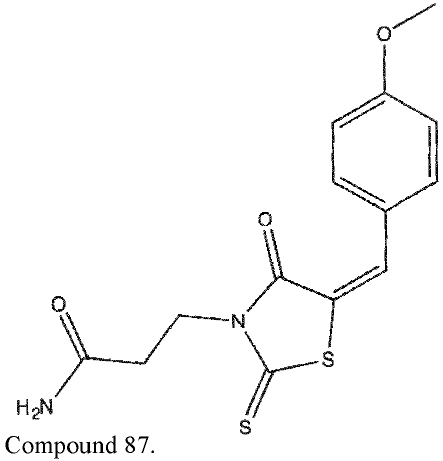

2. The composition for use according to claim 1, wherein the infection is caused by bacteria selected from the group consisting of methicillin-susceptible *Staphyloccous aureus*, *Pseudomonas aeruginosa, Listeria monocytogenes, Burkholderia cepacia, Escherichia coli*, *Enterococcus faecalis*, *Streptococcus pneumoniae*, and combinations thereof.

3. The composition for use according to claim 1 or claim 2, wherein the composition is administered parenterally or orally.

4. The composition for use according to claim 1, wherein the infection is caused by an antibiotic-resistant microorganism selected from the group consisting of a *Streptococcus pneumoniae*, *Campylobacter*, *Neisseria gonorrhoeae*, *Salmonella* (including drug-resistant non-typhoidal *Salmonella* and drug-resistant *Salmonella* serotype typhi), *Shigella*, Vancomycin-resistant *Enterococcus* (VRE), Vancomycin-resistant *Staphylococcus aureus* (VRSA), Erythromycin-resistant Group A *Streptococcus,* Clindamycin-resistant Group B *Streptococcus,* Carbapenem-resistant *Enterobacteriaceae* (CRE), drug-resistant tuberculosis, Extended spectrum *Enterobacteriaceae* (ESBL), multidrug-resistant *Acinetobacter* (including MRAB), *Clostridium difficile*, *Enteropathogenic E. coli* (EPEC), *Pseudomonas aeruginosa*, *H. pylori*, *Streptococcus anginosus* and *Uropathogenic E. coli* (UPEC) and Methicillin-resistant *Staphylococcus aureus* (MRSA).

5. The composition for use according to claim 1, wherein the infection is caused by a multidrug-resistant strain of *Listeria monocytogenes*, *Pseudomonas aeruginosa*, *Burkholderia cepacia*, *Enterococcus faecalis* and *Streptococcus pneumoniae.*

6. The composition for use according to any one of claims 1 to 5, wherein the infection is selected from the group consisting of impetigo, boils, abscesses, folliculitis, cellulitis, necrotizing fasciitis, pyomyositis, surgical/traumatic wound infection, infected ulcers and burns, osteomyelitis, device-related osteoarticular infections, impetigo, secondarily infected skin lesions, meningitis, brain abscess, subdural empyema, and spinal epidural abscess.

7. The composition for use according to any one of claims 1 to 5, wherein the infection is a urinary tract infection, and/or infections of the skin and soft tissue, bone and joint and infections affecting lung and heart valves.

8. The composition for use according to any one of claims 1 to 7, wherein the subject is hospitalized or immunocompromised.

9. The composition for use according to any one of claims 1 to 8, further comprising a therapeutically effective amount of an antibiotic.

10. The composition for use according to claim 9, wherein the antibiotic is selected from the group consisting of gentamicin, amikacin, kanamycin, neomycin, spectinomycin, neamine and combinations thereof.

11. The composition for use according to claim 9 or claim 10, wherein the therapeutically effective amount of the antibiotic is less than a therapeutically effective or therapeutically optimal dose of the antibiotic when administered to a subject in the absence of the composition.

12. The composition for use according to any one of claims 1 to 8, wherein the composition is co-administered with an antibiotic in an amount effective to increase the efficacy of the antibiotic, wherein the antibiotic is selected from the group consisting of gentamicin, amikacin, kanamycin, neomycin, spectinomycin, neamine and combinations thereof.

13. The composition for use according to claim 12, wherein the composition is administered separately, simultaneously, or sequentially with the antibiotic.

14. A compound for use in reducing virulence of bacteria in a subject in need thereof, wherein the bacteria is selected from the group consisting of methicillin-susceptible *Staphyloccous aureus, Pseudomonas aeruginosa, Listeria monocytogenes, Burkholderia cepacia*, *Escherichia coli*, *Enterococcus faecalis*, *Streptococcus pneumoniae* and combinations thereof, and wherein the compound is represented by one of the following formulae, or a pharmaceutically acceptable salt thereof:

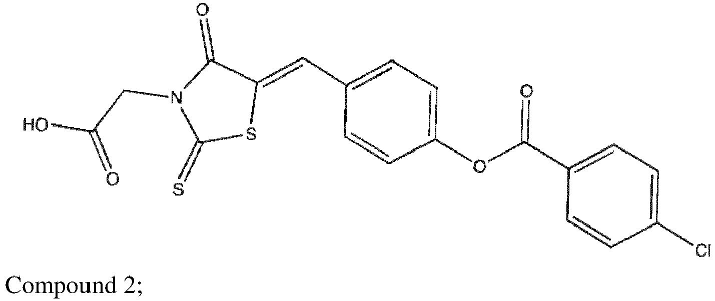

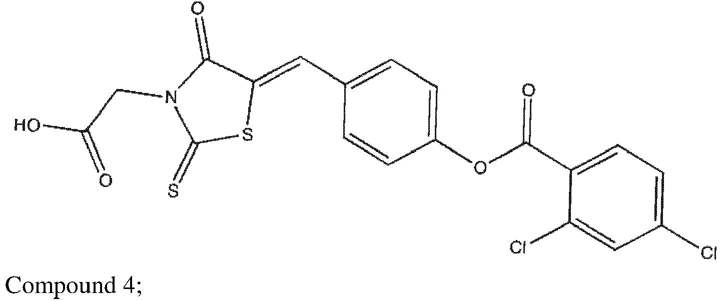

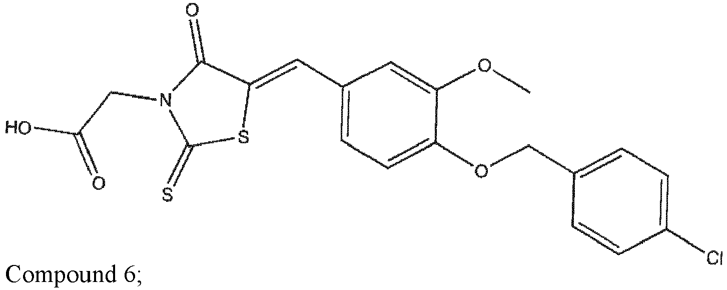

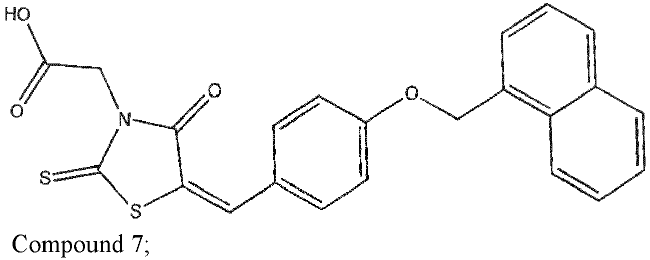

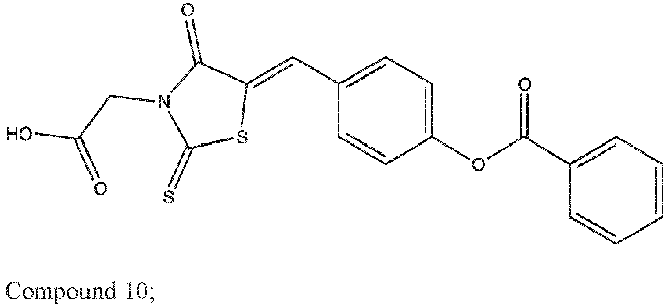

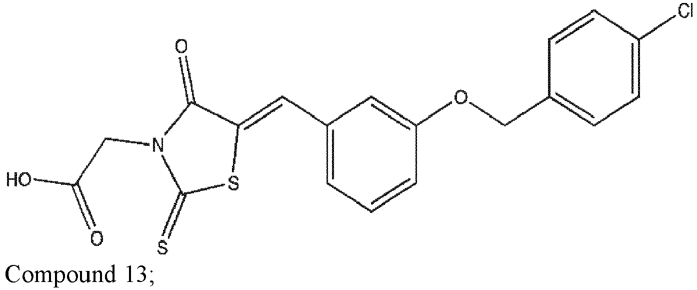

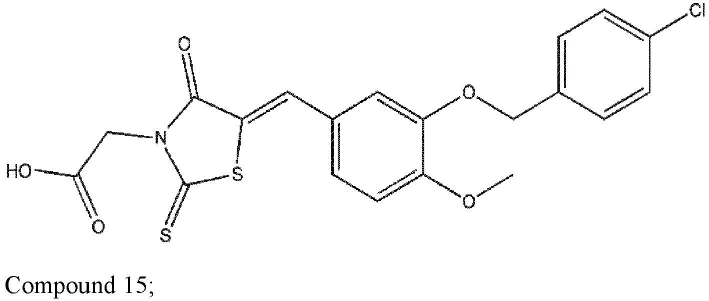

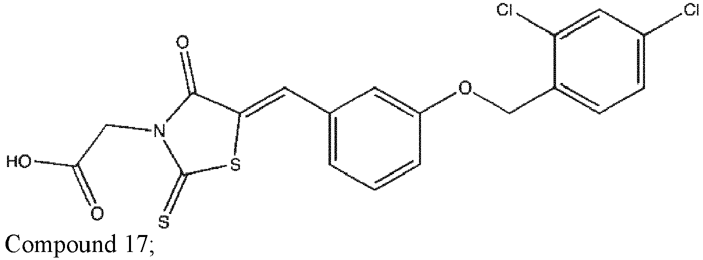

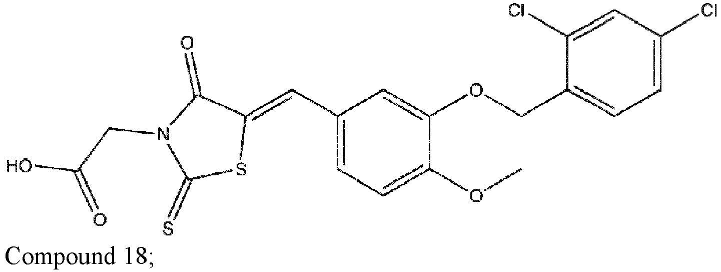

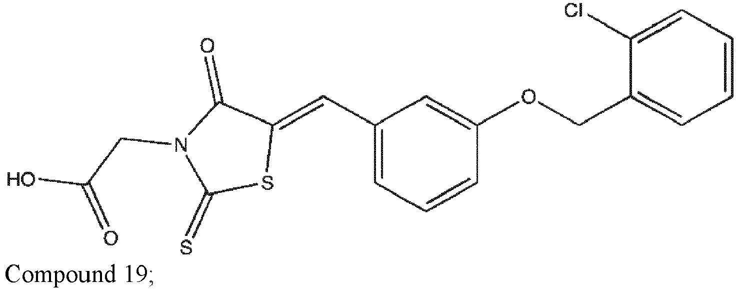

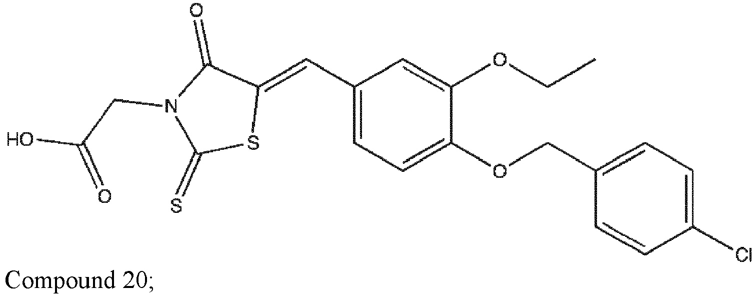

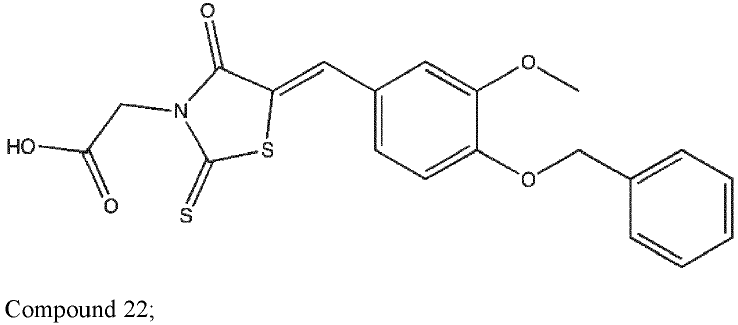

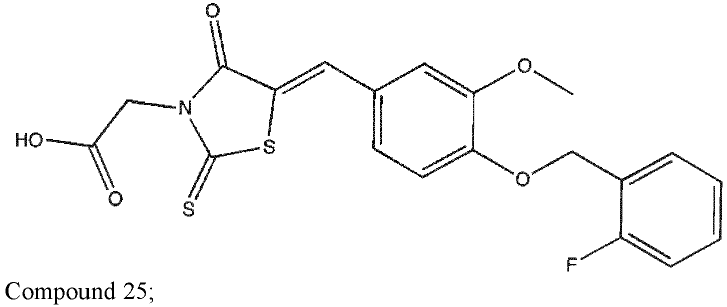

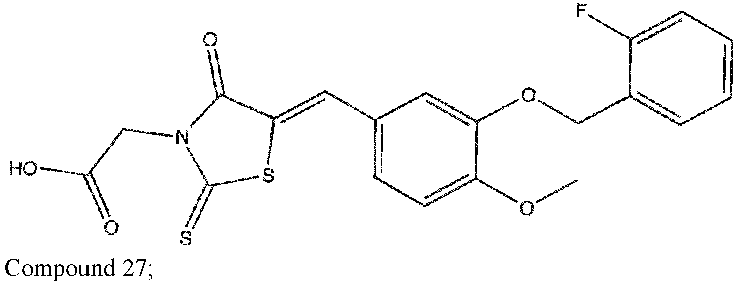

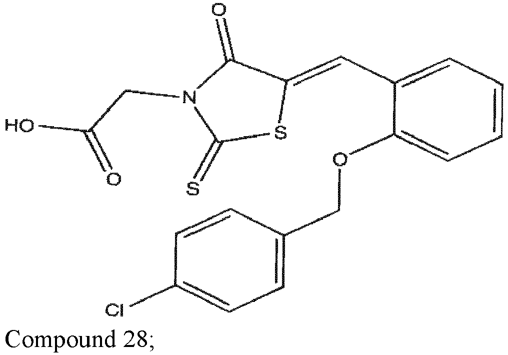

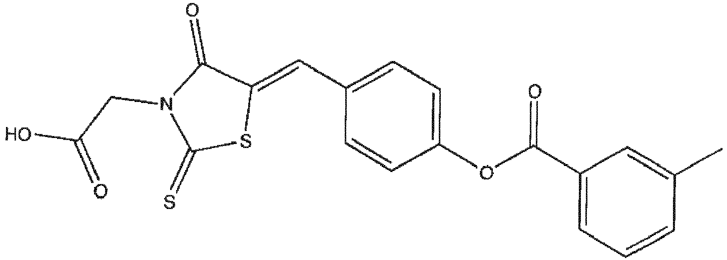

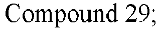

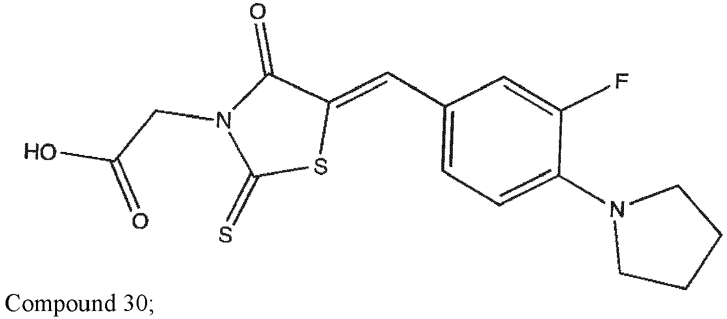

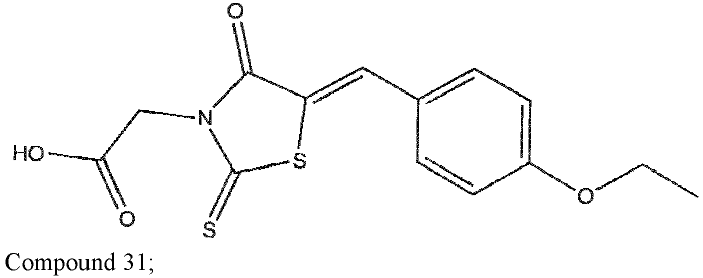

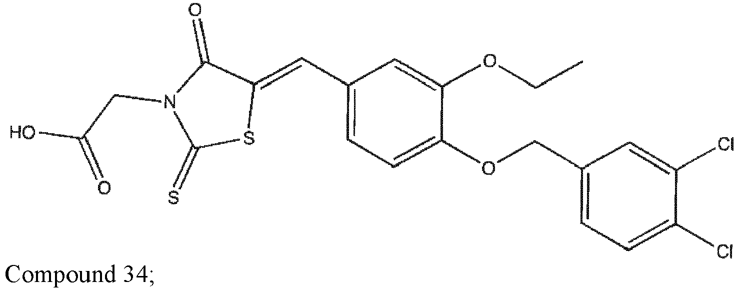

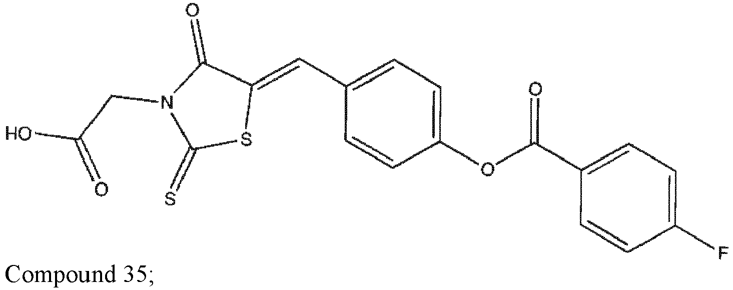

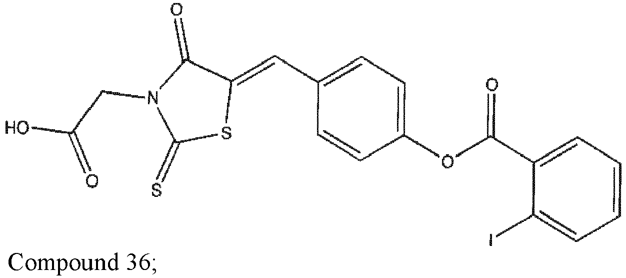

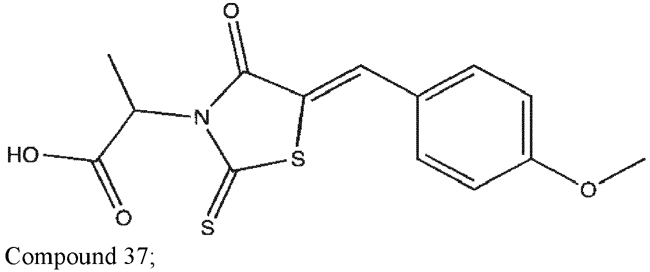

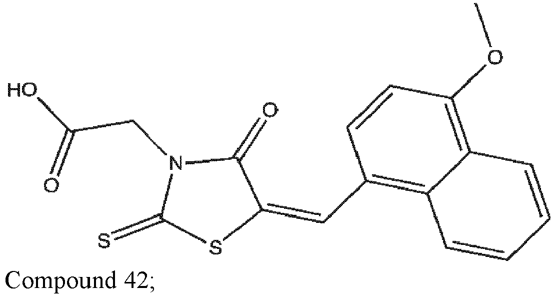

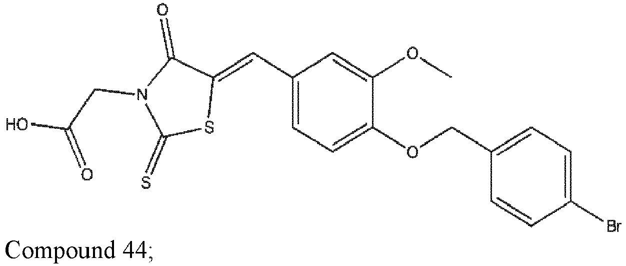

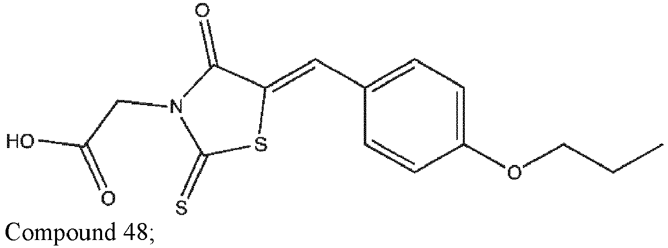

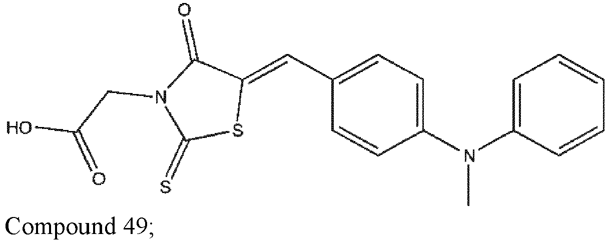

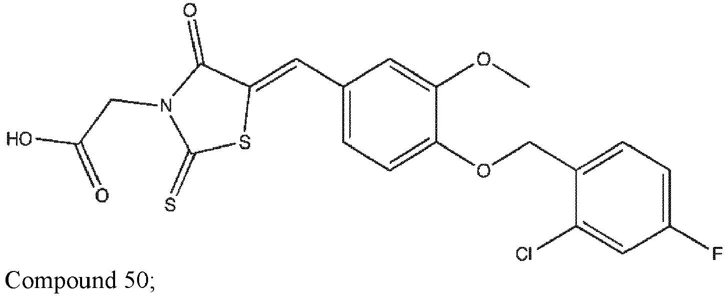

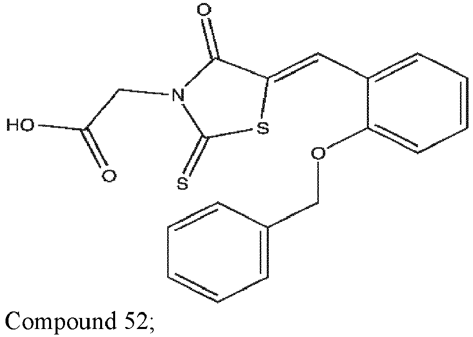

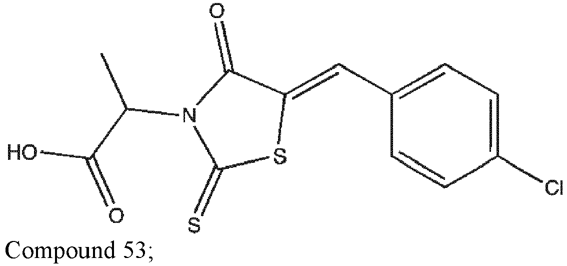

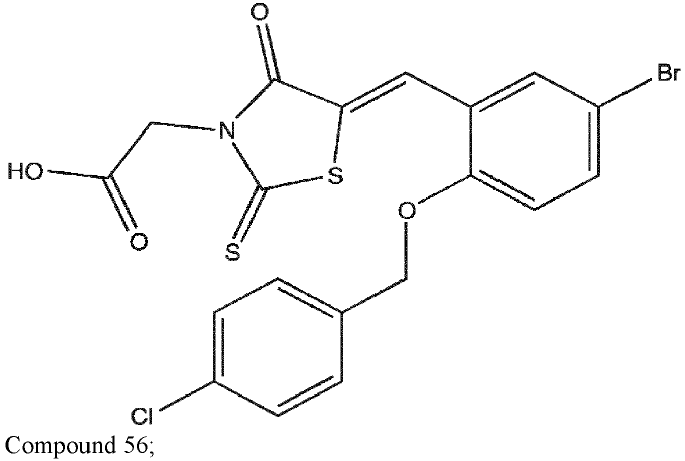

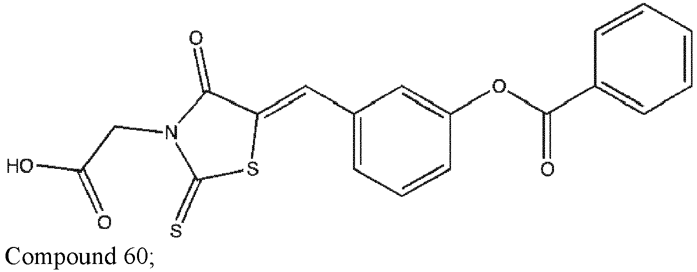

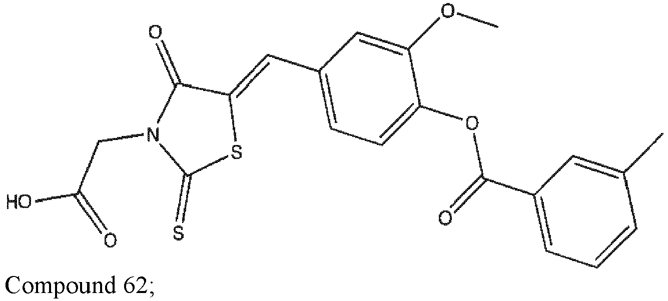

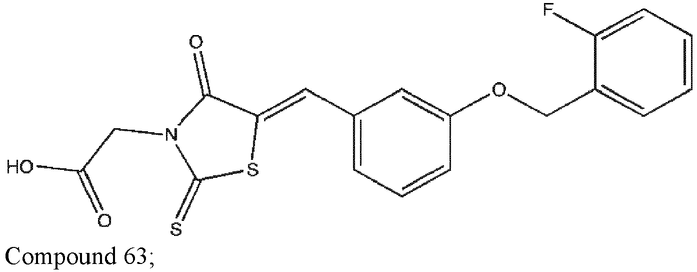

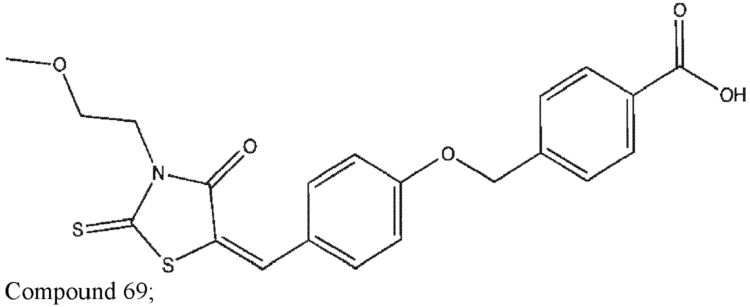

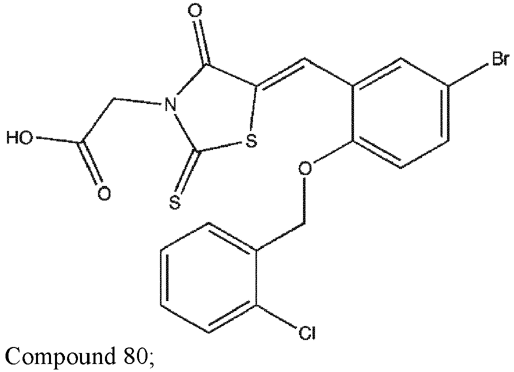

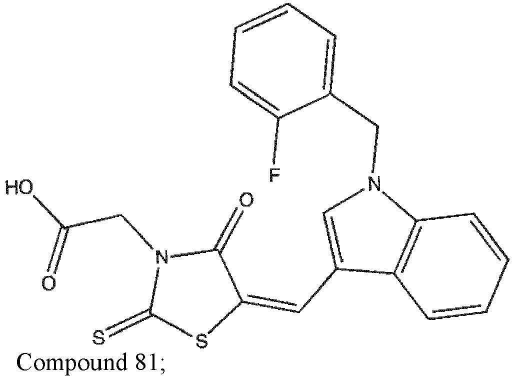

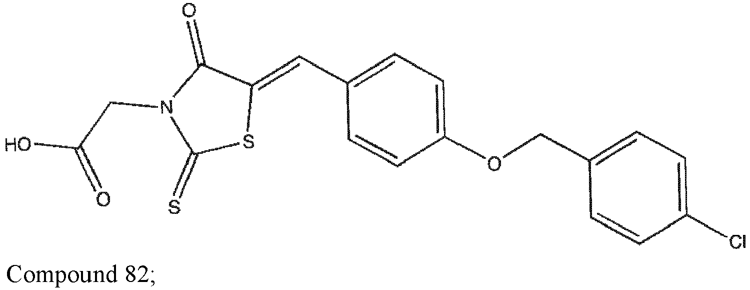

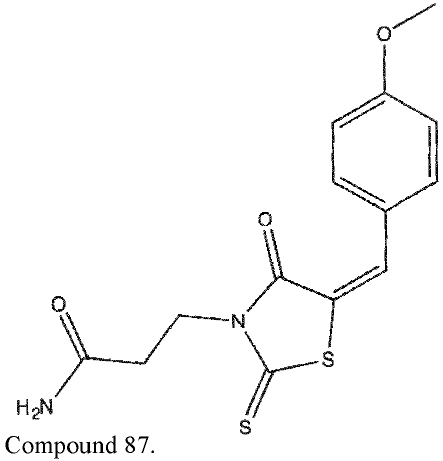

## Patentansprüche

1. Eine Zusammensetzung für die Anwendung bei der Behandlung einer bakteriellen Infektion bei einem Individuum, das einen pharmazeutisch, akzeptierbaren Träger und eine effektive Menge einer Verbindung umfasst, die durch irgendeine folgender Formeln dargestellt ist, oder ein pharmazeutisch akzeptierbares Salz derselben:

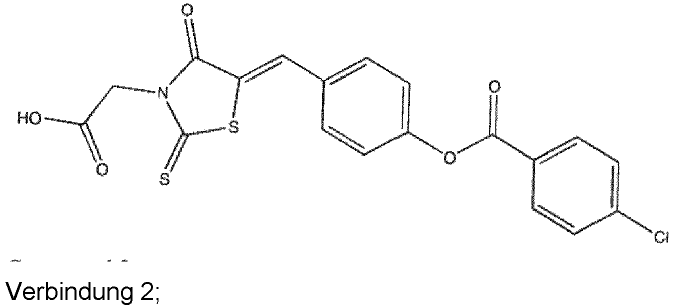

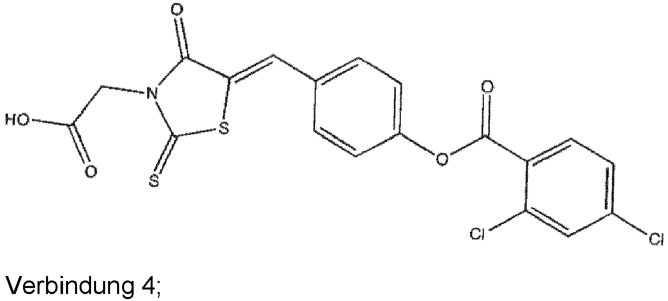

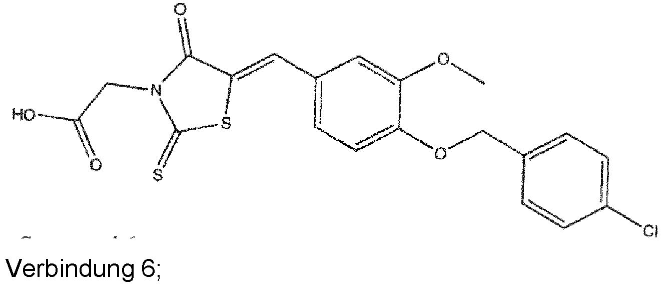

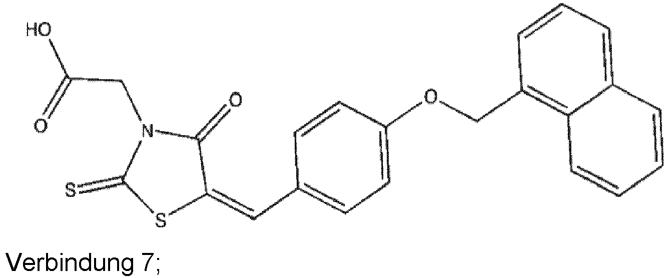

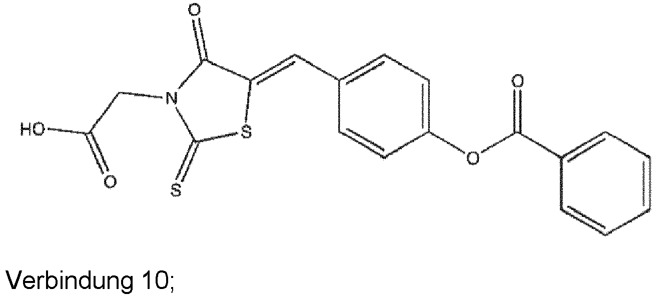

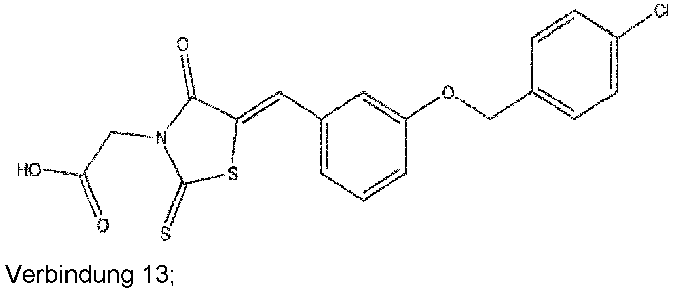

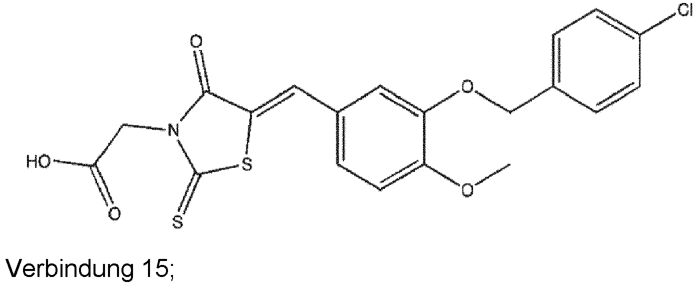

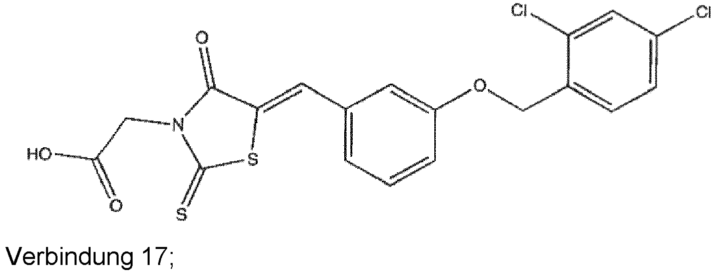

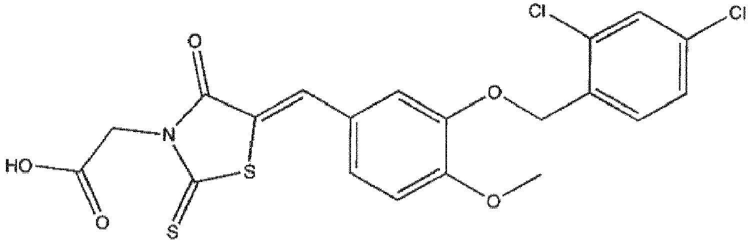

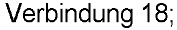

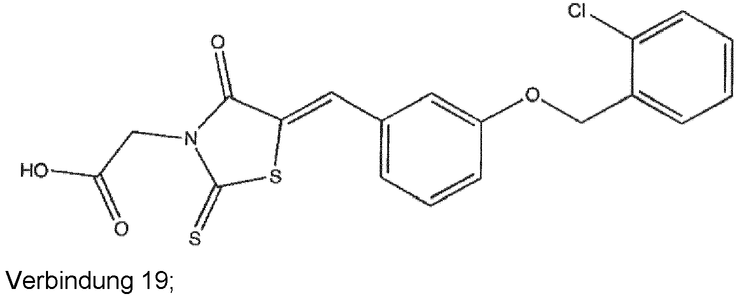

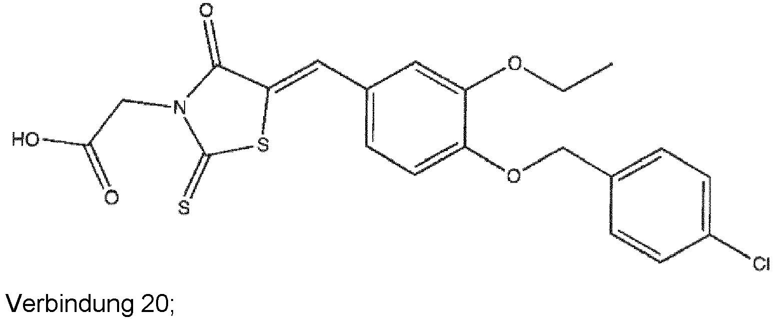

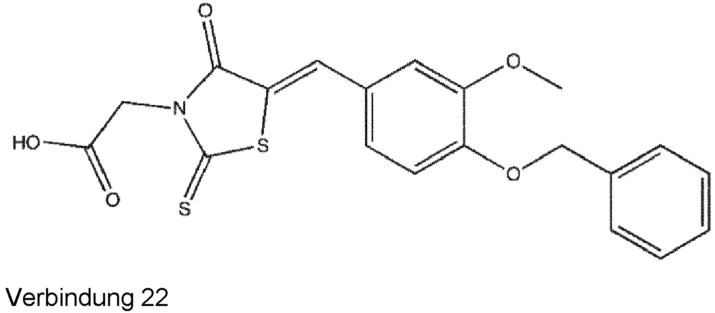

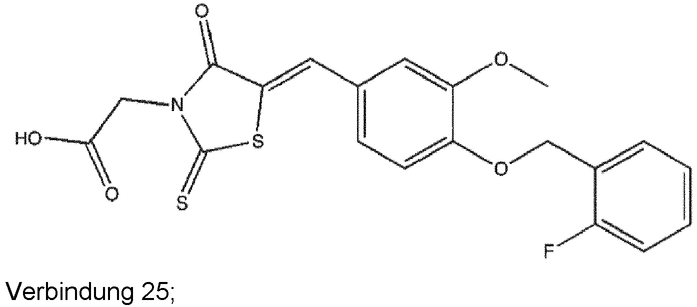

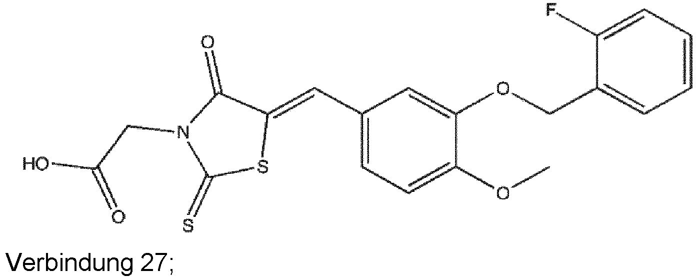

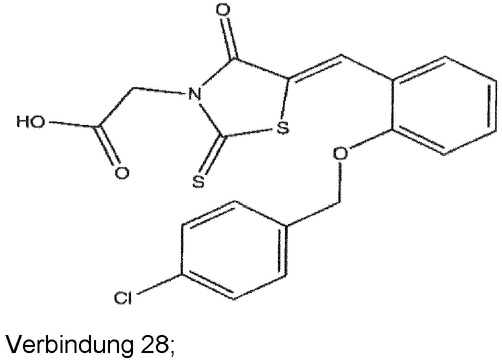

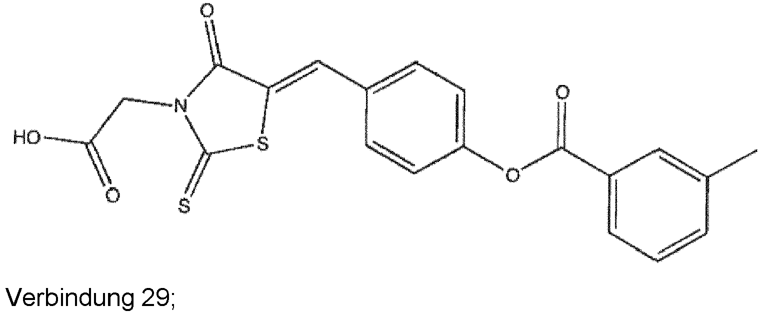

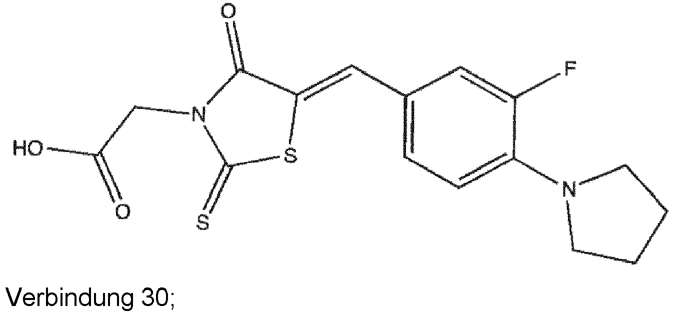

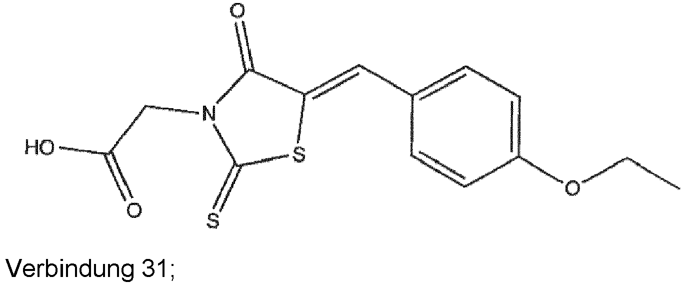

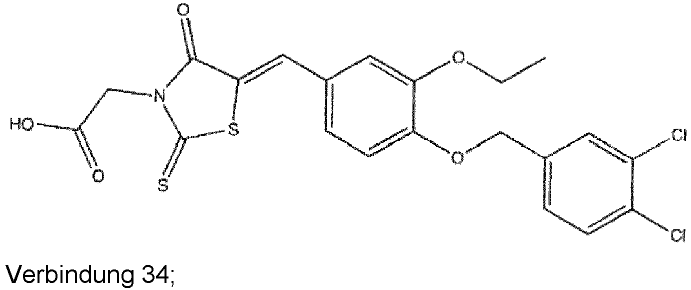

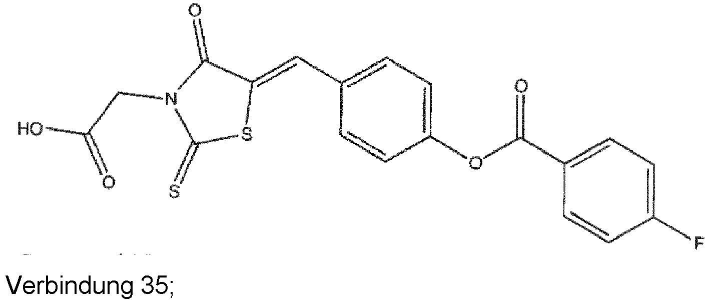

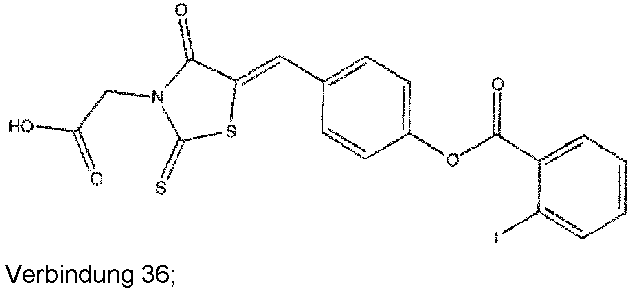

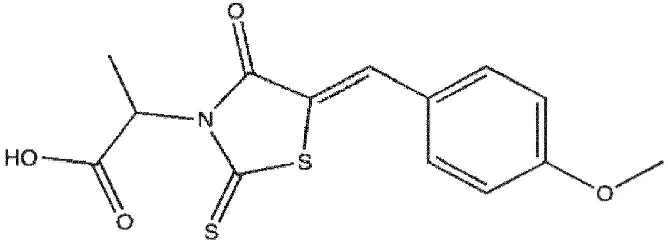

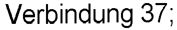

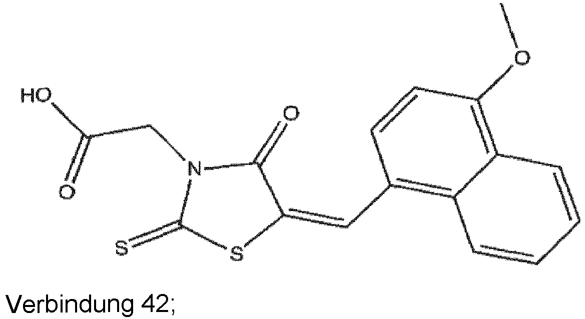

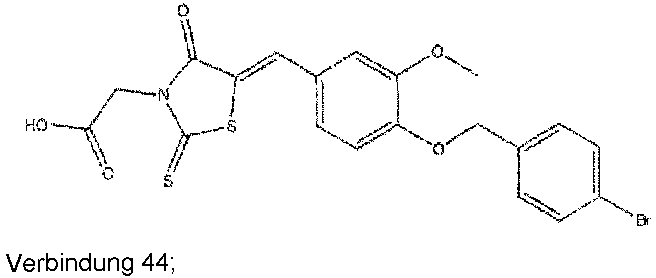

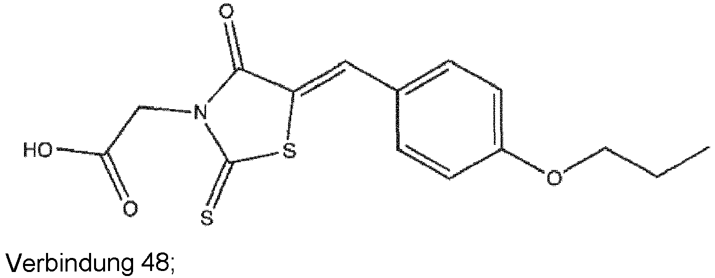

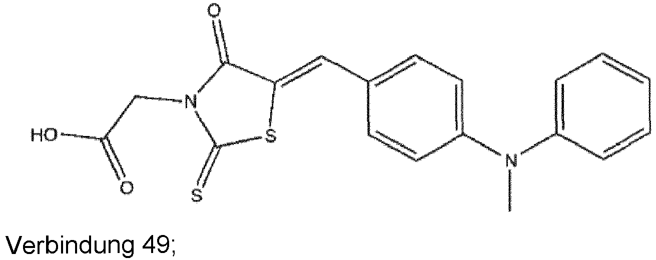

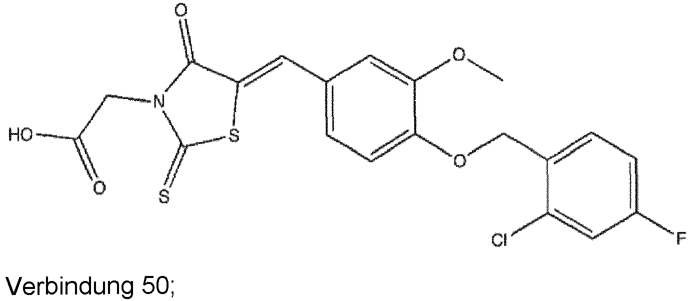

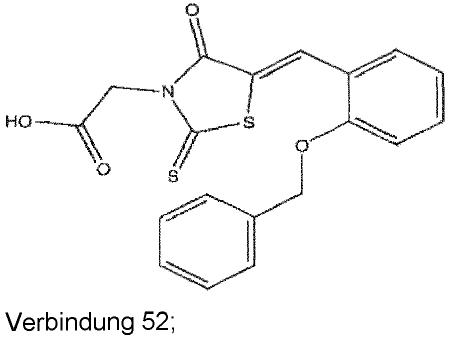

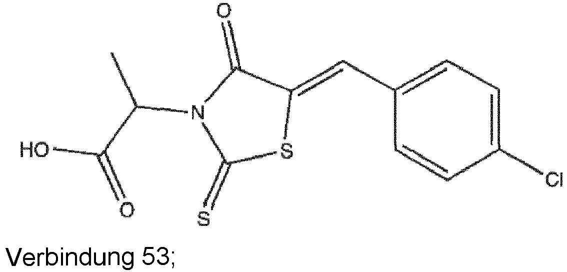

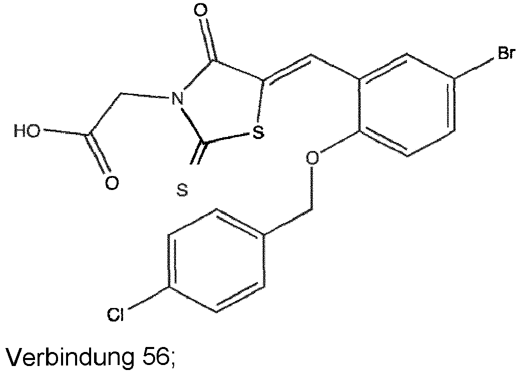

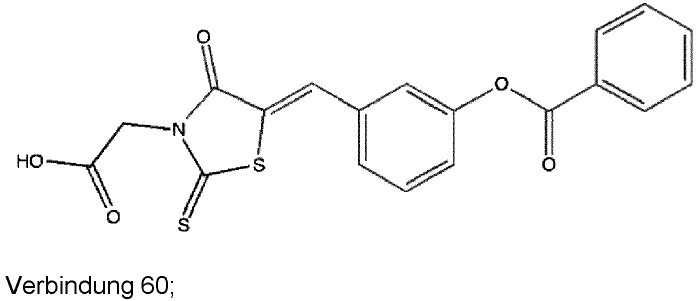

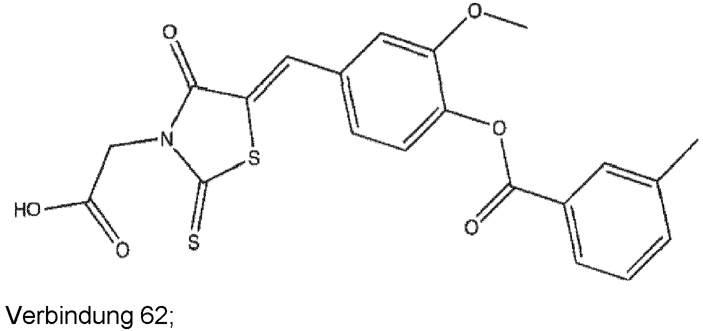

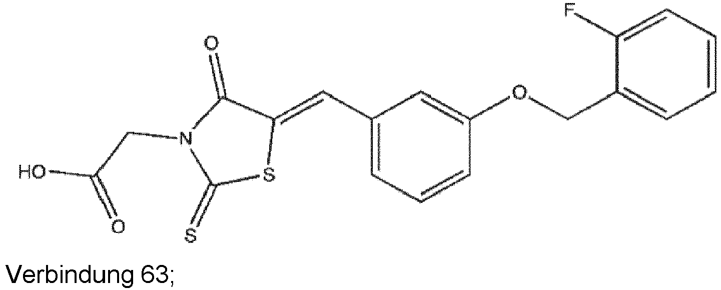

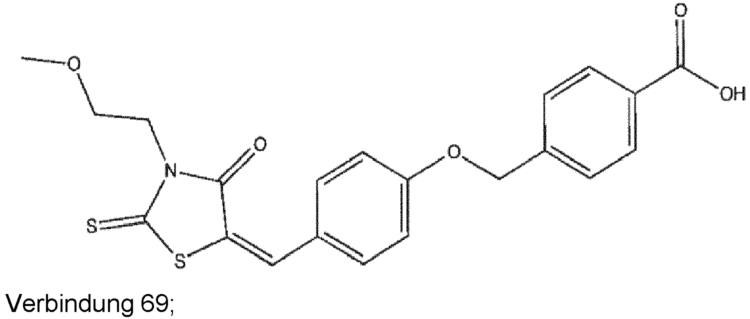

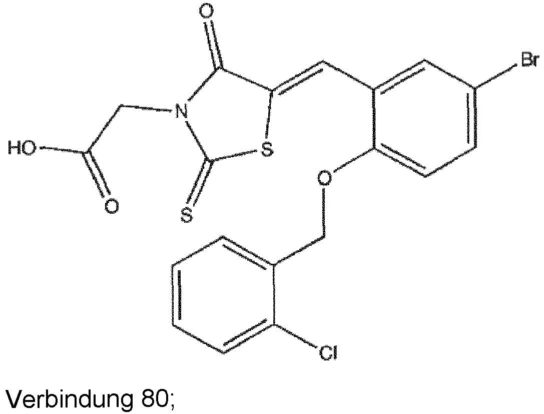

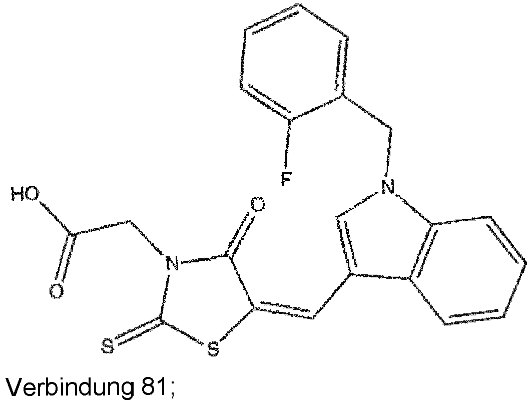

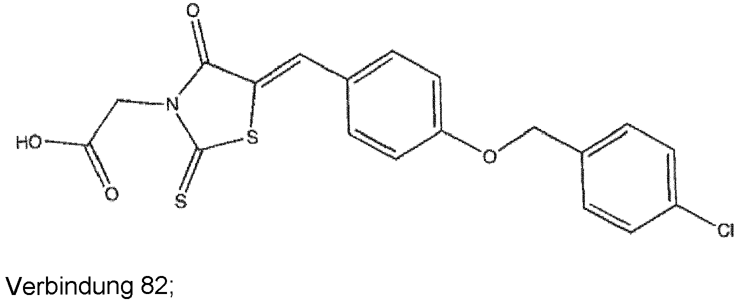

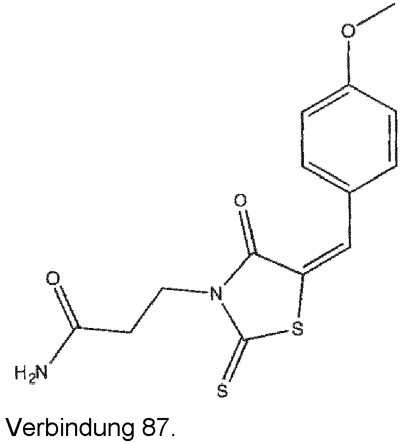

2. Die Zusammensetzung für die Anwendung gemäss Anspruch 1, wobei die Infektion durch Bakterien aus folgender gegen Methicillin empfindlichen Gruppe verursacht wurde: *Staphyloccous aureus*, *Pseudomonas aeruginosa*, *Listeria monocytogenes, Burkholderia cepacia, Escherichiacoli, Enterococcusfaecalis, Streptococcus pneumoniae* und Kombinationen derselben.

3. Die Zusammensetzung für die Anwendung gemäss Anspruch 1 oder Anspruch 2, wobei die Zusammensetzung parental oder oral verabreicht wird.

4. Die Zusammensetzung für die Anwendung gemäss Anspruch 1, wobei die Infektion durch einen gegen Antibiotika resistenten Mikroorganismus aus folgender Gruppe verursacht wurde: *Streptococcuspneumoniae*, *Campylobacter*, *Neisseria gonorrhoeae*, *Salmonella* (einschliesslich nicht typhoidale, gegen Medikamente resistente *Salmonella* und gegen Medikamente resistente *Salmonella* (Serotype Typhi), *Shigella*, vancomycin-resistenter *Enterococcus* (VRE), vancomycin-resistenter *Staphylococcus aureus* (VRSA), erythromycin-resistente Gruppe A *Streptococcus,* clindamycin-resistente Gruppe B *Streptococcus,* carbapenemresistente *Enterobacteriaceae* (CRE), gegen Medikamente resistente Tuberkulose, erweitertes Spektrum *Enterobacteriaceae* (ESBL), multimedikamentöse-resistente *Acinetobacter* (einschliesslich MRAB), *Clostridium difficile*, *Enteropathogenic E. coli* (EPEC) *Pseudomonas aeruginosa*, *H. pylori*, *Streptococcus anginosus* und *Uropathogenic E. coli* (UPEC) sowie methicillin-resistenter *Staphylococcus aureus* (MRSA).

5. Die Zusammensetzung für die Anwendung gemäss Anspruch 1, bei der die Infek(tion durch einen multimedikamentös resistenten Stamm von *Listeria Monocytogenes, Pseudomonas aeruginosa, Burkholderiacepacia, Enterococcus faecali* und *Streptococcus pneumoniae* verursacht wurde.

6. Die Zusammensetzung für die Anwendung übereinstimmend mit irgendeinem der Ansprüche 1 bis 5, bei der die Infektion ausgewählt wird aus folgender Gruppe: Impestigo, Furunkel, Abszesse, Folliculitis, Zellulitis, nekrotisierte Fasciitis, Pyomyositis, chirurgische/traumatische Wundentzündung, entzündete Geschwüre und Brandwunden, Osteomyelitis, gerätetechnische Knochengelenkinfektionen, Herpes pustularis, sekundäre, entzündete Hautverletzungen, Meningitis, Gehirnabszess, subdurales Empyema und epiduraler Wirbelsäulenabszess.

7. Die Zusammensetzung für die Anwendung übereinstimmend mit Anspruch 1 bis 5, bei der die Infektion eine Harnweginfektion ist, und/oder Hautinfektionen und Infektionen von Weichteilen, Knochen und Infektionen, die die Lunge und Herzklappen betreffen.

8. Die Zusammensetzung für die Anwendung übereinstimmend mit Anspruch 1 bis 7, bei der das Individuum ins Krankenhaus eingewiesen wird oder immungeschwächt ist.

9. Die Zusammensetzung für die Anwendung übereinstimmend mit Anspruch 1 bis 8, die weiter eine therapeutisch effektive Menge eines Antibiotikum enthält.

10. Die Zusammensetzung für die Anwendung gemäss Anspruch 9, bei der das Antibiotikum ausgewählt aus folgender Gruppe: Gentamicin, Amikacin, Kanamycin, Neomycin, Spectinomycin, Neamin und Kombinationen derselben.

11. Die Zusammensetzung für die Anwendung gemäss Anspruch 9 oder Anspruch 10, bei de reine therapeutisch effektive Menge des Antibiotikums geringer ist als eine therapeutisch effektive oder therapeutisch optimale Dosis des Antibiotikums, wenn dieses bei Fehlen der Zusammensetzung einem Individuum verabreicht wird.

12. Die Zusammensetzung für die Anwendung gemäss irgendeinem der Ansprüche 1 bis 8, wobei die Zusammensetzung zusammen mit einem Antibiotikum in einer Menge, die zur Erhöhung der Wirkung des Antibiotikums effektiv ist, berabreicht wird, wobei das Antibiotikum aus folgender Gruppe ausgewählt wird: Gentamicin, Amikacin, Kanamycin, Neomycin, Spectinomycin, Neamin und Kombinationen derselben.

13. Die Zusammensetzung für die Anwendung gemäss Anspruch 12, wobei die Zusammensetzung separat, simultan oder sequentiell mit dem Antibiotikum verabreicht wird.

14. Eine Zusammensetzung für die Anwendung zur Verminderung der bakteriellen Virulenz bei einem Individuum, das dies benötigt, wobei die Bakterien aus folgender Gruppe ausgewählt werden: Methicillinempfindliche *Staphyloccous aureus*, *Pseudomonas aeruginosa, Listeria monocytogenes, Burkholderiacepacia, Escherichia coli*, *Enterococcus faecalis*, *Streptococcus pneumoniae* und Kombinationen derselben, und wobei die Zusammensetzung durch eine der folgenden Formeln dargestellt wird, oder ein pharmazeutisch akzeptierbares Salz derselben:

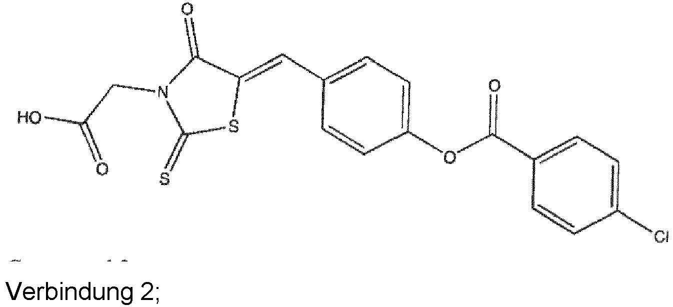

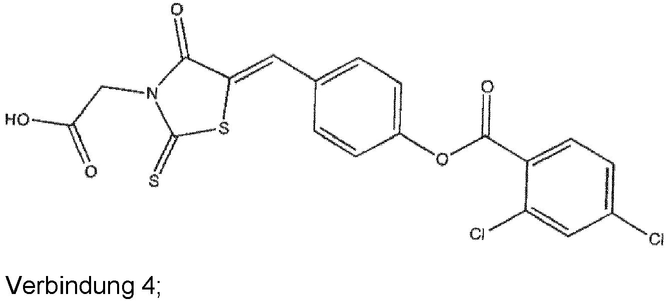

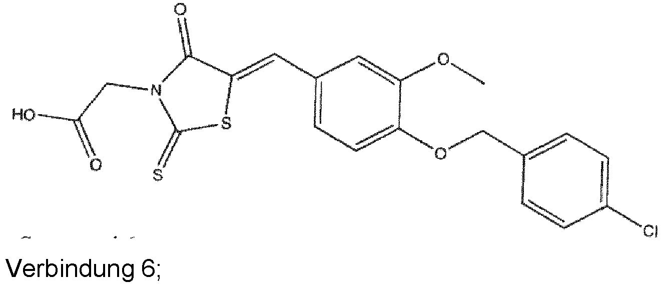

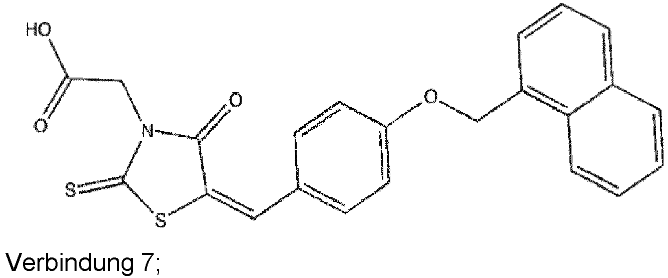

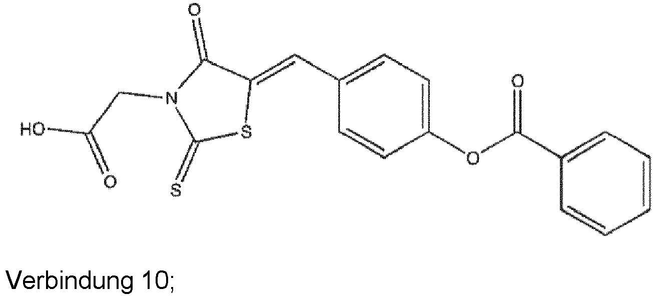

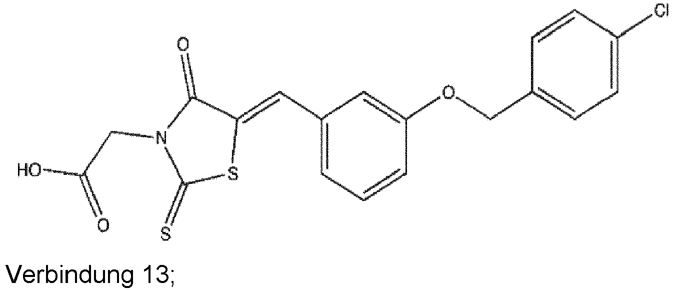

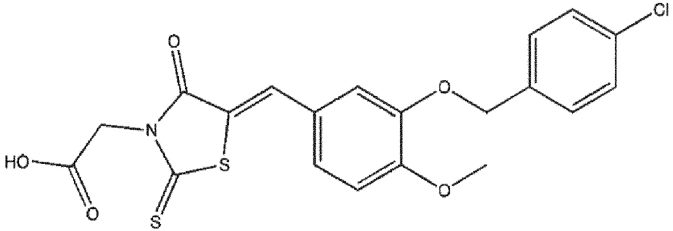

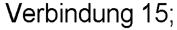

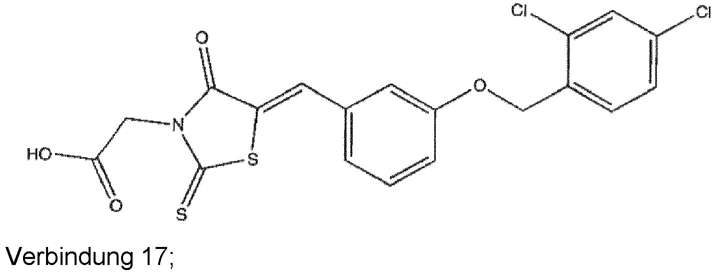

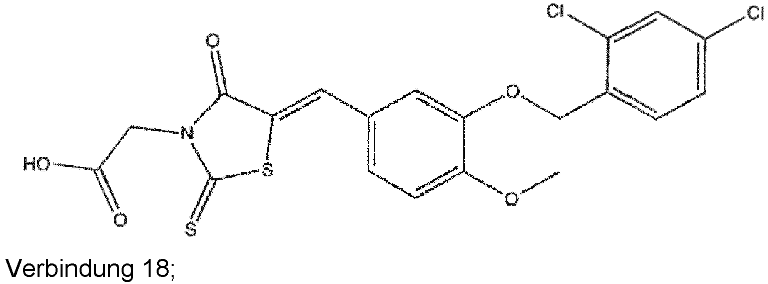

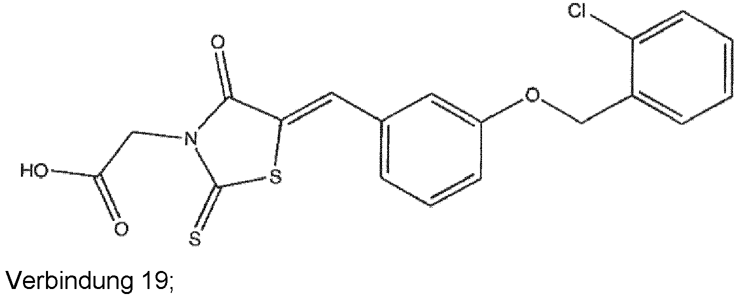

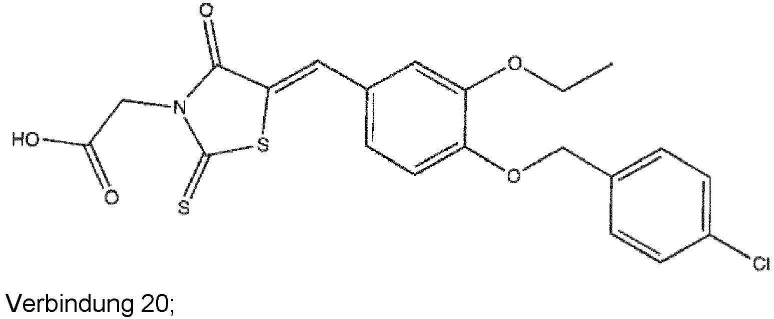

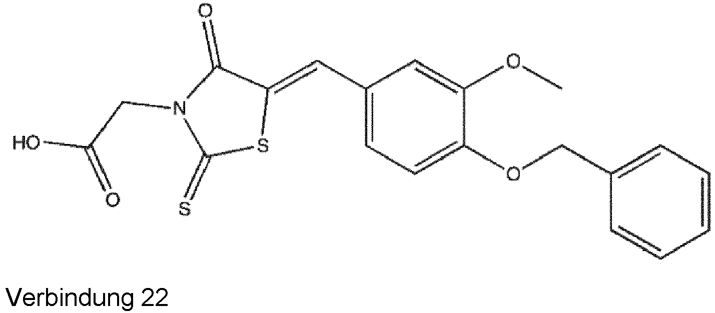

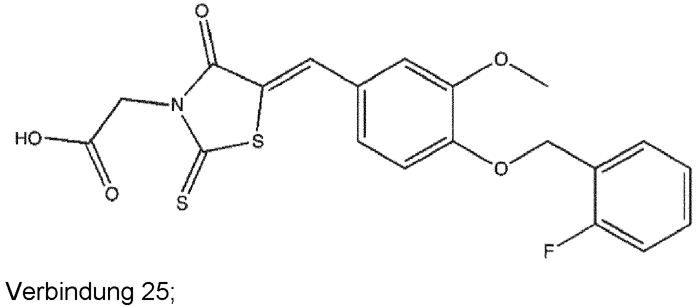

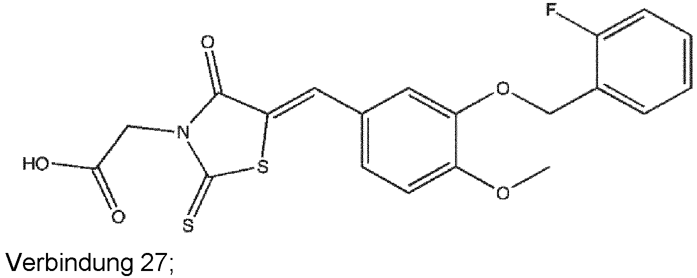

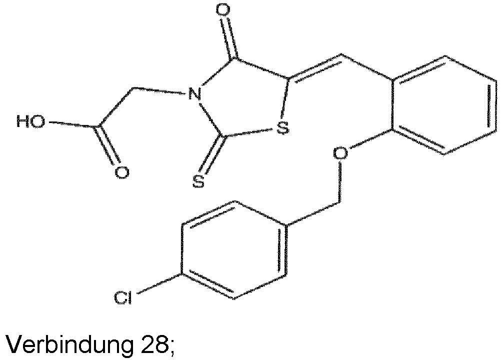

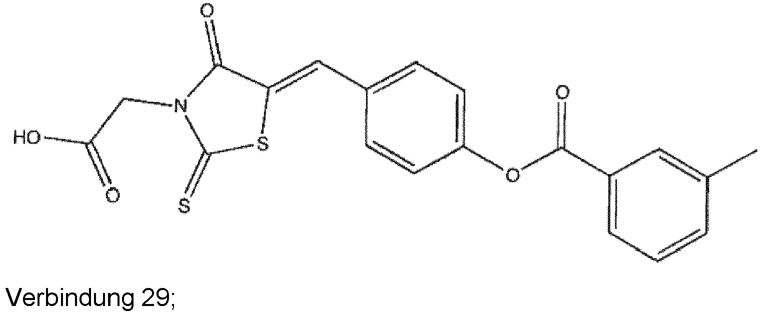

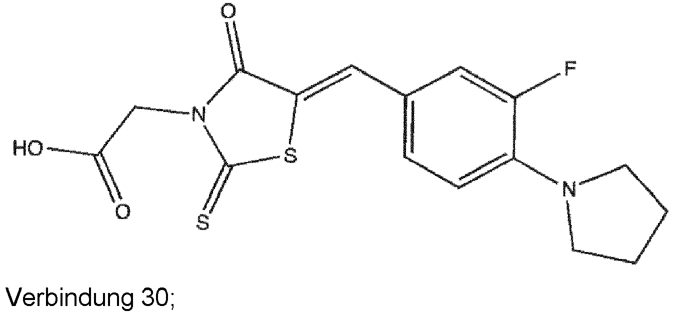

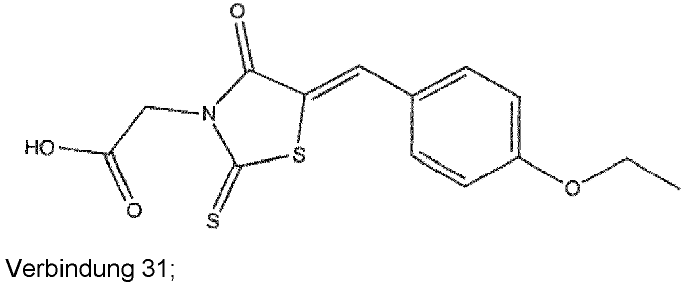

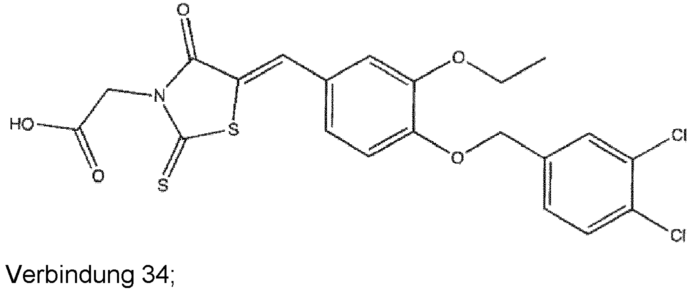

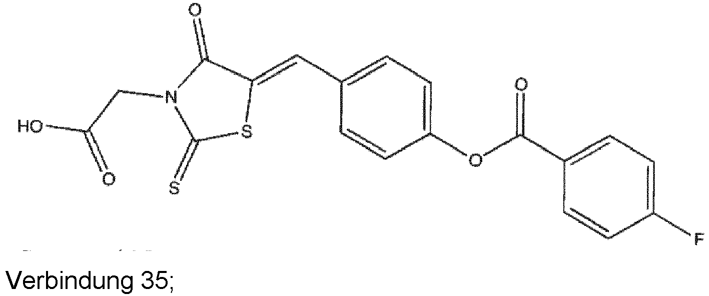

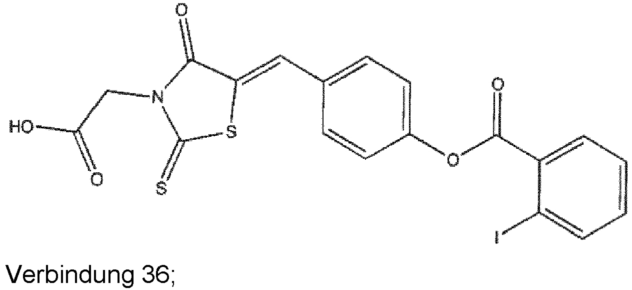

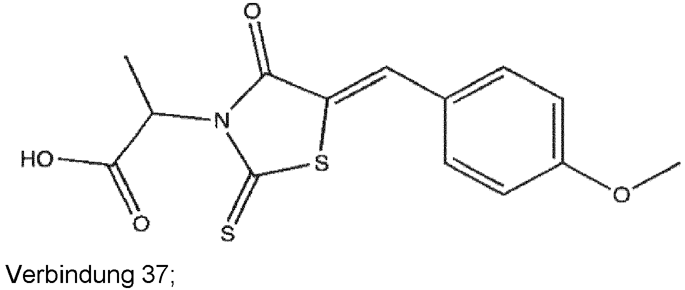

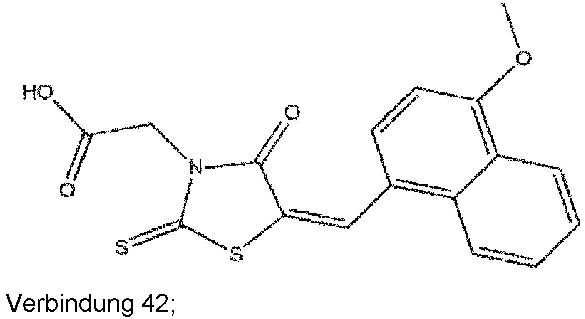

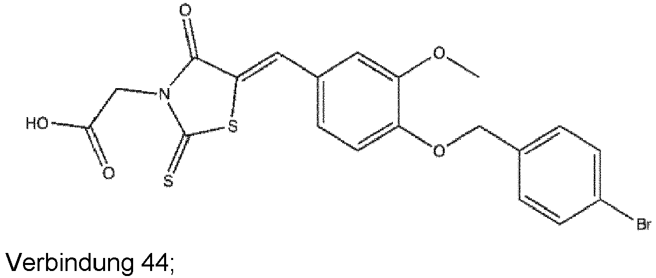

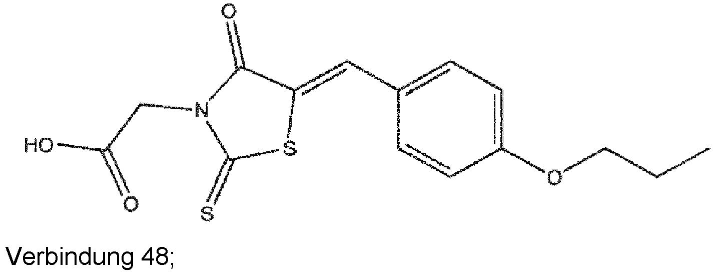

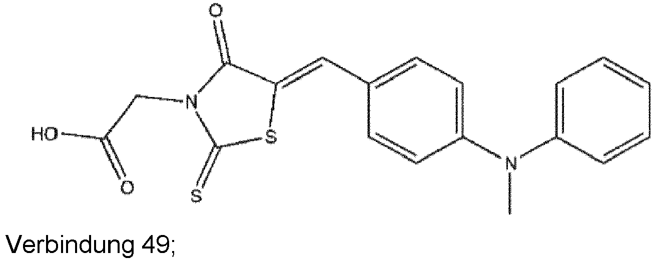

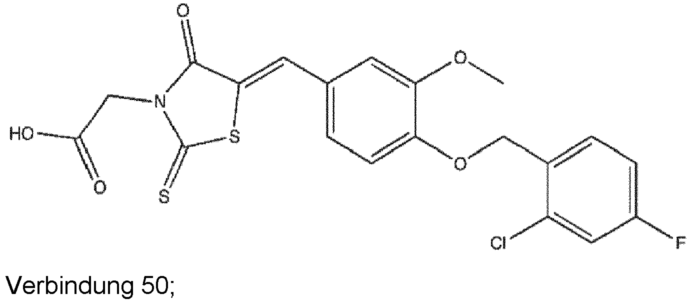

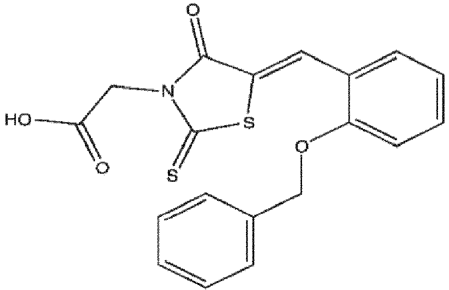

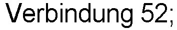

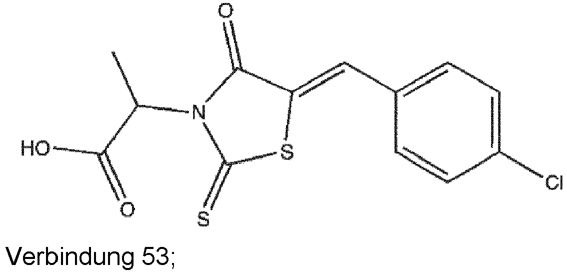

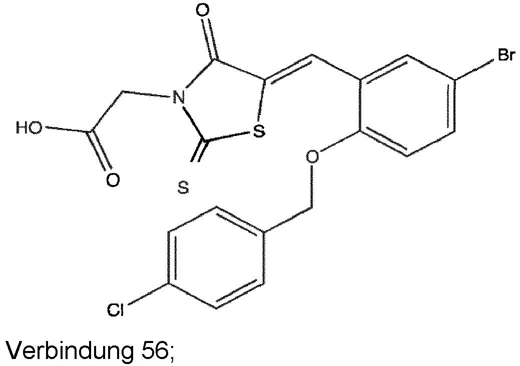

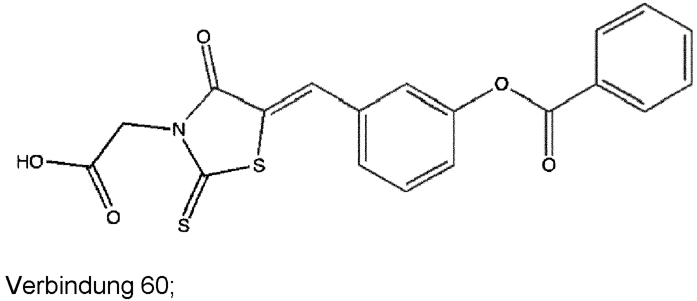

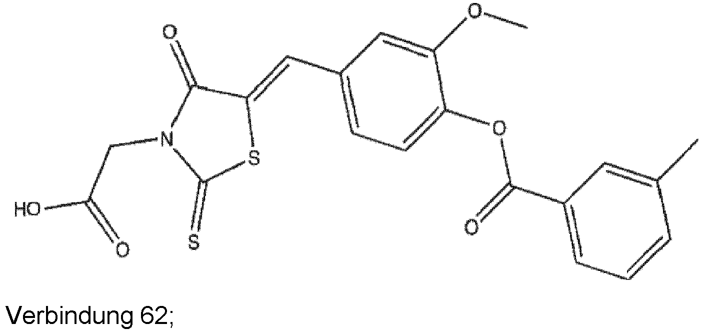

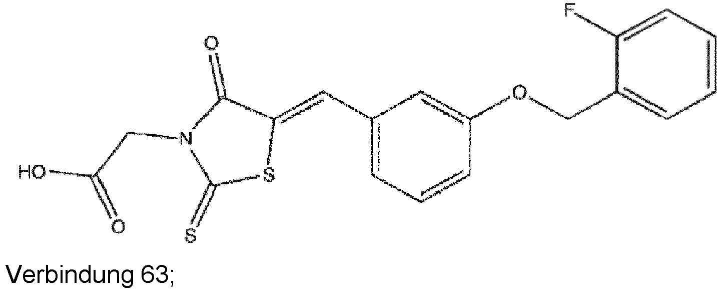

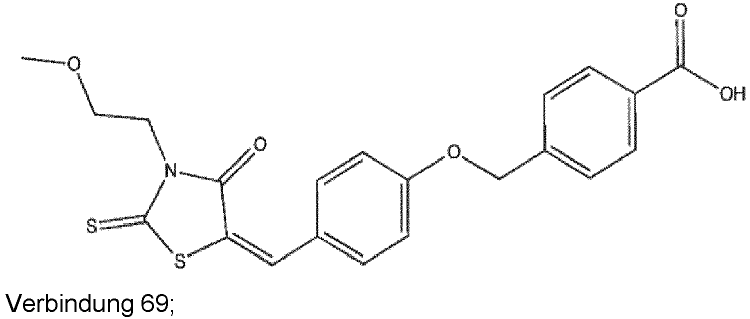

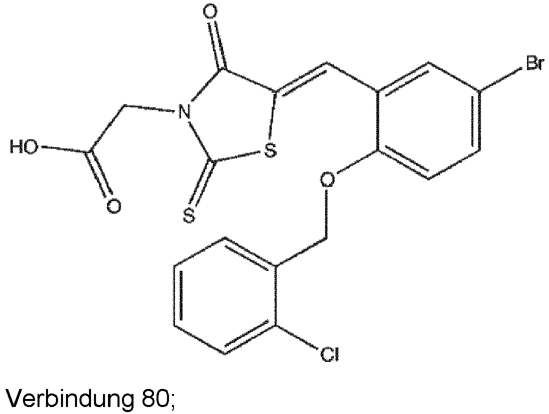

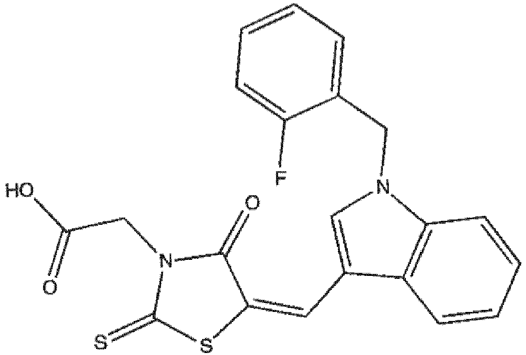

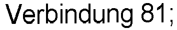

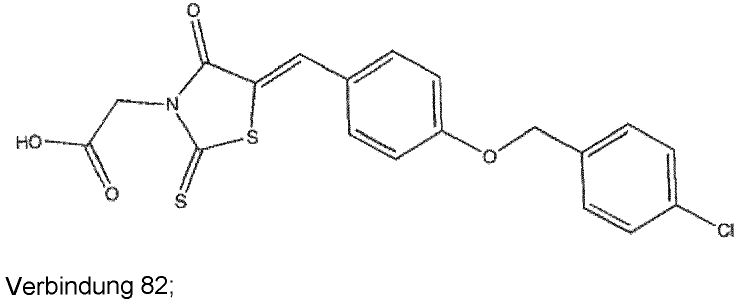

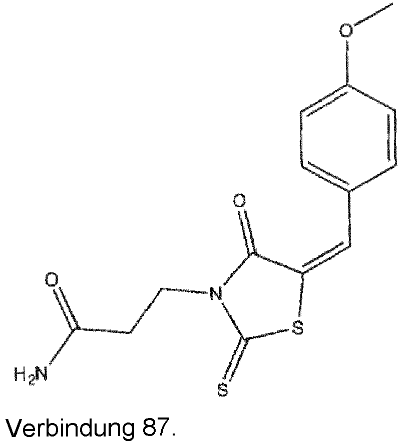

## Revendications

1. Composition pour le traitement d'une infection bactérienne chez un sujet, comprenant un véhicule pharmaceutiquement acceptable et une quantité efficace d'un composé représenté par l'une quelconque des formules suivantes, ou l'un de ses sels pharmaceutiquement acceptables:

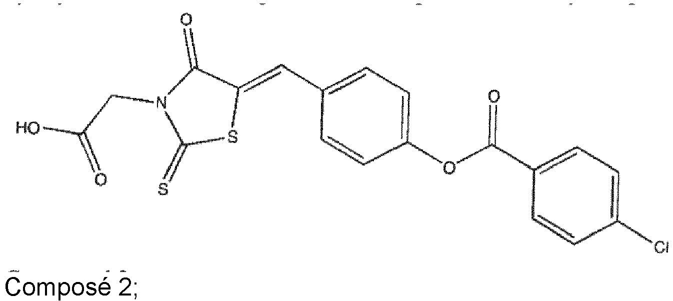

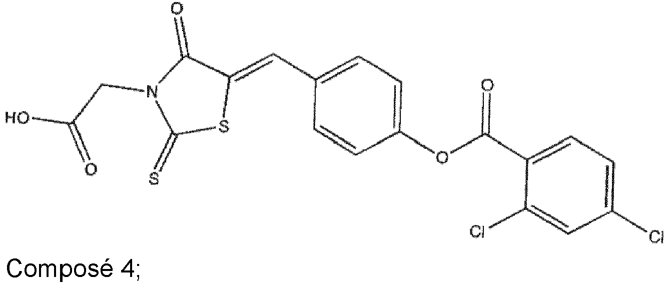

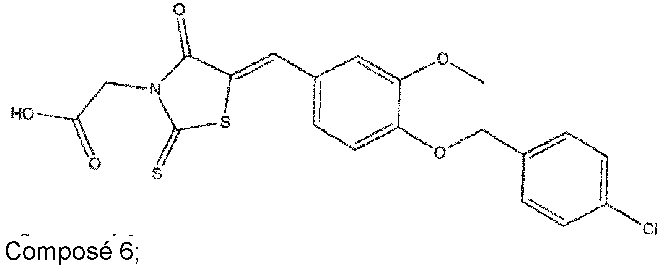

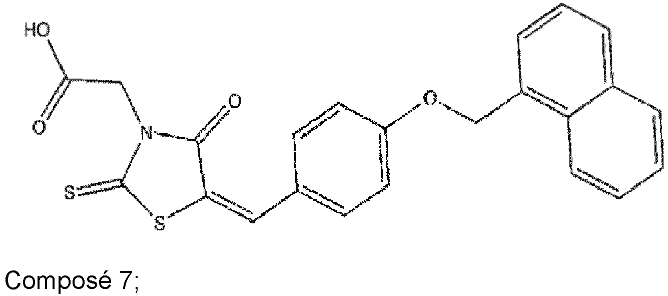

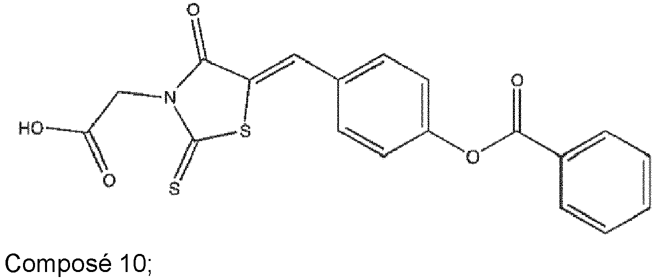

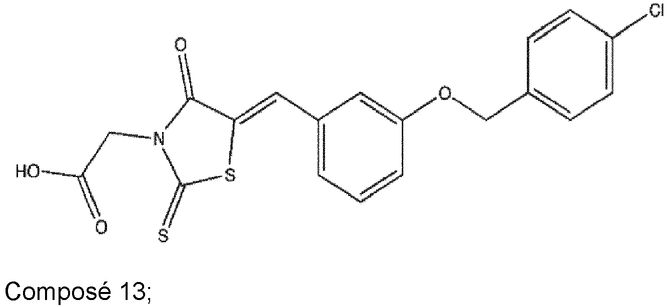

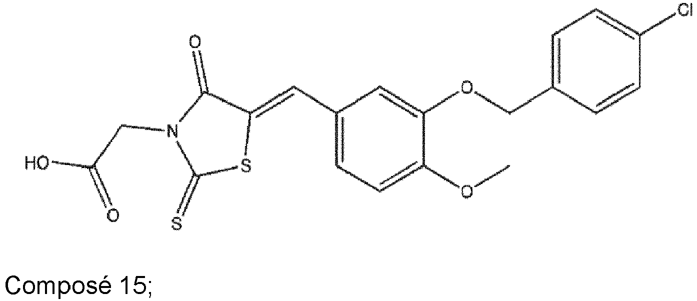

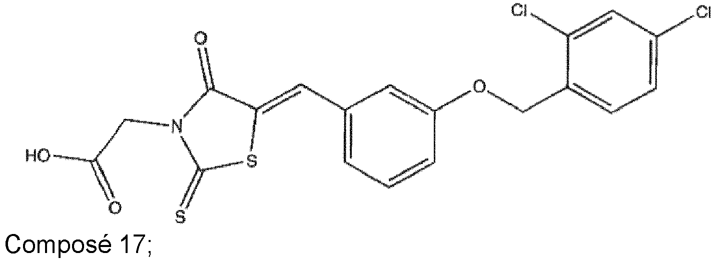

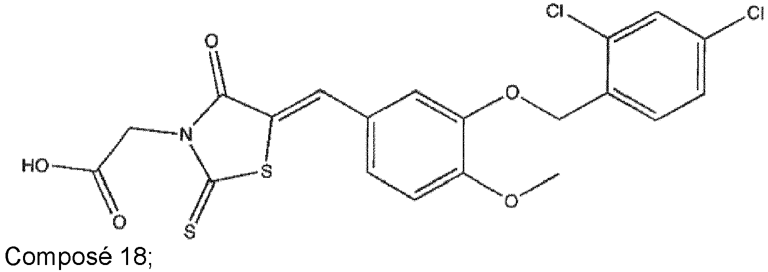

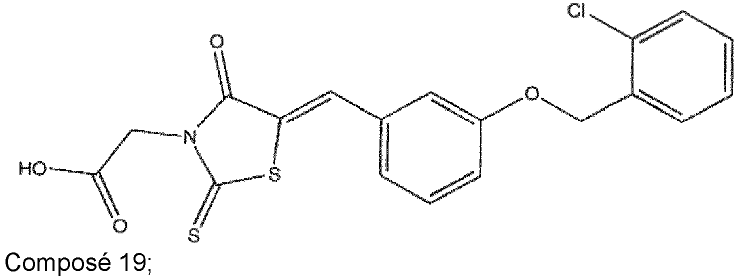

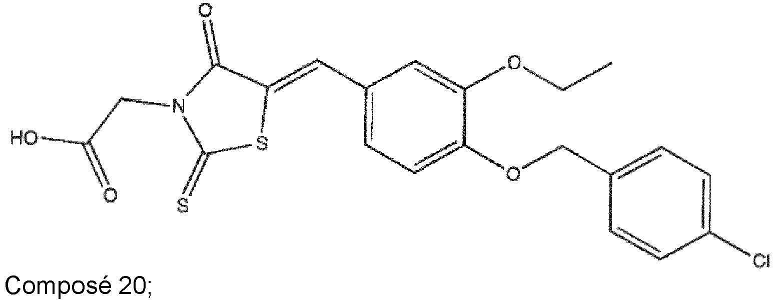

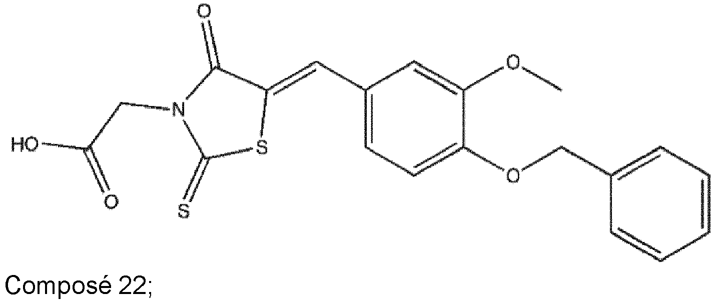

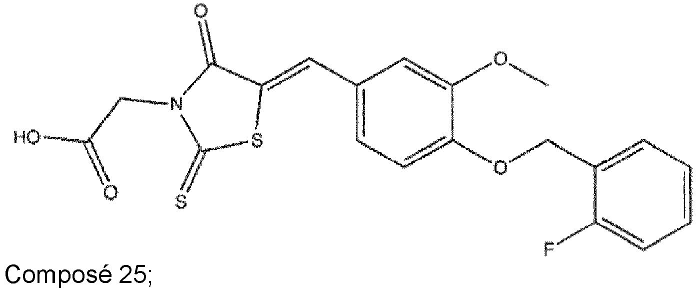

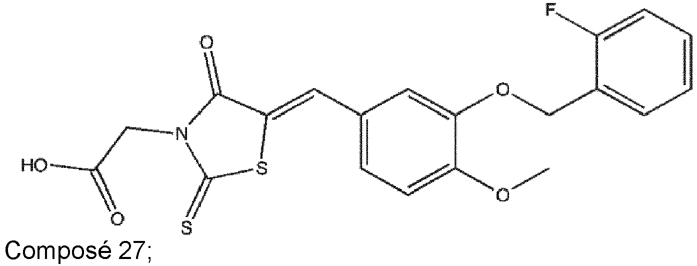

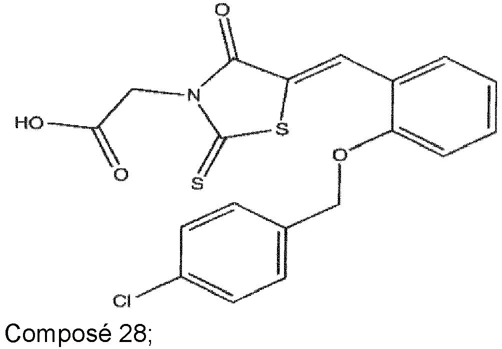

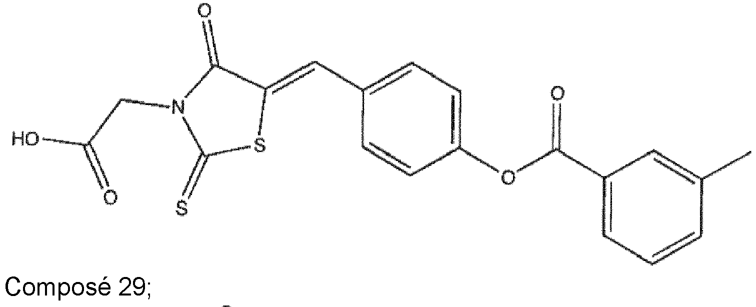

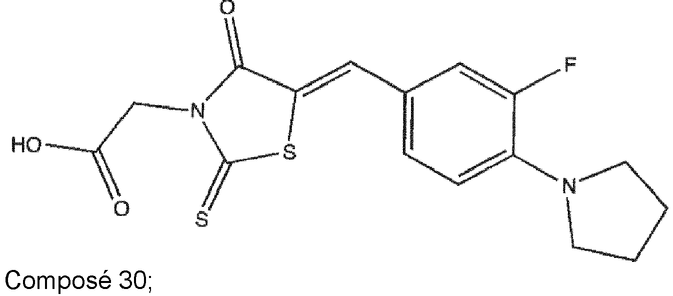

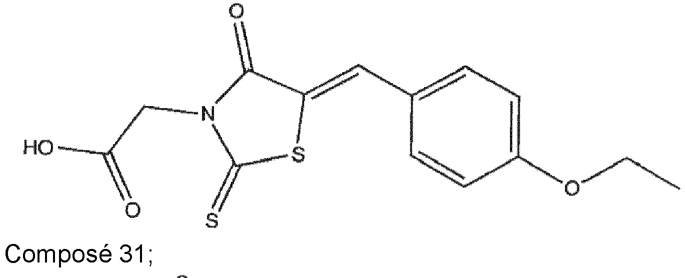

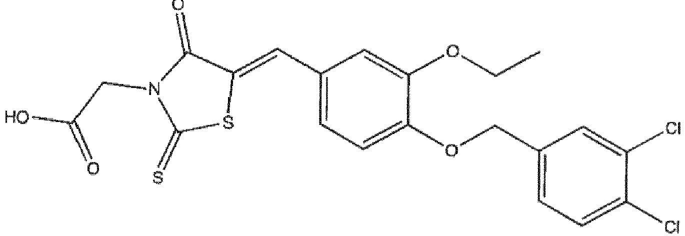

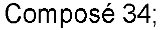

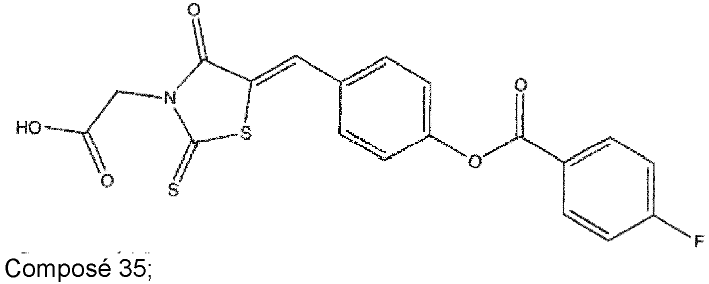

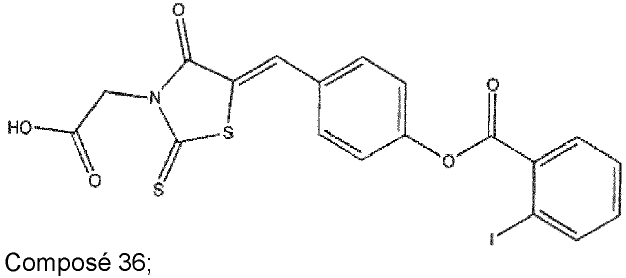

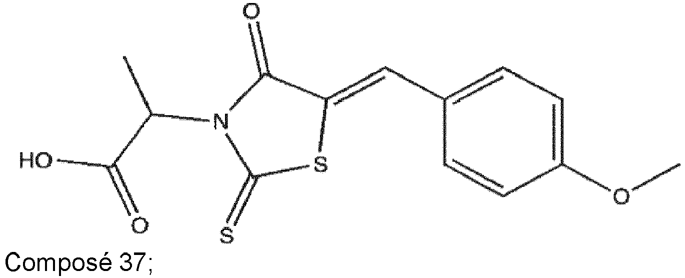

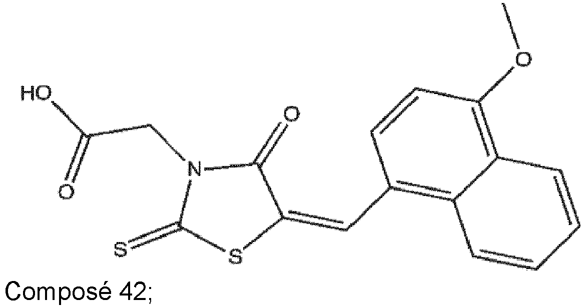

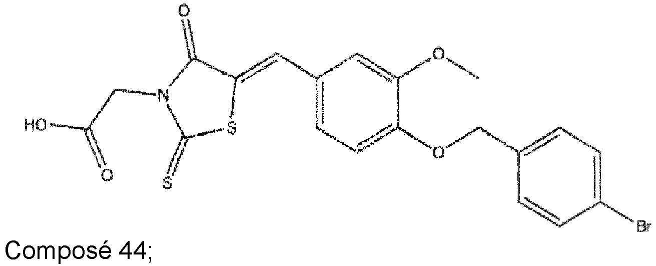

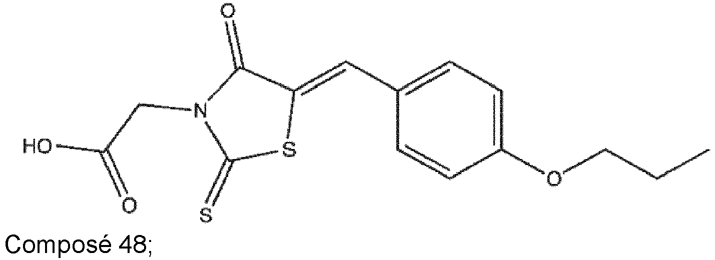

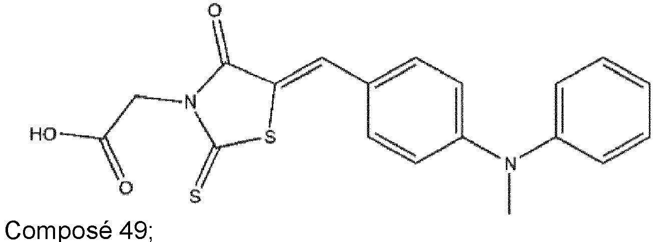

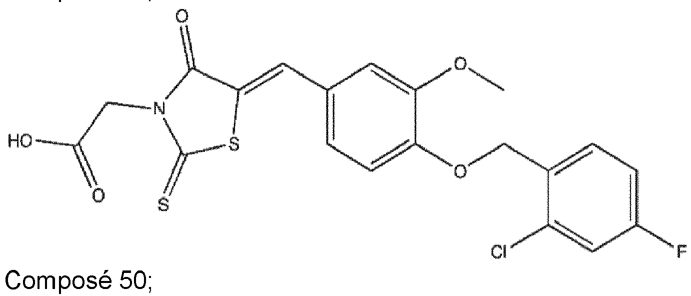

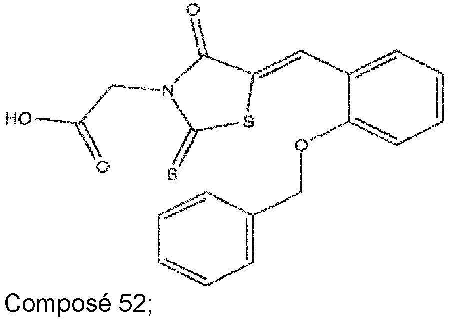

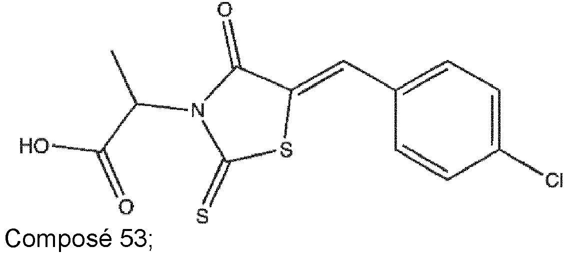

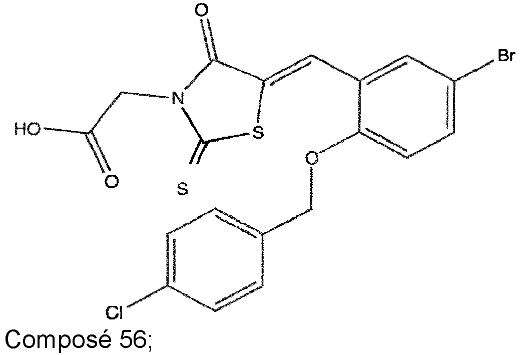

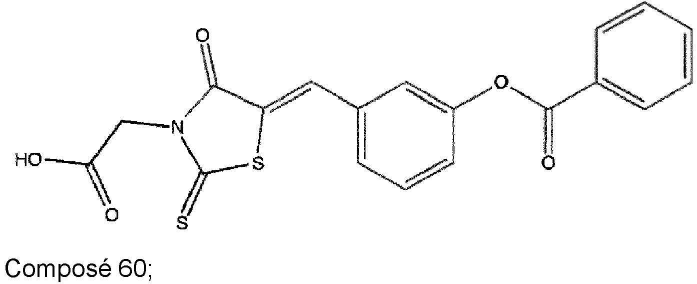

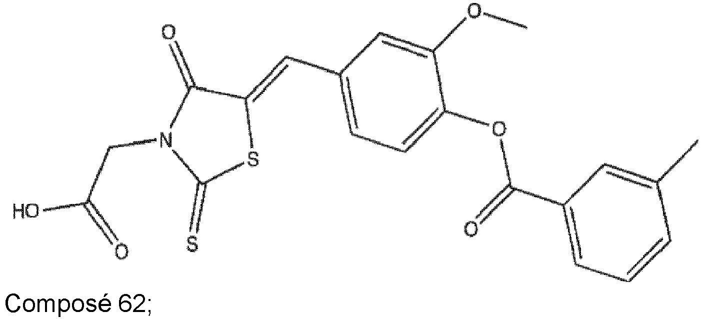

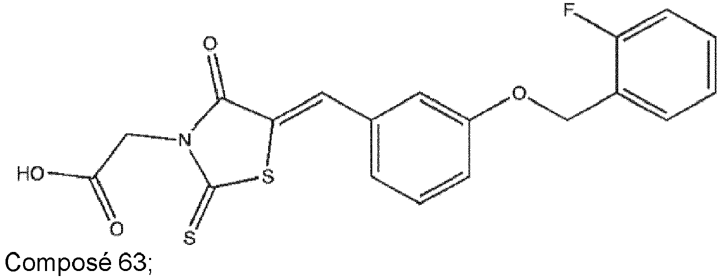

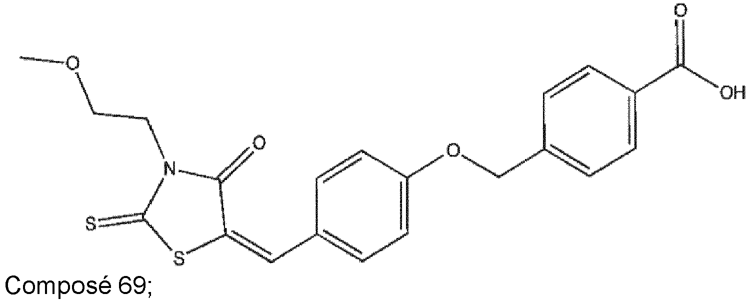

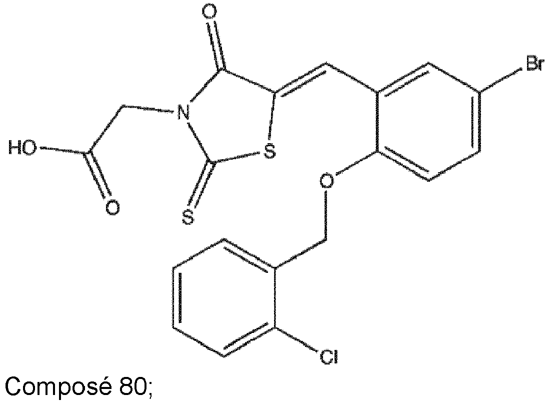

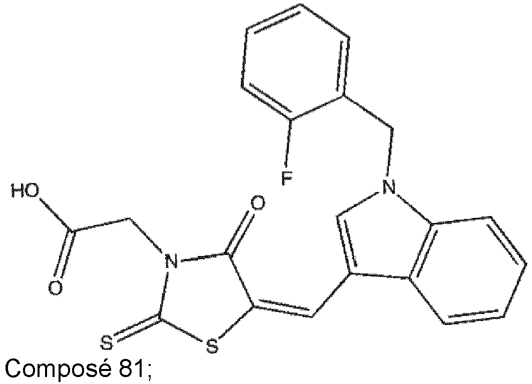

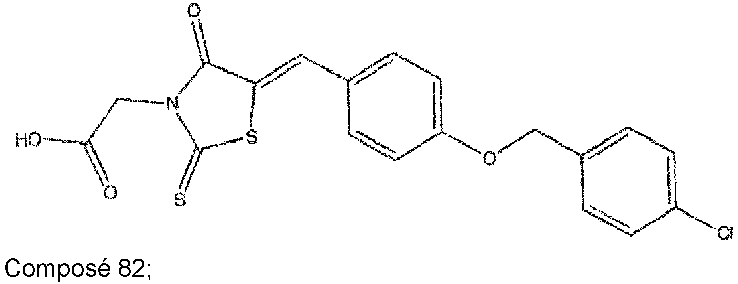

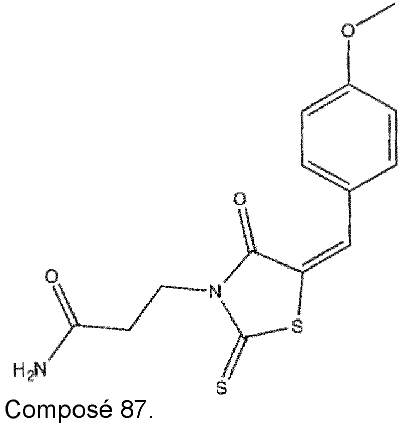

2. Composition pour utilisation selon la revendication 1, dans laquelle l'infection est causée par des bactéries sensibles à la méthicilline appartenant au groupe constitué par le *Staphylococcus aureus*, *Pseudomonas aeruginosa*, *Listeria monocytogenes*, *Burkholderia cepacia*, *Escherichia coli*, *Enterococcus faecalis*, *Streptococcus pneumoniae*, et des combinaisons de ces bactéries.

3. Composition pour utilisation selon la revendication 1 ou la revendication 2, dans laquelle la composition est administrée par voie parentérale ou orale.

4. Composition pour utilisation selon la revendication 1, dans laquelle l'infection est causée par un micro-organisme résistant aux antibiotiques choisi dans le groupe constitué par: *Streptococcus pneumoniae, Campylobacter, Neisseria gonorrhoeae, Salmonella* (y compris *Salmonella* non typhoïde résistante aux médicaments et *Salmonella* sérotype typhi résistante aux médicaments), *Shigella, Enterococcus* résistant à la vancomycine *(*ERV*), Staphylococcus aureus* résistant à la vancomycine (VRSA), *Streptococcus* du groupe A résistant à l'érythromycine, *Streptococcus* du groupe B résistant à la clindamycine, *Entérobactéries* résistantes aux carbapénèmes (CRE), tuberculose résistante aux médicaments, *Entérobactéries* à spectre étendu (BLSE), *Acinetobacter* multirésistant (y compris MRAB), *Clostridium difficile, entéropathogènes E. coli (EPEC), Pseudomonas aeruginosa, H. pylori, Streptococcus anginosus* et *E*. *coli uropathogène* (UPEC) et *Staphylococcus aureus* résistant à la méthicilline (MRSA).

5. Composition pour utilisation selon la revendication 1, dans laquelle l'infection est causée par une souche multirésistante de *Listeria monocytogenes, Pseudomonas aeruginosa, Burkholderia cepacia, Enterococcus faecalis* et *Streptococcus pneumoniae.*

6. Composition pour utilisation selon une quelconque des revendications 1 à 5, dans laquelle l'infection est choisie dans le groupe constitué par l'impétigo, les furoncles, les abcès, la folliculite, la cellulite, la fasciite nécrosante, la pyomyosite, l'infection des plaies chirurgicales/traumatiques, les ulcères et brûlures infectés, l'ostéomyélite, les infections ostéo-articulaires liées aux dispositifs, l'impétigo, les lésions cutanées secondairement infectées, la méningite, l'abcès cérébral, l'empyème sous-dural et l'abcès épidural de la colonne vertébrale.

7. Composition pour utilisation selon une quelconque des revendications 1 à 5, dans laquelle l'infection est une infection des voies urinaires, et/ou des infections de la peau et des tissus mous, des os et des articulations et des infections affectant les poumons et les valves cardiaques.

8. Composition pour utilisation selon une quelconque des revendications 1 à 7, dans laquelle le sujet est hospitalisé ou immunodéprimé.

9. Composition pour utilisation selon une quelconque des revendications 1 à 8, comprenant en outre une quantité thérapeutiquement efficace d'un antibiotique.

10. Composition pour utilisation selon la revendication 9, dans laquelle l'antibiotique est choisi dans le groupe constitué de la gentamicine, de l'amikacine, de la kanamycine, de la néomycine, de la spectinomycine, de la néamine et de leurs combinaisons.

11. Composition pour utilisation selon la revendication 9 ou la revendication 10, dans laquelle la quantité thérapeutiquement efficace de l'antibiotique est inférieure à une dose thérapeutiquement efficace ou thérapeutiquement optimale de l'antibiotique lorsqu'il est administré à un sujet en l'absence de la composition.

12. Composition pour utilisation selon l'une des revendications 1 à 8, dans laquelle la composition est administrée conjointement avec un antibiotique en une quantité suffisante pour augmenter l'efficacité de l'antibiotique, l'antibiotique étant choisi dans le groupe constitué de la gentamicine, de l'amikacine, de la kanamycine, de la néomycine, de la spectinomycine, de la néamine et de leurs combinaisons.

13. Composition pour utilisation selon la revendication 12, dans laquelle la composition est administrée séparément, simultanément ou séquentiellement avec l'antibiotique.

14. Composé utilisé pour réduire la virulence des bactéries chez un sujet qui en a besoin, dans lequel la bactérie est choisie dans le groupe constitué de *Staphylococcus aureus* sensible à la méthicilline, *Pseudomonas aeruginosa, Listeria monocytogenes, Burkholderia cepacia, Escherichia coli, Enterococcus faecalis, Streptococcus pneumoniae* et leurs combinaisons, et le composé est représenté par l'une des formules suivantes, ou un de leurs sels pharmaceutiquement acceptables:
